# EUROPÄISCHE PATENTSCHRIFT

(11) **EP 2 494 003 B1**
(45) Veröffentlichungstag und Bekanntmachung des Hinweises auf die Patenterteilung: **24.07.2013**
(21) Anmeldenummer: 10763136.8
(22) Anmeldetag: 04.10.2010
(51) Int. Cl.: C09K 19/12, C09K 19/04, C09K 19/54

(54) **POLYMERISIERBARE VERBINDUNGEN UND IHRE VERWENDUNG IN FLÜSSIGKRISTALLANZEIGEN**
POLYMERIZABLE COMPOUNDS AND USE THEREOF IN LIQUID CRYSTAL DISPLAYS
COMPOSES POLYMERISABLES ET LEUR UTILISATION DANS DES ECRANS A BASE DE CRISTAUX LIQUIDES

(30) Priorität: 28.10.2009 DE 102009050989
(43) Veröffentlichungstag der Anmeldung: 05.09.2012
(73) Patentinhaber: Merck Patent GmbH, 64293 Darmstadt (DE)
(72) Erfinder: GOETZ, Achim, 64665 Alsbach-Hähnlein (DE); MARTEN, Christoph, 64295 Darmstadt (DE); LAUT, Sven, Christian, 64347 Griesheim (DE); TAUGERBECK, Andreas, 64285 Darmstadt (DE)
(86) Internationale Anmeldenummer: PCT/EP2010/006050
(87) Internationale Veröffentlichungsnummer: WO 2011/050893

(56) Entgegenhaltungen:
- EP-A1- 1 908 814
- WO-A1-00/29505
- WO-A1-2009/030329
- JP-A- 10 036 847
- US-A- 5 723 066
- US-A- 6 090 308
- US-A1- 2009 141 215

## Beschreibung

Die vorliegende Erfindung betrifft polymerisierbare Verbindungen, Verfahren und Zwischenprodukte zu ihrer Herstellung, und ihre Verwendung für optische, elektrooptische und elektronische Zwecke, insbesondere in Flüssigkristall (FK)-Medien und FK-Anzeigen, vor allem in FK-Anzeigen des PS- oder PSA-Typs ("polymer sustained" bzw. "polymer sustained alignment").

Die derzeit verwendeten Flüssigkristallanzeigen (FK-Anzeigen) sind meist solche des TN-Typs ("twisted nematic"). Diese weisen allerdings den Nachteil einer starken Blickwinkelabhängigkeit des Kontrastes auf. Daneben sind sogenannte VA-Anzeigen ("vertically aligned") bekannt, die einen breiteren Blickwinkel aufweisen. Die FK-Zelle einer VA-Anzeige enthält eine Schicht eines FK-Mediums zwischen zwei transparenten Elektroden, wobei das FK-Medium üblicherweise einen negativen Wert der dielektrischen (DK-) Anisotropie aufweist. Die Moleküle der FK-Schicht sind im ausgeschalteten Zustand senkrecht zu den Elektrodenflächen (homöotrop) oder gekippt homöotrop ("tilted") orientiert. Bei Anlegen einer elektrischen Spannung an die beiden Elektroden findet eine Umorientierung der FK-Moleküle parallel zu den Elektrodenflächen statt.

Weiterhin sind OCB-Anzeigen ("optically compensated bend") bekannt, die auf einem Doppelbrechungseffekt beruhen und eine FK-Schicht mit einer sogenannten "bend"-Orientierung und üblicherweise positiver (DK-) Anisotropie aufweisen. Bei Anlegen einer elektrischen Spannung findet eine Umorientierung der FK-Moleküle senkrecht zu den Elektrodenflächen statt. Darüber hinaus enthalten OCB-Anzeigen normalerweise einen oder mehrere doppelbrechende optische Retardationsfilme, um unerwünschte Lichtdurchlässigkeit der "bend"-Zelle im dunklen Zustand zu vermeiden. OCB-Anzeigen besitzen gegenüber TN-Anzeigen einen weiteren Blickwinkel und kürzere Schaltzeiten.

Weiterhin sind sogenannte IPS-Anzeigen ("In-Plane Switching") bekannt, die eine FK-Schicht zwischen zwei Substraten enthalten, wobei die beiden Elektroden nur auf einem der beiden Substrate angeordnet sind, und vorzugsweise ineinander verzahnte, kammförmige Strukturen aufweisen. Dadurch wird bei Anlegen einer Spannung an die Elektroden ein elektrisches Feld zwsichen ihnen erzeugt, welches eine signifikante Komponente parallel zur FK-Schicht aufweist. Dies bewirkt eine Umorientierung der FK-Moleküle in der Schichtebene.

Des weiteren wurden sogenannte FFS-Anzeigen ("Fringe-Field-Switching") vorgeschlagen (siehe u.a. S.H. Jung et al., Jpn. J. Appl. Phys., Band 43, No. 3, 2004, 1028), die ebenfalls zwei Elektroden auf dem gleichen Substrat beinhalten, wovon jedoch im Gegensatz zu IPS-Anzeigen nur eine als kammförmig strukturierte Elektrode ausgebildet ist, und die andere Elektrode unstrukturiert ist. Dadurch wird ein starkes . sogenanntes "fringe field" erzeugt, also ein starkes elektrisches Feld nahe am Rand der Elektroden und in der gesamten Zelle ein elektrisches Feld, welches sowohl eine starke vertikale als auch eine starke horizontale Komponente aufweist. Sowohl IPS-Anzeigen als auch FFS-Anzeigen weisen eine geringe Blickwinkelabhängigkeit des Kontrastes auf.

In VA-Anzeigen des neueren Typs ist die einheitliche Ausrichtung der FK-Moleküle auf mehrere kleinere Domänen innerhalb der FK-Zelle beschränkt. Zwischen diesen Domänen, auch als Tilt-Domänen (engl. "tilt domains") bezeichnet, können Disklinationen existieren. VA-Anzeigen mit Tilt-Domänen weisen, verglichen mit herkömmlichen VA-Anzeigen, eine größere Blickwinkelunabhängigkeit des Kontrastes und der Graustufen auf. Außerdem sind solche Anzeigen einfacher herzustellen, da eine zusätzliche Behandlung der Elektrodenoberfläche zur einheitlichen Orientierung der Moleküle im eingeschalteten Zustand, wie z.B. durch Reiben, nicht mehr notwendig ist. Stattdessen wird die Vorzugsrichtung des Kipp- oder Tiltwinkels (engl. "pretilt") durch eine spezielle Ausgestaltung der Elektroden kontrolliert.

In den sogenannten MVA-Anzeigen ("multidomain vertical alignment") wird dies üblicherweise dadurch erreicht, dass die Elektroden Erhebungen oder Vorsprünge (engl. "protrusions") aufweisen, die einen lokalen pretilt verursachen. Als Folge werden die FK-Moleküle beim Anlegen einer Spannung in verschiedenen, definierten Regionen der Zelle in unterschiedliche Richtungen parallel zu den Elektrodenflächen orientiert. Dadurch wird ein "kontrolliertes" Schalten erreicht und das Entstehen störender Disklinationslinien vermieden. Diese Anordnung verbessert zwar den Blickwinkel der Anzeige, führt aber zu einer Verringerung ihrer Lichtdurchlässigkeit.

Eine Weiterentwicklung von MVA verwendet Protrusions nur auf einer Elektroden-Seite, die gegenüberliegende Elektrode weist hingegen Schlitze (engl. "slits") auf, was die Lichtdurchlässigkeit verbessert. Die geschlitzten Elektroden erzeugen beim Anlegen einer Spannung ein inhomogenes elektrisches Feld in der FK-Zelle, so dass weiterhin ein kontrolliertes Schalten erreicht wird. Zur weiteren Verbesserung der Lichtdurchlässigkeit können die Abstände zwischen den slits und protrusions vergrößert werden, was jedoch wiederum zu einer Verlängerung der Schaltzeiten führt. Beim sogenannten PVA (Patterned VA) kommt man ganz ohne Protrusions aus, indem man beide Elektroden auf den gegenüberliegenden Seiten durch Schlitze strukturiert, was zu einem erhöhten Kontrast und verbesserter Lichtdurchlässigkeit führt, aber technologisch schwierig ist und das Display empfindlicher gegen mechanische Einflüsse macht (Klopfen, engl. "tapping", etc.). Für viele Anwendungen, wie beispielsweise Monitore und vor allem TV-Bildschirme, ist jedoch eine Verkürzung der Schaltzeiten sowie eine Verbesserung des Kontrastes und der Luminanz (Transmission) der Anzeige gefragt.

Eine Weiterentwicklung stellen die sogenannten PS-bzw. PSA-Anzeigen ("Polymer Sustained" bzw. "Polymer Sustained Alignment") dar, für die auch gelegentlich der Begriff "Polymer Stabilized" verwendet wird. In diesen Anzeigen wird dem FK-Medium eine geringe Menge (zum Beispiel 0.3 Gew.%, typischerweise <1 Gew.%) einer oder mehrerer polymerisierbarer Verbindung(en) zugesetzt, welche nach Einfüllen in die FK-Zelle mit oder ohne angelegte elektrische Spannung zwischen den Elektroden in situ polymerisiert bzw. vernetzt wird, üblicherweise durch UV-Photopolymerisation. Als besonders geeignet hat sich der Zusatz von polymerisierbaren mesogenen oder flüssigkristallinen Verbindungen, auch als reaktive Mesogene oder "RM"s bezeichnet, zur FK-Mischung erwiesen.

Nachfolgend wir der Begriff "PSA", falls nicht anders angegeben, stellvertretend für PS-Anzeigen und PSA-Anzeigen verwendet.

Mittlerweile wird das PS(A)-Prizip in diversen klassischen FK-Anzeigen angewendet. So sind beispielsweise PSA-VA-, PSA-OCB-, PSA-IPS-, PSA-FFS- und PSA-TN-Anzeigen bekannt. Die Polymerisation der polymerisierbaren Verbindung(en) erfolgt bei PSA-VA- und PSA-OCB-Anzeigen vorzugsweise bei angelegter elektrischer Spannung, bei PSA-IPS-Anzeigen mit oder ohne angelegte elektrische Spannung. Wie man in Testzellen nachweisen kann, führt das PS(A)-Verfahren zu einem pretilt in der Zelle. Bei PSA-OCB-Anzeigen beispielsweise kann man erreichen, dass die Bend-Struktur stabilisiert wird, so dass man ohne Offset-Spannung auskommt oder diese reduzieren kann. Im Falle von PSA-VA-Anzeigen wirkt sich der pretilt positiv auf die Schaltzeiten aus. Für PSA-VA-Anzeigen kann ein Standard-MVA- bzw. -PVA Pixel- und Elektroden-Layout verwendet werden. Darüber hinaus kann man aber beispielsweise auch mit nur einer strukturierten Elektrodenseite und ohne Protrusions auskommen, was die Herstellung wesentlich vereinfacht und gleichzeitig zu einem sehr guten Kontrast bei sehr guter Lichtdurchlässigkeit führt.

Außerdem haben sich sogenannte Posi-VA-Anzeigen ("Positiv-VA") als besonders günstige Ausführungsform erwiesen. Ebenso wie bei klassischen VA-Anzeigen, ist bei Posi-VA-Anzeigen die Ausgangsorientierung der Flüssigkristalle im spannungsfreien Ausgangszustand, homöotrop, also im wesentlichen senkrecht zu den Substraten. Im Gegensatz zu klassischen VA-Anzeigen werden in Posi-VA-Anzeigen jedoch FK-Medien mit positiver dielektrischer Anisotropie verwendet. Die beiden Elektroden in Posi-VA-Anzeigen sind, wie in den üblicherweise verwendeten IPS-Anzeigen, nur auf einem der beiden Substrate angeordnet, und weisen vorzugsweise ineinander verzahnte, kammförmige (interdigitale) Strukturen auf. Durch Anlegen einer elektrischen Spannung an die interdigitale Elektroden, die ein im wesentlichen zur Schicht des FK-Mediums paralleles Feld erzeugen, werden die FK-Moleküle in eine im wesentlichen zu den Substraten parallele Orientierung überführt. Auch bei Posi-VA-Anzeigen hat sich eine Polymerstabilisierung (PSA) als vorteilhaft erwiesen, d.h. die Zugabe von RMs zum FK-Medium, welche in der Zelle polymerisiert werden, wodurch eine erhebliche Reduzierung der Schaltzeiten realisiert werden konnte.

PSA-VA-Anzeigen sind beispielsweise in JP 10-036847 A, EP 1 170 626 A2, US 6,861,107, US 7,169,449, US 2004/0191428 A1, US 2006/0066793 A1 und US 2006/0103804 A1 beschrieben. PSA-OCB-Anzeigen sind beispielsweise in T.-J- Chen et al., Jpn. J. Appl. Phys. 45, 2006, 2702-2704 und S. H. Kim, L.-C- Chien, Jpn. J. Appl. Phys. 43, 2004, 7643-7647 beschrieben. PSA-IPS-Anzeigen sind zum Beispiel in US 6,177,972 und Appl. Phys. Lett. 1999, 75(21), 3264 beschrieben. PSA-TN-Anzeigen sind zum Beispiel in Optics Express 2004, 12(7), 1221 beschrieben.

PSA-Anzeigen können ebenso wie die oben beschriebenen konventionallen FK-Anzeigen als Aktivmatrix- oder Passivmatrix-Anzeigen betrieben werden. Bei Aktivmatrix-Anzeigen erfolgt die Ansteuerung einzelner Bildpunkte üblicherweise durch integrierte, nicht-lineare aktive Elemente wie beispielsweise Transistoren (z.B. Dünnfilmtransistoren, engl. "thin film transistor" bzw. "TFT"), bei Passivmatrix-Anzeigen üblicherweise nach dem Multiplex-Verfahren, wobei beide Verfahren aus dem Stand der Technik bekannt sind.

Insbesondere für Monitor- und vor allem TV-Anwendungen ist nach wie vor die Optimierung der Schaltzeiten, wie aber auch des Kontrastes und der Luminanz (also auch Transmission) der FK-Anzeige gefragt. Hier kann das PSA-Verfahren entscheidende Vorteile bringen. Insbesondere bei PSA-VA-, PSA-IPS-, PSA-FFS- und PSA-Posi-VA-Anzeigen kann man ohne nennenswerte Einbußen sonstiger Parameter eine Verkürzung der Schaltzeiten erreichen, die mit einem in Testzellen messbaren pretilt korrelieren.

Im Stand der Technik werden beispielsweise polymerisierbare Verbindungen der folgenen Formel verwendet 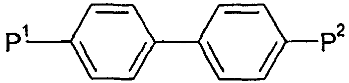 worin P eine polymerisierbare Gruppe, üblicherweise eine Acrylat- oder Methacrylategruppe bedeutet, wie beispielsweise in US 7,169,449 beschrieben.

Es ergibt sich jedoch das Problem, dass nicht alle Kombinationen bestehend aus FK-Mischung (nachfolgend auch als "FK-Hostmischung" bezeichnet) + polymerisierbarer Komponente (typischerweise RMs) für PSA-Anzeigen geeignet sind, weil sich zum Beispiel kein oder kein ausreichender Tilt einstellt, oder weil zum Beispiel die sogenannte "Voltage Holding Ratio" (VHR oder HR) für TFT-Displayanwendungen unzureichend ist. Zudem hat sich gezeigt, dass bei Verwendung in PSA-Anzeigen die aus dem Stand der Technik bekannten FK-Mischungen und RMs noch einige Nachteile aufweisen. So eignet sich nicht jedes bekannte, in FK-Mischungen lösliche RM zur Verwendung in PSA-Anzeigen. Ausserdem ist es oft schwierig, neben der direkten Messung des pretilts in der PSA-Anzeige ein geeignetes Auswahlkriterium für das RM zu finden. Noch kleiner wird die Auswahl geeigneter RMs, wenn eine Polymerisation mittels UV-Licht ohne den Zusatz von Photoinitiatoren gewünscht ist, was für bestimmte Anwendungen von Vorteil sein kann.

Darüber hinaus sollte die gewählte Kombination FK-Hostmischung/RM eine möglichst geringe Rotationsviskosität sowie möglichst gute elektrische Eigenschaften aufweisen, insbesondere sollte sie eine möglichst hohe VHR besitzen. Bei PSA-Anzeigen ist vor allem eine hohe VHR nach Bestrahlung mit UV-Licht erforderlich, da die UV-Belichtung ein notwendiger Teil des Herstellungsprozesses der Anzeige ist, aber auch als normale Belastung im Betrieb der fertigen Anzeige auftritt.

Insbesondere wäre es wünschenswert, neue Materialien für PSA-Anzeigen zur Verfügung zu haben, die einen besonders kleinen pretilt-Winkel erzeugen. Hierbei sind Materialien bevorzugt, die während der Polymerisation bei gleicher Belichtungszeit einen niedrigeren pretilt-Winkel erzeugen als die bisher bekannten Materialien, und/oder durch deren Verwendung der mit den bekannten Materialien erzielbare (höhere) pretilt-Winkel bereits nach kürzerer Belichtungszeit erreicht werden kann. Dadurch könnten die Produktionszeit (engl. "tact time") der Anzeige verkürzt und die Kosten des Produktionsprozesses verringert werden.

Ein weiteres Problem bei der Herstellung von PSA-Anzeigen ist das Vorhandensein bzw. die Entfernung von Restmengen unpolymerisierter RMs insbesondere nach dem Polymerisationsschritt zur Erzeugung des pretilt-Winkels in der Anzeige. Beispielsweise können solche nicht abreagierten RMs die Eigenschaften der Anzeige nachteilig beeinflussen, indem sie z.B. nach Fertigstellung der Anzeige während des Betriebes unkontrolliert polymerisieren.

So zeigen die aus dem Stand der Technik bekannten PSA-Anzeigen oft den unerwünschten Effekt des sogenannten "image sticking" oder "image burn", d.h. dass das in der FK-Anzeige durch vorübergehende Ansteuerung einzelner Bildpunkte (pixel) erzeugte Bild auch nach Abschalten des elektrischen Feldes in diesen Bildpunkten, oder nach Ansteuerung anderer Bildpunkte, noch sichtbar bleibt.

Dieses "image sticking" kann einerseits auftreten, wenn FK-Hostmischungen mit einem geringen VHR verwendet wird. In diesen kann die UV-Komponente des Tageslichtes oder der Hintergrundbeleuchtung unerwünschte Zerfallsreaktionen der FK-Moleküle und dadurch die Erzeugung von ionischen oder radikalischen Verunreinigungen auslösen. Diese können sich insbesondere an den Elektroden bzw. den Orientierungsschichten anreichern und dort die effektive angelegte Spannung reduzieren. Dieser Effekt ist auch bei herkömmlichen FK-Anzeigen ohne Polymerkomponente zu beobachten.

In PSA-Anzeigen wird darüber hinaus oft ein zusätzlicher "image sticking"-Effekt beobachtet, der durch die Gegenwart von unpolymerisierten RMs hervorgerufen wird. Dabei wird durch UV-Licht aus der Umgebung oder von der Hintergrundbeleuchtung eine unkontrollierte Polymerisation der Rest-RMs ausgelöst. In den geschalteten Displaybereichen wird dadurch nach mehreren Ansteuerungszyklen der Tiltwinkel verändert. Als Resultat kann eine Transmissionsänderung in den geschalteten Bereichen auftreten, während sie in den ungeschalteten Bereichen unverändert bleibt.

Es ist deshalb wünschenswert, dass die Polymerisation der RMs bei der Herstellung der PSA-Anzeige möglichst vollständig abläuft und die Anwesenheit von unpolymerisierten RMs in der Anzeige möglichst ausgeschlossen oder auf ein Minimum reduziert wird. Hierzu werden Materialien benötigt, die eine möglichst effektive und vollständige Polymerisation ermöglichen. Zudem wäre ein kontrolliertes Abreagieren dieser Restmengen wünschenswert. Dies wäre einfacher, wenn das RM schneller und effektiver polymerisiert als die bisher bekannten Materialien.

Es besteht somit immer noch ein großer Bedarf an PSA-Anzeigen, insbesondere vom VA- und OCB-Typ, sowie FK-Medien und polymerisierbaren Verbindungen zur Verwendung in solchen Anzeigen, welche die oben beschriebenen Nachteile nicht oder nur in geringem Maße zeigen und verbesserte Eigenschaften besitzen. Zudem besteht ein großer Bedarf nach PSA-Anzeigen, sowie Materialien zur Verwendung in PSA-Anzeigen, die vorteilhate Eigenschaften aufweisen, insbesondere einen hohen spezifischen Widerstand bei gleichzeitig großem Arbeitstemperaturbereich, kurze Schaltzeiten auch bei tiefen Temperaturen und niedriger Schwellenspannung, einen niedrigen pretilt-Winkel, eine Vielzahl von Graustufen, einen hohen Kontrast und einen weiten Blickwinkel ermöglichen, sowie hohe Werte der "voltage holding ratio" (VHR) nach UV-Belastung und der Tieftemperaturstabilität, auch als "LTS" (low temperature stability) bezeichnet, d.h. der Stabilität der FK-Mischung gegen spontane Auskristallisation einzelner Komponenten.

Der Erfindung liegt die Aufgabe zugrunde, neue geeignete Materialien, insbesondere RMs und diese enthaltende FK-Medien, für die Verwendung in PSA-Anzeigen bereitzustellen, die die oben angegebenen Nachteile nicht oder in geringerem Maße aufweisen, möglichst schnell und vollständig polymerisieren, eine möglichst schnelle Einstellung eines niedrigen pretilt-Winkels ermöglichen, das Auftreten von "image sticking" in der Anzeige verringern oder vermeiden, und vorzugsweise gleichzeitig sehr hohe spezifische Widerstände, niedrige Schwellenspannungen und niedrige Schaltzeiten ermöglichen. Zudem sollten die FK-Medien günstige FK-Phaseneigenschaften sowie hohe VHR- und LTS-Werte aufweisen. Weitere Aufgabe der Erfindung ist die Bereitstellung von neuen RMs, insbesondere für optische, elektrooptische und elektronische Anwendungen, sowie von geeigneten Verfahren und Zwischenprodukten zu ihrer Herstellung.

Insbesondere liegt der Erfindung die Aufgabe zugrunde, polymerisierbare Verbindungen bereitzustellen, die nach Photopolymerisation einen größeren maximalen Pretilt erzeugen, was zu einem schnelleren Erreichen des gewünschten Pretilts und so zu deutlich verkürzten Zeiten bei der Herstellung der FK-Anzeige führt

Diese Aufgabe wurde erfindungsgemäß gelöst durch die Bereitstellung von Materialien, Verfahren und FK-Anzeigen wie in der vorliegenden Anmeldung beschrieben. Insbesondere wurde überraschend gefunden, dass die oben beschriebenen Aufgaben teilweise oder vollständig gelöst werden können, indem man PSA-Anzeigen bereitstellt, die eine oder mehrere polymerisierte erfindungsgemäße Verbindungen enthalten, bzw. indem man zur Herstellung solcher PSA-Anzeigen FK-Medien verwendet, welche eine oder mehrere erfindungsgemäße polymerisierbare Verbindungen enthalten. Die erfindungsgemäßen polymerisierbaren Verbindungen enthalten als mesogene Gruppe eine Terphenylgruppe, die mindestens einfach durch ein Halogenatom substituiert ist, sowie eine oder mehrere polymerisierbare Gruppen. Besonders bevorzugt sind erfindungsgemäße Verbindungen enthaltend zwei oder mehr als zwei polymerisierbare Gruppen, worin mindestens eine dieser polymerisierbaren Gruppen über eine Abstandsgruppe mit der Terphenylgruppe verbunden ist, und mindestens eine dieser polymerisierbaren Gruppen direkt, d.h. nicht über eine Abstandsgruppe, mit der Terphenylgruppe verbunden ist.

Die Verwendung solcher polymerisierbarer Verbindungen in erfindungsgemäßen FK-Medien und PSA-Anzeigen führt zu einem besonders schnellen Erreichen des gewünschten Pretilts und zu deutlich verkürzten Zeiten bei der Herstellung der Anzeige. Dies konnte in Verbindung mit einem FK-Medium mittels belichtungszeitabhängiger Pretilt-Messungen in VA-Tilt-Messzellen nachgewiesen werden. Insbesondere konnte ein Pretilt ohne den Zusatz von Photoinitiator erreicht werden.

Da die erfindungsgemäßen polymerisierbaren Verbindungen in den PSA-Anzeigen eine deutlich höhere Polymerisationsgeschwindigkeit zeigen, verbleiben auch weniger unreagierte Restmengen in der FK-Zelle, wodurch deren elektrooptische Eigenschaften verbessert werden, und das kontrollierte Abreagieren dieser Restmengen einfacher wird.

Polymerisierbare Terphenylverbindungen wurden im Stand der Technik bereits beschrieben. Die WO 2009/030329 A1 offenbart PSA-Anzeigen mit RMs, die nur eine Abstandsgruppe aufweisen und auch eine Terphenylgruppe enthalten können, offenbart aber explizit keine erfindungsgemäßen reaktiven Terphenyle mit lateraler Halogensubstitution. WO 93/22397 A1 und US 5,723,066 offenbaren direaktive Terphenyl-RMs mit lateralen Fluorsubstituenten, sowie PDLC-Anzeigen enthaltend phasenseparierte Mikrotröpfchen einer FK-Mischung in einer optisch isotropen und transparenten Polymermatrix, offenbaren aber explizit keine erfindungsgemäßen reaktiven Terphenyle mit nur einer Abstandsgruppe, oder die generelle Verwendung von reaktiven Terphenylen in PSA-Anzeigen.
Die US 6,090,308 und WO 00/29505 A1 offenbaren direaktive polymerisierbare Terphenylverbindungen mit gleichen oder verschiedenen Abstandsgruppen, sowie deren Verwendung beispielsweise in polymerisierbaren FK-Mischungen und FK-Polymerfilmen. Die US 2009/141215 A1 offenbart direaktive polymerisierbare Biphenylverbindungen sowie deren Verwendung in PSA-Anzeigen. Die EP 1 908 814 A1 offenbart FK-Mischungen enthaltend unpolymerisierbare Terphenylverbindungen. Die in der vorliegenden Anmeldung beanspruchten reaktiven Mesogene oder deren Verwendung in PSA-Anzeigen werden durch diese Dokumente jedoch nicht offenbart oder nahegelegt.

Die spezifische Verwendung von erfindungsgemäßen, reaktiven halogensubstituierten Terphenylen in PSA-Anzeigen zur schnellen Einstellung eines Tiltwinkels durch in situ-Polymerisation im elektrischen Feld wurde somit im Stand der Technik weder beschrieben noch nahegelegt.

Zudem wurde völlig überraschend gefunden, dass erfindungsgemäße polymerisierbare Verbindungen mit nur einer Abstandsgruppe und einer mesogenen Terphenylgruppe, welche mindestens einfach mit Halogen substituiert ist, bei Verwendung in PSA-Anzeigen eine deutlich schnellere Tiltwinkel-Generierung und eine schnellere und vollständigere Polymerisation zeigen, als strukturanaloge polymerisierbare Verbindungen mit einer unsubstituierten mesogenen Terphenylgruppe. Dies konnte durch direkte Vergleichsversuche bestätigt werden. Dieses Ergebnis war durch den Stand der Technik weder beschrieben noch nahegelegt.

Gegenstand der Erfindung ist somit die Verwendung von polymerisierbaren Verbindungen enthaltend eine Terphenylgruppe die mindestens einfach durch ein Halogenatom, vorzugsweise durch F oder Cl, substituiert ist, und eine oder mehrere, vorzugsweise zwei oder mehr als zwei, polymerisierbare Gruppen, wobei vorzusgweise mindestens eine dieser polymerisierbaren Gruppen über eine Abstandsgruppe und mindestens eine dieser polymerisierbaren Gruppen direkt, d.h. nicht über eine Abstandsgruppe, mit der Terphenylgruppe verbunden ist (nachfolgend auch als "erfindungsgemäße polymerisierbare Verbindungen" bezeichnet), in Flüssigkristall-(FK-)medien und FK-Anzeigen des PS- oder PSA- (polymer sustained alignment) Typs.

Ein weiterer Gegenstand der Erfindung ist ein FK-Medium enthaltend eine oder mehrere erfindungsgemäße polymerisierbare Verbindungen und eine oder mehrere zusätzliche Verbindungen, welche auch mesogen, flüssigkristallin und/oder polymerisierbar sein können.

Ein weiterer Gegenstand der Erfindung ist ein FK-Medium enthaltend ein Polymer erhältlich durch Polymerisation einer oder mehrerer erfindungsgemäßer polymerisierbarer Verbindungen und eine oder mehrere zusätzliche Verbindungen, welche auch mesogen, flüssigkristallin und/oder polymerisierbar sein können.

Ein weiterer Gegenstand der Erfindung ist ein FK-Medium enthaltend
- eine polymerisierbare Komponente A), enthaltend eine oder mehrere erfindungsgemäße polymerisierbare Verbindungen, sowie
- eine flüssigkristalline Komponente B), im Folgenden auch als "FK-Hostmischung" bezeichnet, enthaltend eine oder mehrere, vorzugsweise zwei oder mehr niedermolekulare (monomere und unpolymerisierbare) Verbindungen wie vor- und nachstehend beschrieben.

Ein weiterer Gegenstand der Erfindung ist ein Verfahren zur Herstellung eines FK-Mediums wie vor- und nachstehend beschrieben, indem man eine oder mehrere niedermolekulare flüssigkristalline Verbindungen, oder eine FK-Hostmischung wie vor- und nachstehend beschrieben, mit einer oder mehreren erfindungsgemäßen polymerisierbaren Verbindungen und gegebenenfalls mit weiteren flüssigkristallinen Verbindungen und/oder Additiven, mischt.

Ein weiterer Gegenstand der Erfindung ist die Verwendung von erfindungsgemäßen polymerisierbaren Verbindungen und erfindungsgemäßen FK-Medien in PS- und PSA-Anzeigen, insbesondere die Verwendung in PS- und PSA-Anzeigen enthaltend ein FK-Medium, zur Erzeugung eines Tiltwinkels im FK-Medium durch in situ-Polymerisation der erfindungsgemäßen polymerisierbaren Verbindung(en) in der PSA-Anzeige, vorzugsweise unter Anlegen eines elektrischen oder magnetischen Feldes.

Ein weiterer Gegenstand der Erfindung ist eine FK-Anzeige enthaltend eine oder mehrere erfindungsgemäße polymerisierbare Verbindungen oder ein erfindungsgemäßes FK-Medium, insbesondere eine PS- oder PSA-Anzeige, besonders bevorzugt eine PSA-VA-, PSA-OCB-, PSA-IPS-, PSA-FFS-, PSA-Posi-VA- oder PSA-TN-Anzeige.

Ein weiterer Gegenstand der Erfindung ist eine FK-Anzeige des PS- oder PSA-Typs, enthaltend eine FK-Zelle mit zwei Substraten und zwei Elektroden, wobei mindestens ein Substrat lichtdurchlässig ist und mindestens ein Substrat eine oder zwei Elektroden aufweist, sowie einer zwischen den Substraten befindlichen Schicht eines FK-Mediums enthaltend eine polymerisierte Komponente und eine niedermolekulare Komponente, wobei die polymerisierte Komponente erhältlich ist durch Polymerisation einer oder mehrerer polymerisierbarer Verbindungen zwischen den Substraten der FK-Zelle im FK-Medium, vorzugsweise unter Anlegen einer elektrischen Spannung an die Elektroden, wobei mindestens eine der polymerisierbaren Verbindungen aus erfindungsgemäßen polymerisierbaren Verbindungen ausgewählt ist.

Ein weiterer Gegenstand der Erfindung ist ein Verfahren zur Herstellung einer FK-Anzeige wie vor- und nachstehend beschrieben, indem man ein FK-Medium, enthaltend eine oder mehrere niedermolekulare flüssigkristalline Verbindungen oder eine FK-Hostmischung wie vor- und nachstehend beschrieben sowie eine oder mehrere erfindungsgemäße polymerisierbare Verbindungen, in eine FK-Zelle mit zwei Substraten und zwei Elektroden wie vor- und nachstehend beschrieben füllt, und die polymerisierbaren Verbindungen, vorzugsweise unter Anlegen einer elektrischen Spannung an die Elektroden, polymerisiert.

Die erfindungsgemäßen PS- und PSA-Anzeigen weisen zwei Elektroden, vorzugsweise in Form von transparenten Schichten, auf, wobei diese auf einem oder beiden der Substrate aufgebracht sind, die die FK-Zelle bilden. Dabei ist entweder jeweils eine Elektrode auf je einem der beiden Substrate aufgebracht, wie zum Beispiel in erfindungsgemäßen PSA-VA-, PSA-OCB- oder PSA-TN-Anzeigen, oder beide Elektroden sind auf nur einem der beiden Substrate aufgebracht, während das andere Substrat keine Elektrode aufweist, wie zum Beispiel in erfindungsgemäßen PSA-Posi-VA-, PSA-IPS- oder PSA-FFS-Anzeigen.

Weiterer Gegenstand der Erfindung sind neue erfindungsgemäße polymerisierbareVerbindungen, Verfahren zu ihrer Herstellung, sowie in diesen Verfahren verwendete oder daraus erhaltene neue Zwischenprodukte.

Vor- und nachstehend gelten folgende Bedeutungen:

Die Begriffe "Tilt" und "Tiltwinkel" beziehen sich auf eine gekippte oder geneigte Orientierung der FK-Moleküle eines FK-Mediums relativ zu den Oberflächen der Zelle in einer FK-Anzeige (hier vorzugsweise einer PS- oder PSA-Anzeige). Der Tiltwinkel bezeichnet dabei den durchschnittlichen Winkel (<90°) zwischen den Moleküllängsachsen der FK-Moleküle (FK-Direktor) und der Oberfläche der planparallelen Trägerplatten, welche die FK-Zelle bilden. Ein niedriger Wert des Tiltwinkels (d.h. eine große Abweichung vom 90°-Winkel) entspricht dabei einem großen Tilt. Eine geeignete Methode zur Messung des Tiltwinkels findet sich in den Beispielen. Soweit nicht anders angegeben, beziehen sich vor- und nachstehend offenbarte Werte des Tiltwinkels auf diese Messmethode.

Der Begriff "mesogene Gruppe" ist dem Fachmann bekannt und in der Literatur beschrieben, und bedeutet eine Gruppe, die durch die Anisotropie ihrer anziehenden und abstoßenden Wechselwirkungen wesentlich dazu beiträgt, in niedermolekularen oder polymeren Substanzen eine Flüssigkristall(FK-)Phase hervorzurufen. Verbindungen enthaltend mesogene Gruppen (mesogene Verbindungen) müssen nicht unbedingt selbst eine FK-Phase aufweisen. Es ist auch möglich, dass mesogene Verbindungen FK-Phasenverhalten nur nach Vermischung mit anderen Verbindungen und/oder nach Polymerisation zeigen. Typische mesogene Gruppen sind beispielsweise starre stäbchen- oder scheibchenförmige Einheiten. Ein Überblick über die im Zusammenhang mit mesogenen bzw. FK-Verbindungen verwendeten Begriffe und Definitionen findet sich in Pure Appl. Chem. 73(5), 888 (2001) und C. Tschierske, G. Pelzl, S. Diele, Angew. Chem. 2004, 116, 6340-6368.

Der Begriff "Abstandsgruppe" (engl. "spacer" oder "spacer group"), vor- und nachstehend auch als "Sp" bezeichnet, ist dem Fachmann bekannt und in der Literatur beschrieben, siehe beispielsweise Pure Appl. Chem. 73(5), 888 (2001) und C. Tschierske, G. Pelzl, S. Diele, Angew. Chem. 2004, 116, 6340-6368. Falls nicht anders angegeben, bezeichnet der Begriff "Abstandsgruppe" bzw. "Spacer" vor- und nachstehend eine flexible Gruppe, die in einer polymerisierbaren mesogenen Verbindung die mesogene Gruppe und die polymerisierbare(n) Gruppe(n) miteinander verbindet.

Der Begriff "reaktives Mesogen" oder "RM" bezeichnet eine Verbindung enthaltend eine mesogene Gruppe und eine oder mehrere funktionelle Gruppen, die zur Polymerisation geeignet sind (auch als polymerisierbare Gruppe oder Gruppe P bezeichnet).

Die Begriffe "niedermolekulare Verbindung" und "unpolymerisierbare Verbindung" bezeichnen, üblicherweise monomere, Verbindungen, die keine funktionelle Gruppe aufweisen, welche zur Polymerisation unter den üblichen dem Fachmann bekannten Bedingungen, insbesondere unter den zur Polymerisation der RMs verwendeten Bedingungen, geeignet ist.

"Halogen" bedeutet F, Cl, Br oder I.

Besonders bevorzugte erfindungsgemäße polymerisierbare Verbindungen sind solche der Formel I 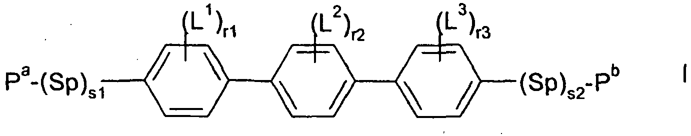 worin die einzelnen Reste folgende Bedeutung besitzen
- P^{a}, P^{b}: jeweils unabhängig voneinander eine polymerisierbare Gruppe,
- Sp: bei jedem Auftreten gleich oder verschieden eine Abstandsgruppe,
- L¹, L², L³: jeweils unabhängig voneinander F oder Cl, einer oder mehrere der Reste L¹, L² und L³ auch vollständig oder teilweise fluoriertes Alkyl oder Alkoxy mit 1, 2 oder 3 C-Atomen, P^{a} oder P^{a}-Sp-, wobei mindestens einer der Reste L¹, L² und L³ F oder Cl, vorzugsweise F, bedeutet,
- r1, r2, r3: jeweils unabhängig voneinander 0, 1, 2, 3 oder 4 wobei r1 +r2+r3 ≥1,
- s1, s2: jeweils unabhängig voneinander 0 oder 1.

Die polymerisierbare Gruppe P^{a,b} ist eine Gruppe, die für eine Polymerisationsreaktion, wie beispielsweise die radikalische oder ionische Kettenpolymerisation, Polyaddition oder Polykondensation, oder für eine polymeranaloge Umsetzung, beispielsweise die Addition oder Kondensation an eine Polymerhauptkette, geeignet ist. Besonders bevorzugt sind Gruppen für die Kettenpolymerisation, insbesondere solche enthaltend eine C=C-Doppelbindung oder -C≡C-Dreifachbindung, sowie zur Polymerisation unter Ringöffnung geeignete Gruppen wie beispielsweise Oxetan- oder Epoxygruppen

Bevorzugte Gruppen P^{a,b} sind ausgewählt aus der Gruppe bestehend aus CH₂=CW¹-CO-O-, CH₂=CW¹-CO-, 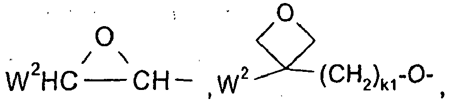 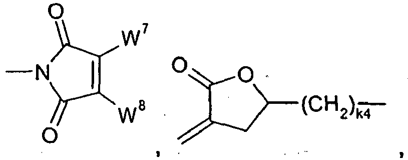 CH₂=CW²-(O)ₖ₃-, CW¹=CH-CO-(O)ₖ₃-, CW¹=CH-CO-NH-, CH₂=CW¹-CO-NH-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, CH₂=CH-(CO)ₖ₁-Phe-(O)ₖ₂-, Phe-CH=CH-, HOOC-, OCN-, und W⁴W⁵W⁶Si-, worin W¹ H, F, Cl, CN, CF₃, Phenyl oder Alkyl mit 1 bis 5 C-Atomen, insbesondere H, F, Cl oder CH₃ bedeutet, W² und W³ jeweils unabhängig voneinander H oder Alkyl mit 1 bis 5 C-Atomen, insbesondere H, Methyl, Ethyl oder n-Propyl bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet, welches optional mit einem oder mehreren, von P-Sp- verschiedenen Resten L wie oben definiert substiuiert ist, k₁, k₂ und k₃ jeweils unabhängig voneinander 0 oder 1 bedeuten, k₃ vorzugsweise 1 bedeutet, und k₄ eine ganze Zahl von 1 bis 10 bedeutet.

Besonders bevorzugte Gruppen P^{a,b} sind ausgewählt aus der Gruppe bestehend aus CH₂=CW¹-CO-O-, CH₂=CW¹-CO-, 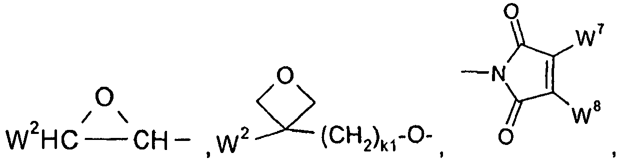 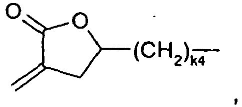 CH₂=CW²-O-, CW¹=CH-CO-(O)ₖ₃-, CW¹=CH-CO-NH-, CH₂=CW¹-CO-NH-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, CH₂=CW¹-CO-NH-, CH₂=CH-(COO)ₖ₁-Phe-(O)ₖ₂-, CH₂=CH-(CO)ₖ₁-Phe-(O)ₖ₂-, Phe-CH=CH- und W⁴W^{S}W⁶Si-, worin W¹ H, F, Cl, CN, CF₃, Phenyl oder Alkyl mit 1 bis 5 C-Atomen, insbesondere H, F, Cl oder CH₃ bedeutet, W² und W³ jeweils unabhängig voneinander H oder Alkyl mit 1 bis 5 C-Atomen, insbesondere H, Methyl, Ethyl oder n-Propyl bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet, k₁, k₂ und k₃ jeweils unabhängig voneinander 0 oder 1 bedeuten, k₃ vorzugsweise 1 bedeutet, und k₄ eine ganze Zahl von 1 bis 10 bedeutet.

Ganz besonders bevorzugte Gruppen P^{a,b} sind sind ausgewählt aus der Gruppe bestehend aus CH₂=CW¹-CO-O-, insbesondere CH₂=CH-CO-O-, CH₂=C(CH₃)-CO-O- und CH₂=CF-CO-O-, ferner CH₂=CH-O-, (CH₂=CH)₂CH-O-CO-, (CH₂=CH)₂CH-O-, 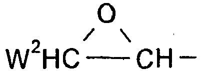 und

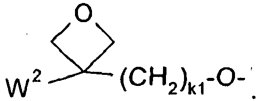

Weitere ganz besonders bevorzugte Gruppen P^{a,b} sind ausgewählt aus der Gruppe bestehend aus Vinyloxy-, Acrylat-, Methacrylat-, Fluoracrylat-, Chloracrylat-, Oxetan- und Epoxygruppen, und bedeuten besonders bevorzugt eine Acrylat- oder Methacrylatgruppe.

Bevorzugte Abstandsgruppen Sp sind ausgewählt aus der Formel Sp"-X", so dass der Rest "P^{a/b}-Sp- " der Formel "P^{a/b}-Sp"-X"- " entspricht, wobei
- Sp": Alkylen mit 1 bis 20, vorzugsweise 1 bis 12 C-Atomen bedeutet, welches optional durch F, Cl, Br, I oder CN ein- oder mehrfach substituiert ist, und worin auch eine oder mehrere nicht benachbarte CH₂-Gruppen jeweils unabhängig voneinander so durch -O-, -S-, -NH-, -N(R⁰)-, -Si(R⁰⁰R⁰⁰⁰)-, -CO-, -CO-O-, -O-CO-, -O-CO-O-, -S-CO-, -CO-S-, -N(R⁰⁰)-CO-O-, -O-CO-N(R⁰⁰)-, -N(R⁰⁰)-CO-N(R⁰⁰)-, -CH=CH- oder -C≡C- ersetzt sein können, dass O- und/oder S-Atome nicht direkt miteinander verknüpft sind,
- X": -O-, -S-, -CO-, -CO-O-, -O-CO-, -O-CO-O-, -CO-N(R⁰⁰)-, -N(R⁰⁰)-CO-, -N(R⁰⁰)-CO-N(R⁰⁰)-, -OCH₂-, -CH₂O- -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CY²=CY³-, -C≡C-, -CH=CH-CO-O-, -O-CO-CH=CH- oder eine Einfachbindung bedeutet,

R⁰⁰ und R⁰⁰⁰ jeweils unabhängig voneinander H oder Alkyl mit 1 bis 12 C-Atomen bedeuten, und

Y² und Y³ jeweils unabhängig voneinander H, F, Cl oder CN bedeuten.

X' ist vorzugsweise -O-, -S -CO-, -COO-, -OCO-, -O-COO-, -CO-NR⁰-, - NR⁰-CO-, -NR⁰-CO-NR⁰- oder eine Einfachbindung.

Typische Abstandsgruppen Sp" sind beispielsweise -(CH₂)ₚ₁-, -(CH₂CH₂O)_{q1}-CH₂CH₂-, -CH₂CH₂-S-CH₂CH₂-, -CH₂CH₂-NH-CH₂CH₂- oder -(SiR⁰⁰R⁰⁰⁰-O)p₁-, worin p1 eine ganze Zahl von 1 bis 12 ist, q1 eine ganze Zahl von 1 bis 3 ist, und R⁰⁰ und R⁰⁰⁰ die oben angegebenen Bedeutungen besitzen.

Besonders bevorzugte Gruppen -Sp"-X"- sind -(CH₂)ₚ₁-, -(CH₂)ₚ₁-O-, -(CH₂)ₚ₁-O-CO-, -(CH₂)ₚ₁-O-CO-O-, worin p1 und q1 die oben angebene Bedeutung haben.

Besonders bevorzugte Gruppen Sp" sind beispielsweise jeweils geradkettiges Ethylen, Propylen, Butylen, Pentylen, Hexylen, Heptylen, Octylen, Nonylen, Decylen, Undecylen, Dodecylen, Octadecylen, Ethylenoxyethylen, Methylenoxybutylen, Ethylenthioethylen, Ethylen-N-methyl-iminoethylen, 1-Methylalkylen, Ethenylen, Propenylen und Butenylen.

In einer weiteren bevorzugten Ausführungsform der Erfindung bedeuten P^{a} und/oder P^{b} in Formel I einen Rest mit zwei oder mehreren polymerisierbaren Gruppen (multifunktionelle polymerisierbare Reste). Geeignete Reste dieses Typs, sowie diese enthaltende polymerisierbare Verbindungen und ihre Herstellung sind beispielsweise in US 7,060,200 B1 oder US 2006/0172090 A1 beschrieben. Besonders bevorzugt sind multifunktionelle polymerisierbare Reste ausgewählt aus folgenden Formeln

-X-alkyl-CHP¹-CH₂-CH₂P² I*a

-X-alkyl-C(CH₂P)(CH₂P²)-CH₂P³ I*b

-X-alkyl-CHP¹CHP²-CH₂P³ I*c

-X-alkyl-C(CH₂P¹)(CH₂P²)-CₐₐH₂ₐₐ₊₁ I*d

-X-alkyl-CHP¹-CH₂P² I*e

-X-alkyl-CHP¹P² I*f

-X-alkyl-CP¹P²-CₐₐH₂ₐₐ₊₁ I*g

-X-alkyl-C(CH₂P)(CH₂P²)-CH₂OCH₂-C(CH₂P³)(CH₂P⁴)CH₂P⁵ I*h

-X-alkyl-CH((CH₂)ₐₐP¹)((CH₂)_{bb}P²) I*i

-X-alkyl-CHP¹CHP²-CₐₐH₂ₐₐ₊₁ I*k

-X'-alkyl-C(CH3)(CH₂P¹)(CH₂P²) I*m

worin
- alkyl: eine Einfachbindung oder geradkettiges oder verzweigtes Alkylen mit 1 bis 12 C-Atomen bedeutet, worin eine oder mehrere nicht benachbarte CH₂-Gruppen jeweils unabhängig voneinander durch -C(R⁰⁰)=C(R⁰⁰⁰)-, -C≡C-, -N(R⁰⁰)-, -O-, -S-, - CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl oder CN ersetzt sein können, wobei R⁰⁰ und R⁰⁰⁰ die oben angegebene Bedeutung haben,
- aa und bb: jeweils unabhängig voneinander 0, 1, 2, 3, 4, 5 oder 6 bedeuten,
- X: eine der für X' angegebenen Bedeutungen besitzt, und
- P¹⁻⁵: jeweils unabhängig voneinander eine der für P^{a} angegebenen Bedeutungen besitzen.

In den vor- und nachstehend beschriebenen Formeln bedeutet 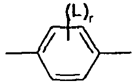 vorzugsweise 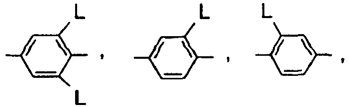 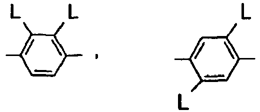 oder 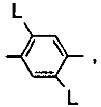 worin L bei jedem Auftreten gleich oder verschieden eine der vor- und nachstehend angegebenen Bedeutungen hat, und vorzugsweise F, Cl, CN, NO₂, CH₃, C₂H₅, C(CH₃)₃, CH(CH₃)₂, CH₂CH(CH₃)C₂H₅, OCH₃, OC₂H₅, COCH₃, COC₂H₅, COOCH₃, COOC₂H₅, CF₃, OCF₃, OCHF₂, OC₂F₅ oder P-Sp-, besonders bevorzugt F, Cl, CN, CH₃, C₂H₅, OCH₃, COCH₃, OCF₃ oder P-Sp-, ganz besonders bevorzugt F, Cl, CH₃, OCH₃, COCH₃ oder OCF₃ bedeutet.

Besonders bevorzugte Verbindungen der Formel I sowie deren vor- und nachstehend angegebenen Unterformeln sind solche worin
- L¹, L² und L³ von P^{a}-(Sp)ₛ₁- und P^{b}-(Sp)ₛ₂-verschieden sind,
- einer oder mehrere, vorzugsweise alle Reste L¹, L² und L³ F und/oder Cl, besonders bevorzugt F, bedeuten,
- die Summe r1+r2+r3 1, 2, 3 oder 4 ist,
- einer oder zwei von r1, r2 und r3 0 bedeuten,
- r2 1 oder 2 bedeutet,
- r1 und/oder r3 1 bedeutet,
- s1 und s2 jeweils 0 bedeuten
- s1 und s2 jeweils 1 bedeuten,
- s1 1 und s2 0 bedeutet oder s1 0 und s2 1 bedeutet,
- einer der Indices s1 und s2 0 bedeutet, und der andere der Indices s1 und s2 1 bedeutet.

Ganz besonders bevorzugte Verbindungen der Formel I sind ausgewählt aus der Gruppe bestehend aus den folgenden Unterformeln 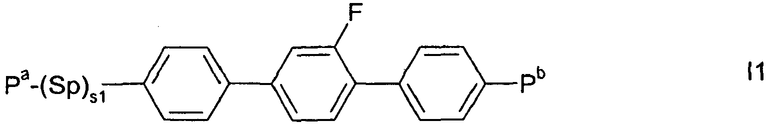 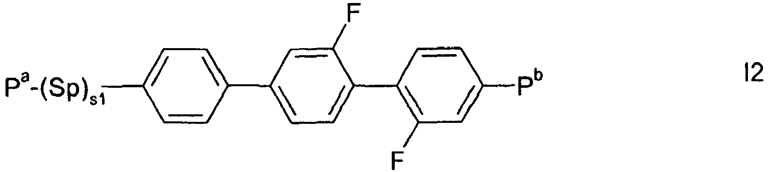 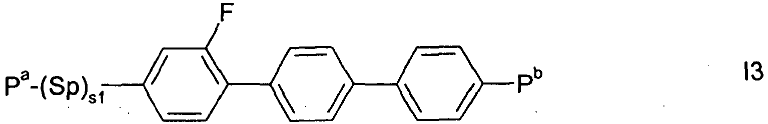 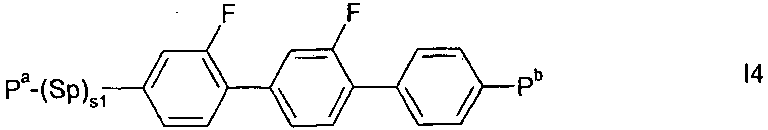 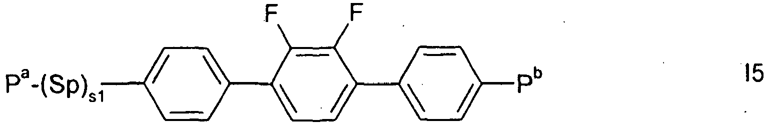 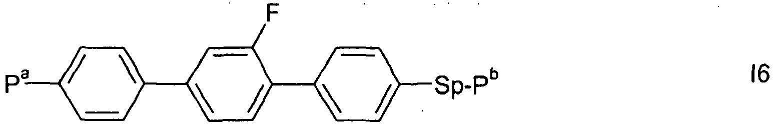 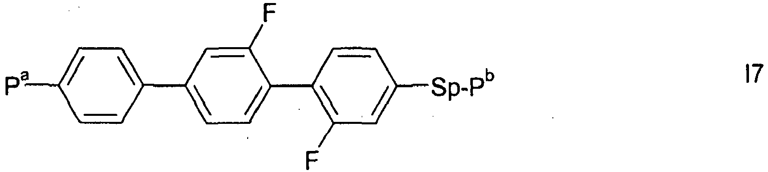 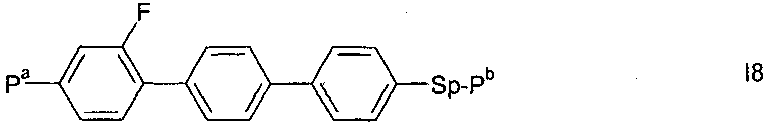 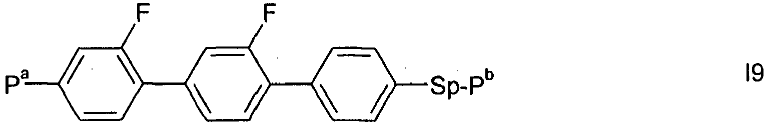 worin P^{a}, P^{b}, Sp und s1 eine der vor-und nachstehend angegebenen Bedeutungen haben, und s1 vorzugsweise 1 ist.

P^{a} und P^{b} bedeuten in den Verbindungen der Formel I sowie deren Unterformeln vorzugsweise Acrylat oder Methacrylat, ferner Fluoracrylat.

Sp bedeutet in den Verbindungen der Formeln I und deren Unterformeln vorzugsweise -(CH₂)ₚ₁-, -(CH₂)p₁-O-, -(CH₂)ₚ₁-O-CO- oder -(CH₂)ₚ₁-O-CO-O-, worin p1 eine ganze Zahl von 1 bis 12, vorzugsweise von 1 bis 6, besonders bevorzugt 1, 2 oder 3 bedeutet, wobei diese Gruppen so mit P^{a} oder P^{b} verknüpft sind, dass O-Atome nicht direkt benachbart sind.

Ein weiterer Gegenstand der Erfindung sind neue Verbindungen der Formel I sowie deren Unterformeln wie vor- und nachstehend definiert, worin s1 1 und s2 O bedeutet, oder s1 O und s2 1 bedeutet.

Ein weiterer Gegenstand der Erfindung sind neue Zwischenprodukte zur Herstellung von Verbindungen der Formel I, ausgewählt aus Formel II 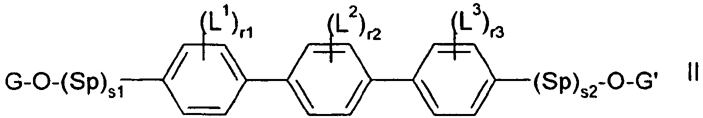 worin Sp, L¹, L², L³, r1, r2, r3, s1 und s2 die in Formel I oder vor- und nachstehend angegebene Bedeutung besitzen, wobei s1 und s2 nicht beide gleichzeitig 1 bedeuten, und G und G' jeweils unabhängig voneinander ein H-Atom oder eine Schutzgruppe bedeuten.

Geeignete Schutzgruppen G sind dem Fachmann bekannt. Bevorzugte Schutzguppen sind Alkyl, Acyl und Alkylsilyl- oder Arylsilylgruppen, 2-Tetrahydropyranyl oder Methoxymethyl.

Besonders bevorzugte Zwischenprodukte der Formel II sind ausgewählt aus der Gruppe bestehend aus den oben genannten Unterformeln 11-19, worin P^{a} jeweils G-O- und P^{b} jeweils -O-G' bedeutet, wobei G und G' vorzugsweise H bedeuten.

In einer weiteren bevorzugten Ausführungsform der Erfindung enthalten die Abstandsgruppen in den erfindungsgemäßen polymerisierbaren Verbindungen keine C-C-Dreifachbindung (-C≡C-), bzw. Sp in Formel I und Formel II sowie deren Unterformeln, sowie Sp" wie oben beschrieben, enthalten keine C-C-Dreifachbindung (-C≡C-).

Besonders geeignete und bevorzugte Verfahren zur Herstellung von Verbindungen und Zwischenprodukten der Formel I und II sowie deren Unterformeln sind in folgenden Schemata beispielhaft dargestellt und enthalten vorzugsweise einen oder mehrere der nachfolgend beschriebenen Schritte.

Die Verbindungen und Zwischenprodukte der Formel I und II sowie deren Unterformeln können in Analogie zu dem Fachmann bekannten und in Standardwerken der organischen Chemie beschriebenen Verfahren, wie beispielsweise in Houben-Weyl, Methoden der organischen Chemie, Thieme-Verlag, Stuttgart, hergestellt werden.

Beispielsweise erfolgt die Synthese von Verbindungen der Formel I durch Veresterung oder Veretherung der Zwischenprodukte der Formel II mit entsprechenden Säuren, Säurederivaten, oder halogenierten Verbindungen enthaltend eine Gruppe P. Wie in Schema 1 beispielhaft dargestellt (worin R H oder CH₃ bedeutet), können Verbindungen der Formel I, worin P^{a} und P^{b} eine Acrylat- oder Methacrylatgruppe bedeuten, durch Veresterung der entsprechenden Alkohole der Formel II, worin G=G'=H mit Säurederivaten wie zum Beispiel (Meth)acrylsäurechlorid oder (Meth)acrylsäureanhydrid in Gegenwart einer Base und ggf. 4-(N,N-Dimethylamino)pyridin (DMAP) erhalten werden. Weiterhin können die Alkohole auch mit (Meth)acrylsäure in Gegenwart eines wasserentziehenden Mittels verestert werden, beispielsweise nach Steglich mit Dicyclohexylcarbodiimid (DCC). 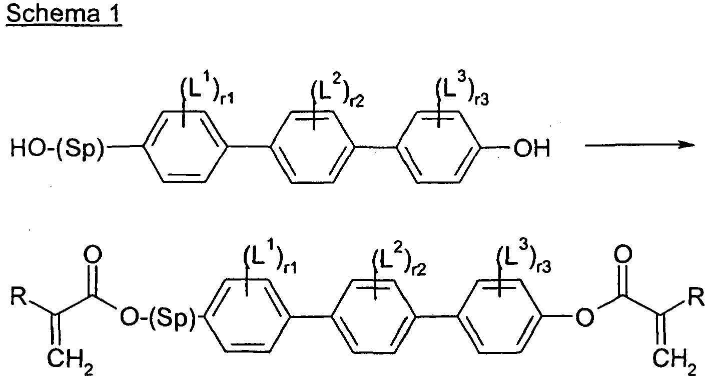 (R = H oder CH₃, "(Sp)" = Sp oder Einfachbindung)

Die Synthese der Zwischenprodukte der Formel II erfolgt beispielsweise durch übergangsmetallkatalysierte Kreuzkupplung (siehe Schema 2) von Arylhalogeniden (Hal = Cl, Br, I, OTf) mit Arylmetallverbindungen, beispielsweise durch Suzuki-Kupplung (M = B(OH)₂, B(OR)₂), Negishi- (M = ZnHal) oder Kumada-Kupplung (M = MgHal).

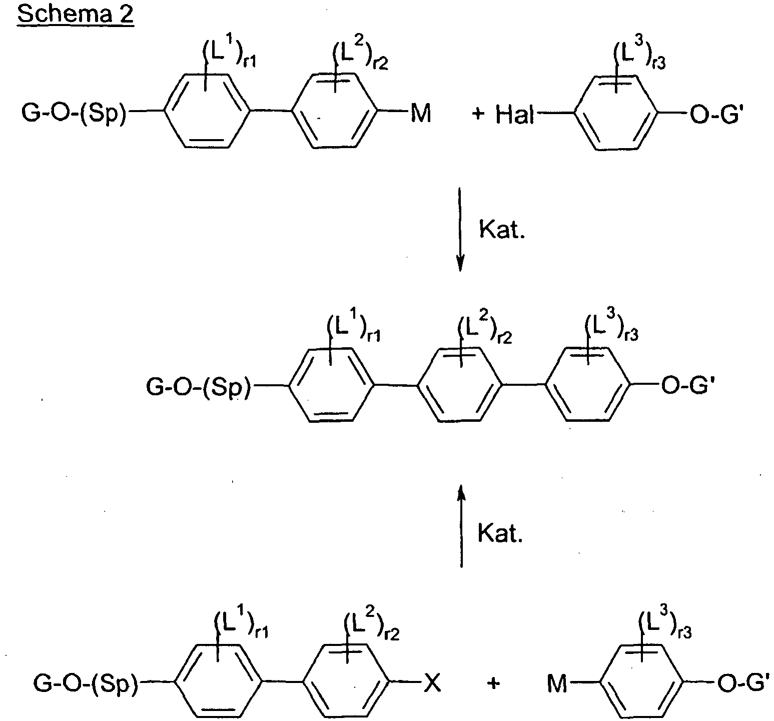

Dabei sind die Arylmetallverbindungen ihrerseits aus Arylhalogeniden zugänglich, entweder direkt durch Umsetzung mit Magnesium zu Grignardverbindungen oder durch Halogen-Lithium-Austausch mit Butyllithium und anschließende Transmetallierung mit Zink- oder Magnesiumsalzen. Umsetzung der Lithiumverbindungen mit Trialkylboraten liefert Boronsäureester, aus denen durch Hydrolyse die freien Boronsäuren erhalten werden können.

In Analogie dazu lassen sich die in Schema 2 dargestellten Biphenyl-Synthesebausteine herstellen (siehe Schema 3), indem Dihalogenaromaten mit Arylmetallverbindungen in einer Kreuzkupplungsreaktion umgesetzt werden. Die Substituenten Hal und Hal' können dabei gleich oder verschieden sein.

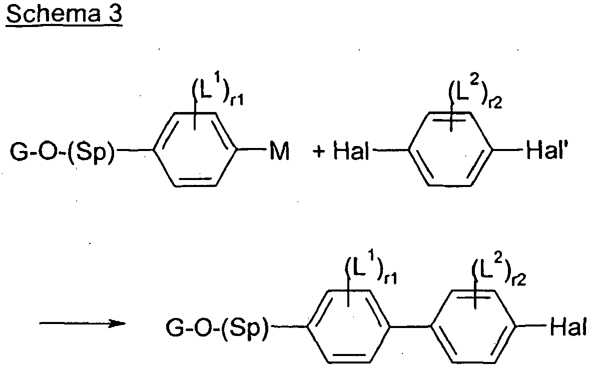

Zur Herstellung von PSA-Anzeigen werden die polymerisierbaren Verbindungen im FK-Medium zwischen den Substraten der FK-Anzeige unter Anlegen einer Spannung durch in-situ-Polymerisation polymerisiert oder vernetzt (falls eine Verbindung zwei oder mehr polymerisierbare Gruppen enthält). Die Polymerisation kann in einem Schritt durchgeführt werden. Es ist auch möglich, zunächst in einem ersten Schritt die Polymerisation unter Anlegen einer Spannung durchzuführen, um einen pretilt-Winkel zu erzeugen, und anschließend in einem zweiten Polymerisationsschritt ohne anliegende Spannung die im ersten Schritt nicht abreagierten Verbindungen zu polymerisieren bzw. zu vernetzen ("end curing").

Geeignete und bevorzugte Polymerisationsmethoden sind beispielsweise die thermische oder Photopolymerisation, vorzugsweise Photopolymerisation, insbesondere UV-Photopolymerisation. Dabei können gegebenfalls auch ein oder mehrere Initiatoren zugesetzt werden. Geeignete Bedingungen für die Polymerisation, sowie geeignete Arten und Mengen der Initiatoren, sind dem Fachmann bekannt und in der Literatur beschrieben. Für die radikalische Polymerisation eignen sich zum Beispiel die kommerziell erhältlichen Photoinitiatoren Irgacure651®, Irgacure184®, Irgacure907®, Irgacure369®, oder Darocure1173® (Ciba AG). Falls ein Initiator eingesetzt wird, beträgt dessen Anteil vorzugsweise 0,001 bis 5 Gew.-%, besonders bevorzugt 0,001 bis 1 Gew.-%.

Die erfindungsgemäßen polymerisierbaren Verbindungen eignen sich auch für die Polymerisation ohne Initiator, was erhebliche Vorteile mit sich bringt, wie beispielsweise geringere Materialkosten und insbesondere eine geringere Verunreinigung des FK-Mediums durch mögliche Restmengen des Initiators oder dessen Abbauprodukte. Die Polymerisation kann somit auch ohne Zusatz eines Initiators erfolgen. Somit enthält das FK-Medium in einer bevorzugten Ausführungsform keinen Polymerisationsinitiator.

Die polymerisierbare Komponente A) oder das FK-Medium können auch einen oder mehrere Stabilisatoren enthalten, um eine unerwünschte spontane Polymerisation der RMs, beispielsweise während der Lagerung oder des Transports, zu verhindern. Geeignete Arten und Mengen der Stabilisatoren sind dem Fachmann bekannt und in der Literatur beschrieben. Besonders geeignet sind zum Beispiel die kommerziell erhältlichen Stabilisatoren der Serie Irganox® (Ciba AG), wie beispielsweise Irganox® 1076. Falls Stabilisatoren eingesetzt werden, beträgt deren Anteil, bezogen auf die Gesamtmenge der RMs beziehungsweise der polymerisierbaren Komponente A), vorzugsweise 10 -10,000 ppm, besonders bevorzugt 50 - 500 ppm.

Die erfindungsgemäßen FK-Medien zur Verwendung in PSA-Anzeigen enthalten vorzugsweise < 5 Gew.-%, besonders bevorzugt < 1 Gew.-%, ganz besonders bevorzugt < 0.5 Gew.-% an polymerisierbaren Verbindungen, insbesondere polymerisierbaren Verbindungen der oben genannten Formeln I und deren Unterformeln.

Besonders bevorzugt sind FK-Medien enthaltend eine, zwei oder drei erfindungsgemäße polymerisierbare Verbindungen.

Ferner bevorzugt sind FK-Medien, worin die polymerisierbare Komponente (Komponente A) ausschließlich erfindungsgemäße polymerisierbare Verbindungen enthält.

Ferner bevorzugt sind FK-Medien, worin Komponente B) eine FK-Verbindung oder eine FK-Mischung ist, die eine nematische Flüssigkristallphase aufweist.

Ferner bevorzugt sind achirale erfindungsgemäße polymerisierbare Verbindungen, sowie FK-Medien worin die Verbindungen der Komponente A) und/oder B) ausschließlich aus der Gruppe bestehend aus achiralen Verbindungen ausgewählt sind.

Ferner bevorzugt sind FK-Medien, worin die polymerisierbare Komponente bzw. Komponente A) eine oder mehrere erfindungsgemäße polymerisierbare Verbindungen mit einer polymerisierbaren Gruppe (monoreaktiv) und eine oder mehrere erfindungsgemäße polymerisierbare Verbindungen mit zwei oder mehr, vorzugsweise zwei polymerisierbaren Gruppen (di- oder multireaktiv) enthält.

Ferner bevorzugt sind PSA-Anzeigen und FK-Medien, worin die polymerisierbare Komponente bzw. Komponente A) ausschließlich erfindungsgemäße polymerisierbare Verbindungen mit zwei polymerisierbaren Gruppen (direaktiv) enthält.

Der Anteil der polymerisierbaren Komponente bzw. Komponente A) in den erfindungsgemäßen FK-Medien ist vorzugsweise < 5%, besonders bevorzugt < 1 %, ganz besonders bevorzugt < 0.5 %.

Der Anteil der flüssigkristallinen Komponente bzw. Komponente B) in den erfindungsgemäßen FK-Medien ist vorzugsweise > 95%, besonders bevorzugt > 99%.

Die erfindungsgemäßen polymerisierbaren Verbindungen können einzeln polymerisiert werden, es können aber auch Mischungen polymerisiert werden, welche zwei oder mehr erfindungsgemäße polymerisierbare Verbindungen enthalten, oder Mischungen enthaltend eine oder mehrere erfindungsgemäße polymerisierbare Verbindungen und eine oder mehrere weitere polymerisierbare Verbindungen (Comonomere), welche vorzugsweise mesogen oder flüssigkristallin sind. Bei Polymerisation solcher Mischungen entstehen Copolymere. Die vor- und nachstehend genannten polymerisierbaren Mischungen sind ein weiterer Gegenstand der Erfindung. Die polymerisierbaren Verbindungen und Comonomere sind mesogen oder nicht-mesogen, vorzugsweise mesogen oder flüssigkristallin.

Geeignete und bevorzugte mesogene Comonomere, besonders für die Verwendung in PSA-Anzeigen, sind beispielsweise ausgewählt aus den folgenden Formeln: 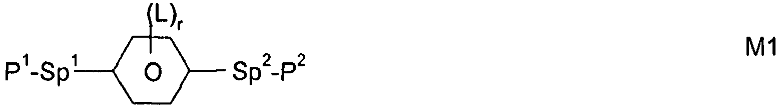 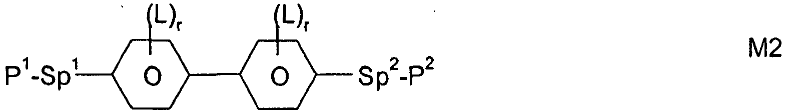 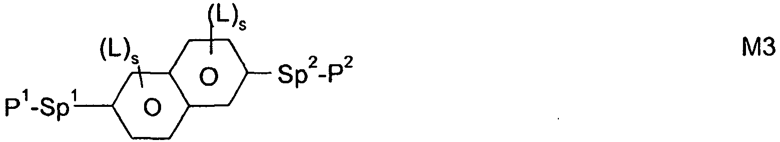 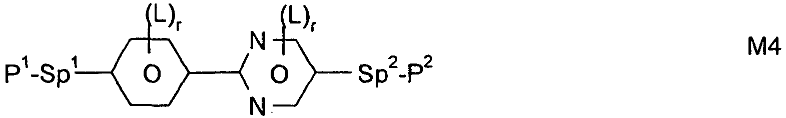 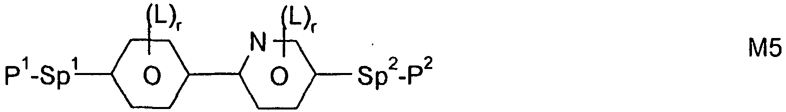 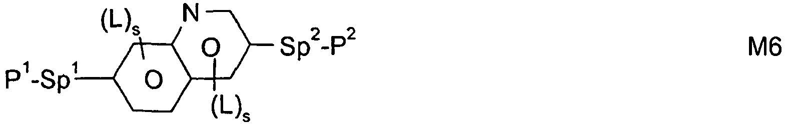 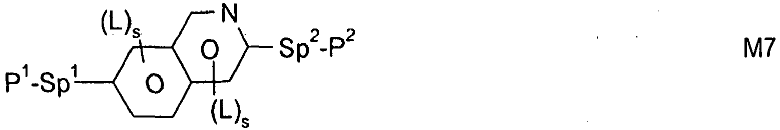 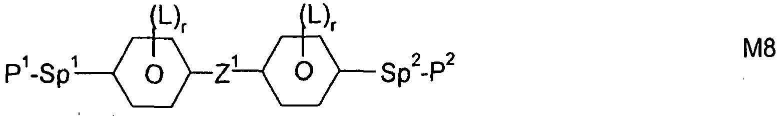 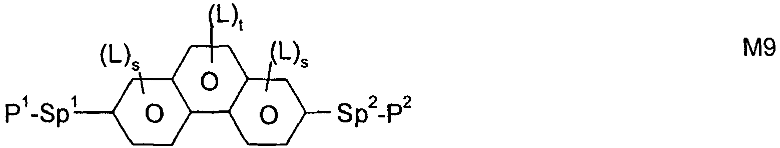 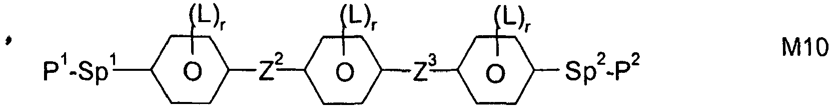 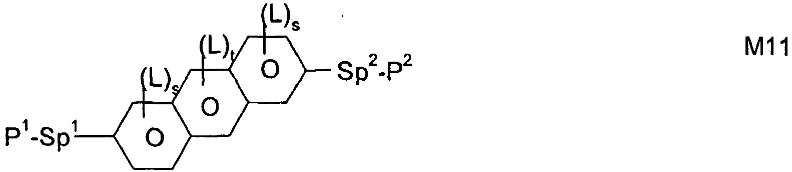 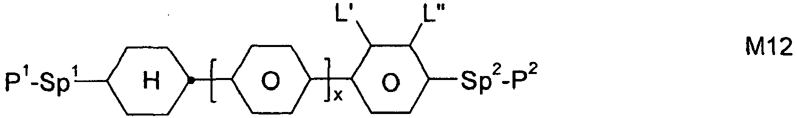 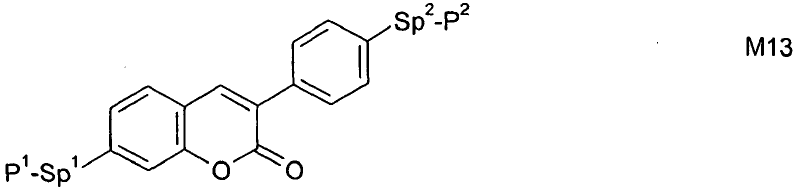 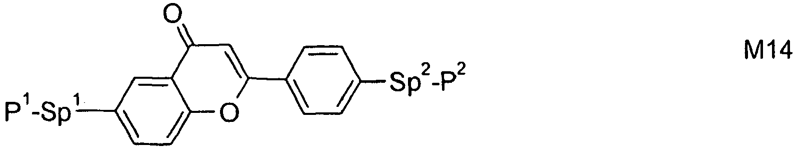 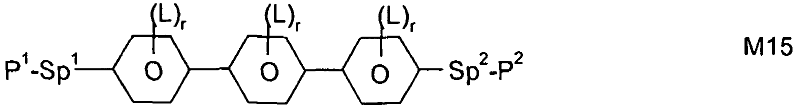 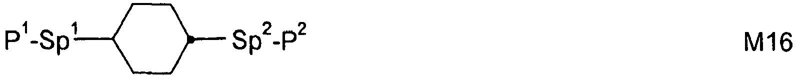 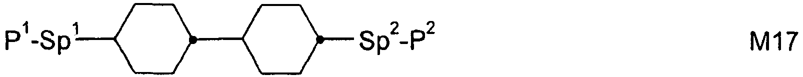 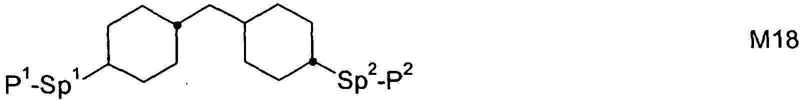 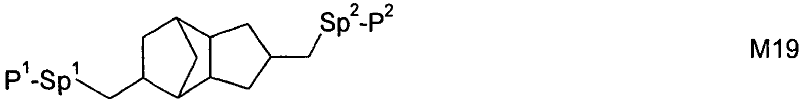 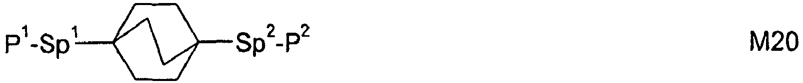 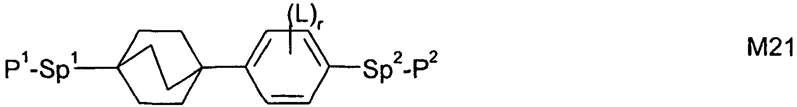 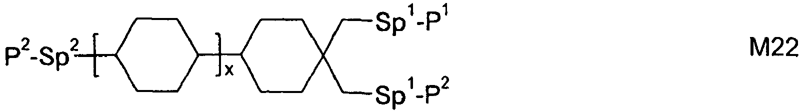 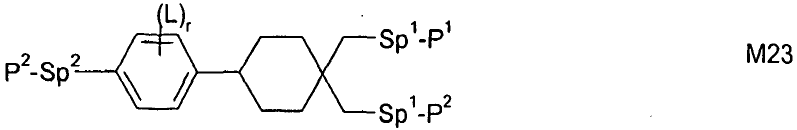 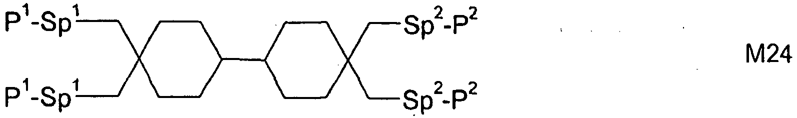 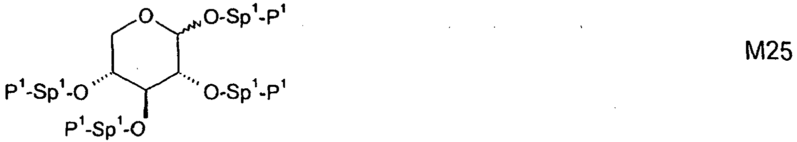 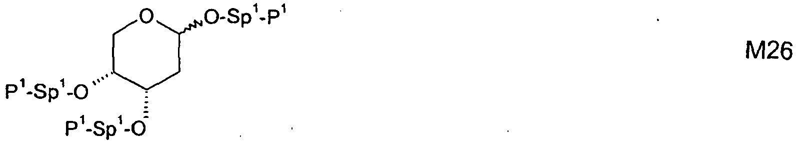 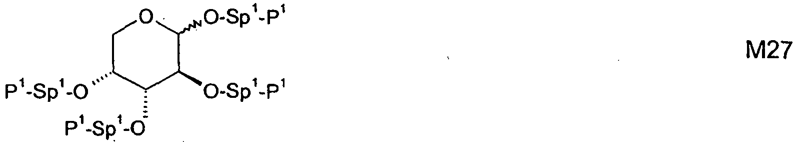 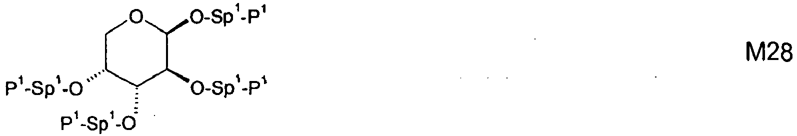 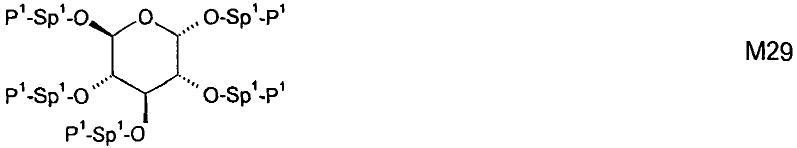 worin die einzelnen Reste folgende Bedeutung besitzen:
- P¹ und P²: jeweils unabhängig voneinander eine polymerisierbare Gruppe, vorzugsweise mit einer der vor- und nachstehend für P angegebenen Bedeutungen, besonders bevorzugt eine Acrylat-, Methacrylat-, Fluoracrylat-, Oxetan-, Vinyloxy- oder Epoxygruppe,
- Sp¹ und Sp²: jeweils unabhängig voneinander eine Einfachbindung oder eine Abstandsgruppe, vorzugsweise mit einer der vor- und nachstehend für Sp angegebenen Bedeutungen, und besonders bevorzugt -(CH₂)ₚ₁-, -(CH₂)ₚ₁-O-, -(CH₂)ₚ₁-CO-O- oder -(CH₂)ₚ₁-O-CO-O- bedeuten, worin p1 eine ganze Zahl von 1 bis 12 ist, und wobei in den letztgenannten Gruppen die Verknüpfung zur benachbarten Ring über das O-Atom erfolgt,
wobei auch einer oder mehrere der Reste P¹-Sp¹- und P²-Sp²- R^{aa} bedeuten können, mit der Maßgabe dass mindestens einer der vorhandenen Reste P¹-Sp¹- und P²-Sp¹- nicht R^{aa} bedeutet,
- R^{aa}: H, F, Cl, CN oder geradkettiges oder verzweigtes Alkyl mit 1 bis 25 C-Atomen, worin auch eine oder mehrere nicht benachbarte CH₂-Gruppen jeweils unabhängig voneinander durch C(R⁰)=C(R⁰⁰)-, -C≡C-, -N(R⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, CN oder P¹-Sp¹- ersetzt sein können, besonders bevorzugt geradkettiges oder verzweigtes, optional ein- oder mehrfach fluoriertes, Alkyl, Alkoxy, Alkenyl, Alkinyl, Alkylcarbonyl, Alkoxycarbonyl, Alkylcarbonyloxy oder Alkoxycarbonyloxy mit 1 bis 12 C-Atomen (wobei die Alkenyl- und Alkinylreste mindestens zwei und die verzweigten Reste mindestens drei C-Atome aufweisen),
- R⁰, R⁰⁰: jeweils unabhängig voneinander und bei jedem Auftreten gleich oder verschieden H oder Alkyl mit 1 bis 12 C-Atomen,
- R^{y} und R^{z}: jeweils unabhängig voneinander H, F, CH₃ oder CF₃,
- Z¹: -O-, -CO-, -C(R^{y}R^{z})-,oder -CF₂CF₂-,
- Z² und Z³: jeweils unabhängig voneinander -CO-O-, -O-CO-, -CH₂O-, -OCH₂-, -CF₂O-, -OCF₂-, oder -(CH₂)ₙ-, wobei n 2, 3 oder 4 ist,
- L: bei jedem Auftreten gleich oder verschieden F, Cl, CN, oder geradkettiges oder verzweigtes, optional ein- oder mehrfach fluoriertes, Alkyl, Alkoxy, Alkenyl, Alkinyl, Alkylcarbonyl, Alkoxycarbonyl, Alkylcarbonyloxy oder Alkoxycarbonyloxy mit 1 bis 12 C-Atomen vorzugsweise F,
- L' und L": jeweils unabhängig voneinander H, F oder Cl,
- r: 0, 1, 2, 3 oder 4,
- s: 0, 1, 2 oder 3,
- t: 0, 1 oder 2,
- x: 0 oder 1.

In den Verbindungen der Formeln M1 bis M29 bedeutet 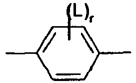 vorzugsweise 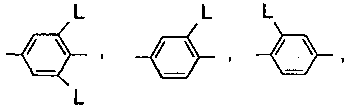 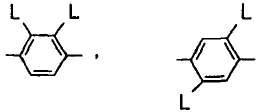 oder 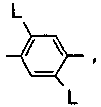 worin L bei jedem Auftreten gleich oder verschieden eine der vor- und nachstehend angegebenen Bedeutungen hat, und vorzugsweise F, Cl, CN, NO₂, CH₃, C₂H₅, C(CH₃)₃, CH(CH₃)₂, CH₂CH(CH₃)C₂H₅, OCH₃, OC₂H₅, COCH₃, COC₂H₅, COOCH₃, COOC₂H₅, CF₃, OCF₃, OCHF₂, OC₂F₅ oder P-Sp-, besonders bevorzugt F, Cl, CN, CH₃, C₂H₅, OCH₃, COCH₃, OCF₃ oder P-Sp-, ganz besonders bevorzugt F, Cl, CH₃, OCH₃, COCH₃ oder OCF₃ , insbesondere F oder CH₃ bedeutet.

Die FK-Medien zur Verwendung in den erfindungsgemäßen FK-Anzeigen enthalten, neben den oben beschriebenen polymerisierbaren Verbindungen, eine FK-Mischung ("Host-Mischung") enthaltend eine oder mehr, vorzugsweise zwei oder mehr niedermolekulare (d.h. monomere bzw. unpolymerisierte) Verbindungen. Letztere sind stabil bzw. unreaktiv gegenüber einer Polymerisationsreaktion unter den zur Polymerisation der polymerisierbaren Verbindungen verwendeten Bedingungen. Prinzipiell eignet sich als Host-Mischung jede zur Verwendung in herkömmlichen VA- und OCB-Anzeigen geeignete FK-Mischung. Geeignete FK-Mischungen sind dem Fachmann bekannt und in der Literatur beschrieben, beispielsweise Mischungen in VA-Anzeigen in EP 1 378 557 A1, und Mischungen für OCB-Anzeigen in EP 1 306 418 A1 und DE 102 24 046 A1.

Besonders bevorzugte FK-Anzeigen, FK-Host-Mischungen und FK-Medien werden im Folgenden genannt:

In einer ersten bevorzugten Ausführungsform enthält das FK-Medium eine FK-Host-Mischung basierend auf Verbindungen mit negativer dielektrischer Anisotropie. Solche FK-Medien eignen sich besonders für PSA-VA-Anzeigen. Besonders bevorzugte Ausführungsformen eines solchen FK-Mediums werden in den folgenden Abschnitten a)-x) genannt:
a) FK-Medium, welches eine oder mehrere Verbindungen der Formel CY und/oder PY enthält: 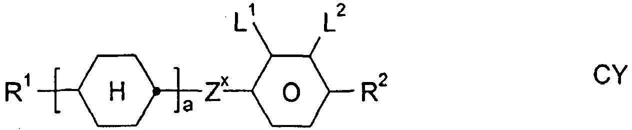 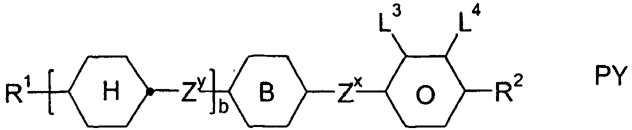 worin die einzelnen Reste folgende Bedeutung besitzen
   - a: 1 oder 2,
   - b: 0 oder 1,

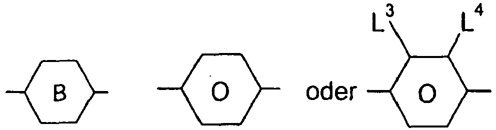

   - R¹ und R²: jeweils unabhängig voneinander Alkyl mit 1 bis 12 C-Atomen, wobei auch eine oder zwei nicht benachbarte CH₂-Gruppen durch -O-, -CH=CH-, -CO-, -OCO- oder - COO- so ersetzt sein können, dass O-Atome nicht direkt miteinander verknüpft sind, vorzugsweise Alkyl oder Alkoxy mit 1 bis 6 C-Atomen,
   - Z^{x} und Z^{y}: jeweils unabhängig voneinander -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, oder eine Einfachbindung, vorzugsweise eine Einfachbindung,
   - L¹⁻⁴: jeweils unabhängig voneinander F, Cl, OCF₃, CF₃, CH₃, CH₂F, CHF₂.
   Vorzugsweise bedeuten beide Reste L¹ und L² F, oder einer der Reste L¹ und L² F und der andere Cl, bzw. beide Reste L³ und L⁴ F, oder einer der Reste L³ und L⁴ F und der andere Cl.
   Die Verbindungen der Formel CY sind vorzugsweise ausgewählt aus der Gruppe bestehend aus folgenden Unterformeln 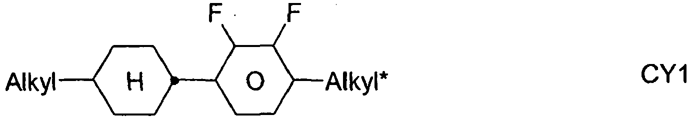 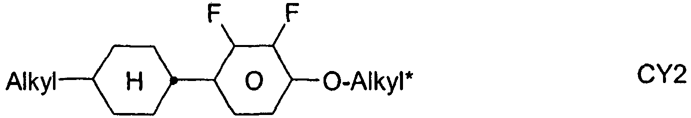 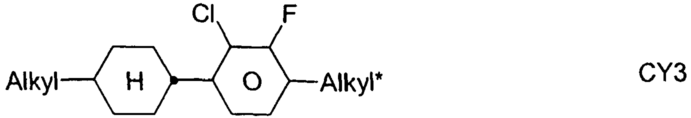 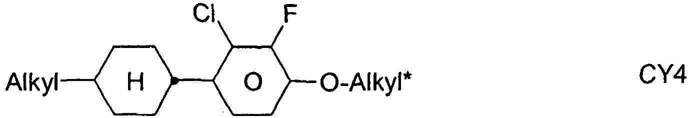 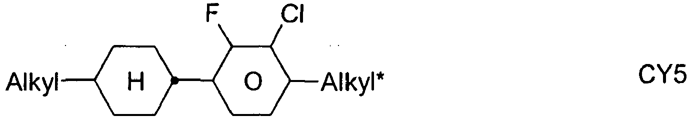 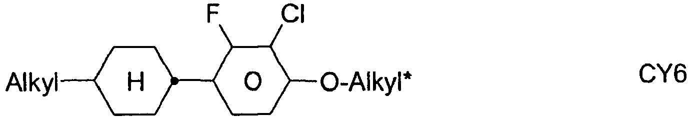 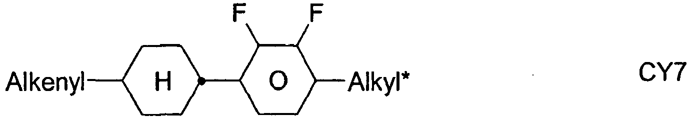 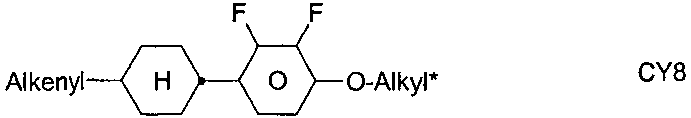 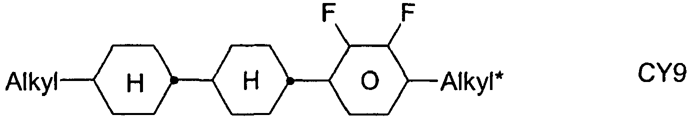 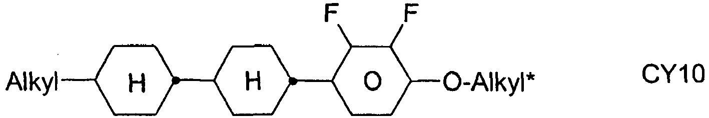 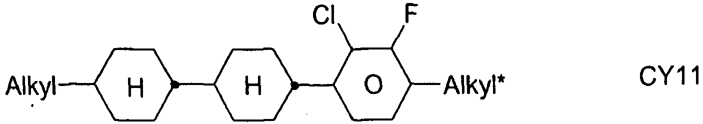 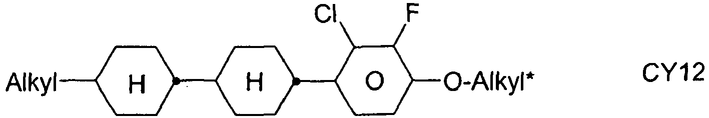 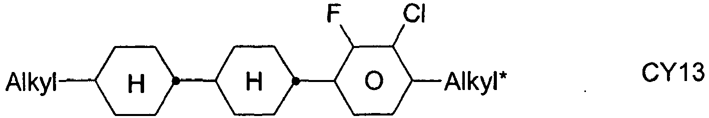 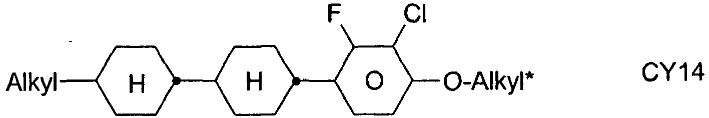 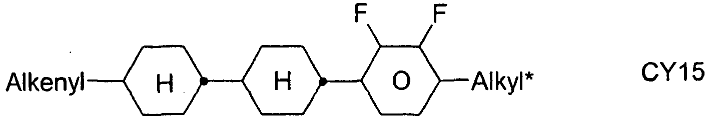 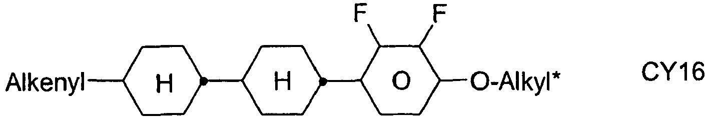 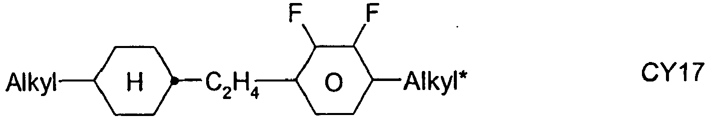 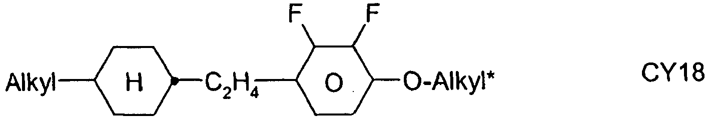 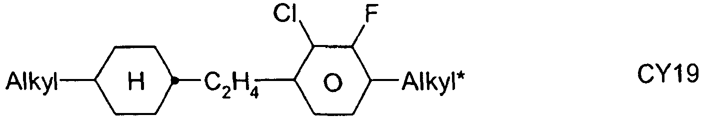 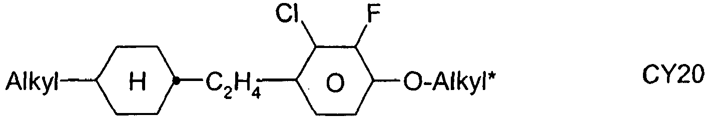 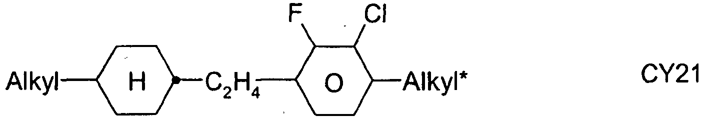 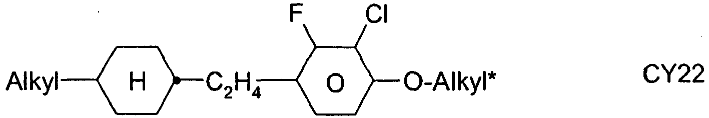 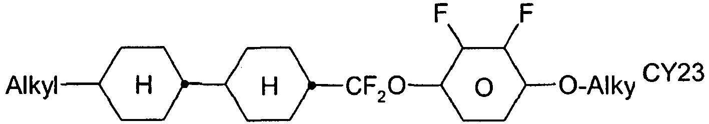 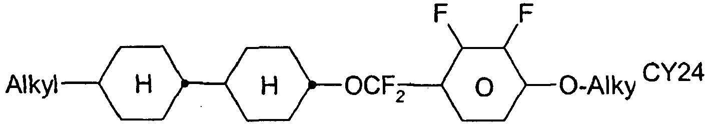 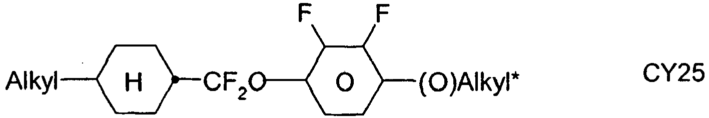 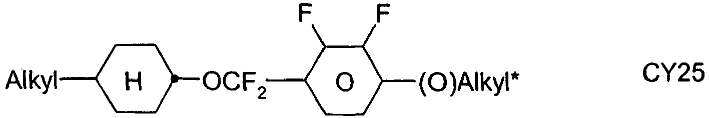 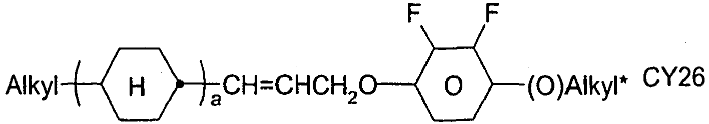 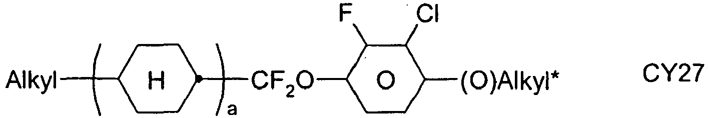 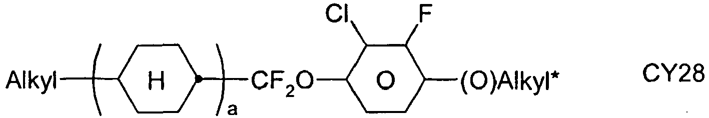 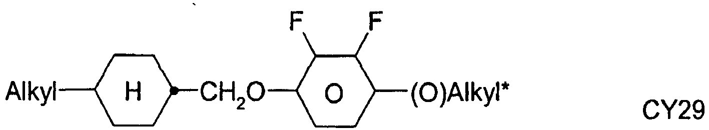 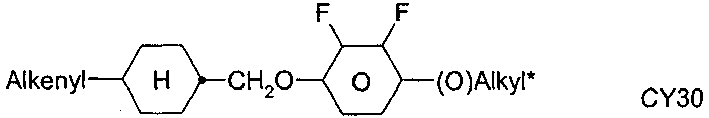 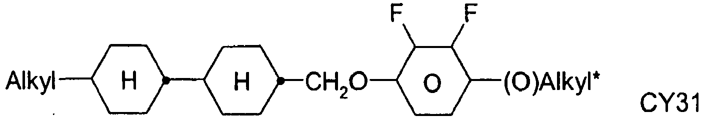 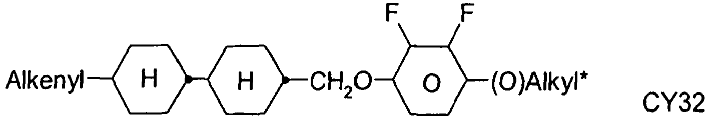 worin a 1 oder 2, Alkyl und Alkyl* jeweils unabhängig voneinander einen geradkettigen Alkylrest mit 1-6 C-Atomen, und Alkenyl einen geradkettigen Alkenylrest mit 2-6 C-Atomen, und (O) ein Sauerstoffatom oder eine Einfachbindung bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
   Die Verbindungen der Formel PY sind vorzugsweise ausgewählt aus der Gruppe bestehend aus folgenden Unterformeln: 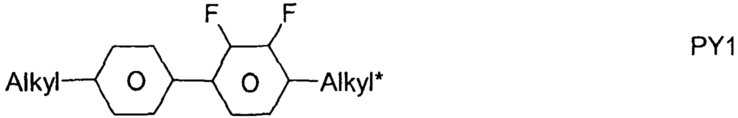 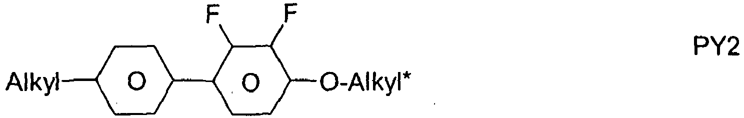 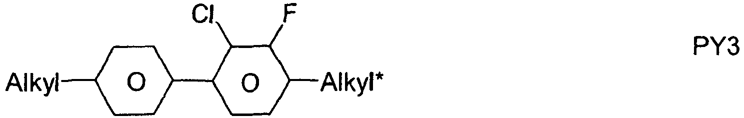 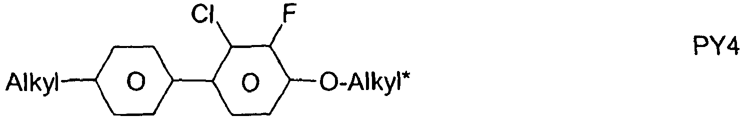 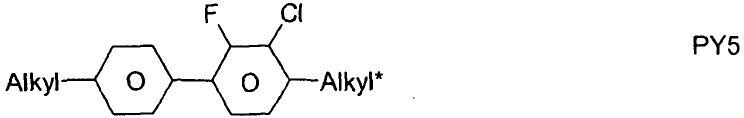 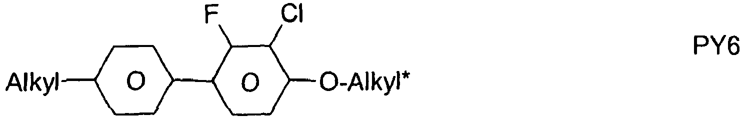 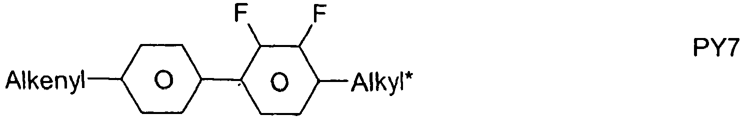 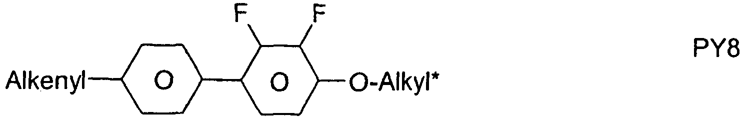 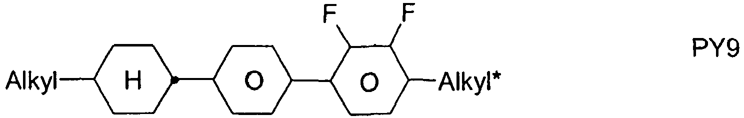 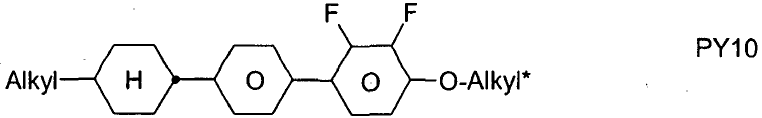 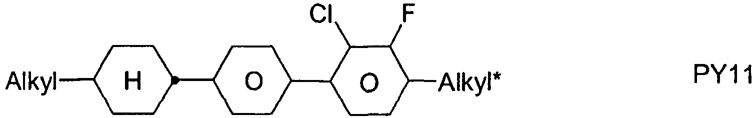 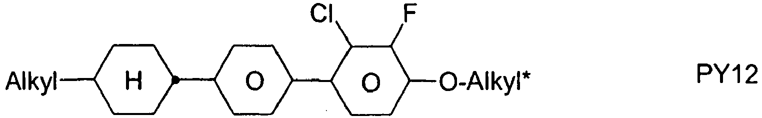 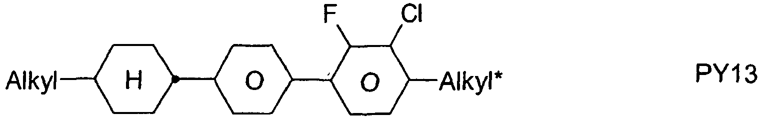 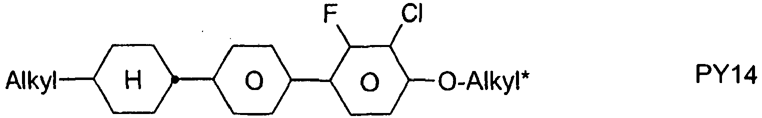 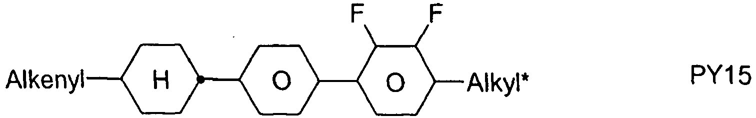 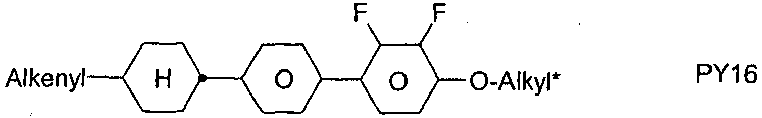 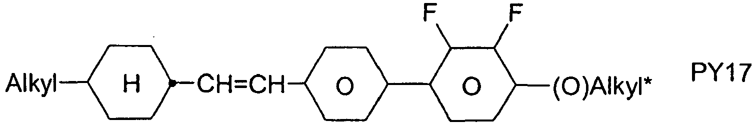 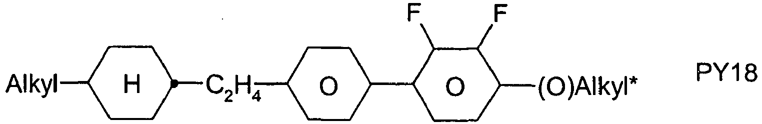 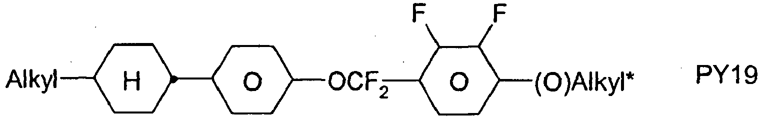 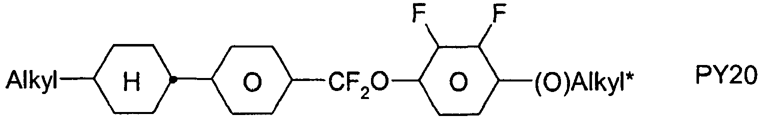 worin Alkyl und Alkyl* jeweils unabhängig voneinander einen geradkettigen Alkylrest mit 1-6 C-Atomen und Alkenyl einen geradkettigen Alkenylrest mit 2-6 C-Atomen, und (O) ein Sauerstoffatom oder eine Einfachbindung bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
b) FK-Medium, welches zusätzlich eine oder mehrere Verbindungen der folgenden Formel enthält: 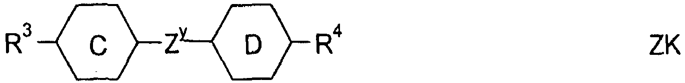 worin die einzelnen Reste folgende Bedeutung besitzen 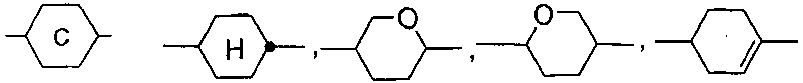 oder 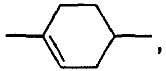 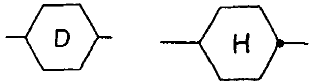 oder 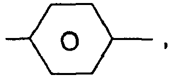
   - R³ und R⁴: jeweils unabhängig voneinander Alkyl mit 1 bis 12 C-Atomen, worin auch eine oder zwei nicht benachbarte CH₂-Gruppen durch -O-, -CH=CH-, -CO-, -O-CO- oder -CO-O- so ersetzt sein können, dass O-Atome nicht direkt miteinander verknüpft sind,
   - Z^{y}: -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, oder eine Einfachbindung, vorzugsweise eine Einfachbindung.
   Die Verbindungen der Formel ZK sind vorzugsweise ausgewählt aus der Gruppe bestehend aus folgenden Unterformeln: 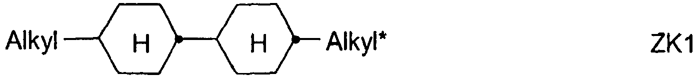 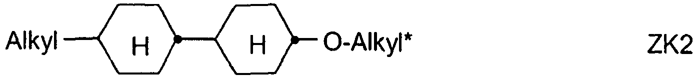 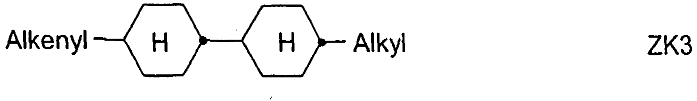 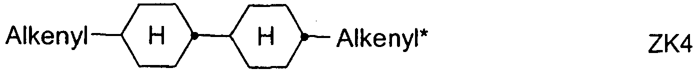 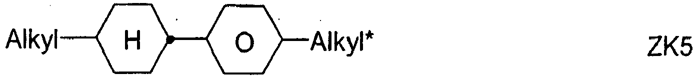 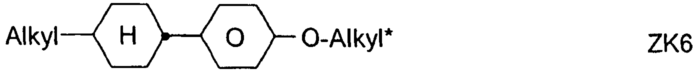 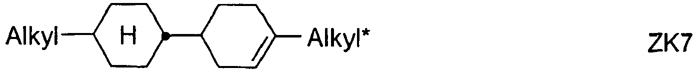 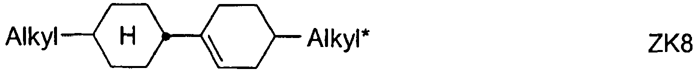 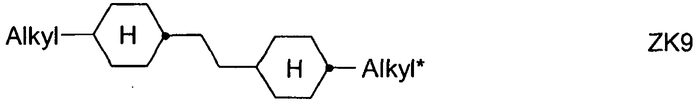 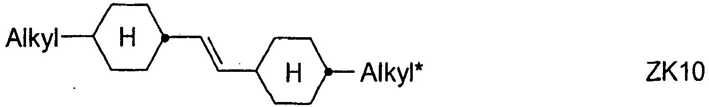 worin Alkyl und Alkyl* jeweils unabhängig voneinander einen geradkettigen Alkylrest mit 1-6 C-Atomen bedeuten, und Alkenyl einen geradkettigen Alkenylrest mit 2-6 C-Atomen bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
c) FK-Medium, welches zusätzlich eine oder mehrere Verbindungen der folgenden Formel enthält: 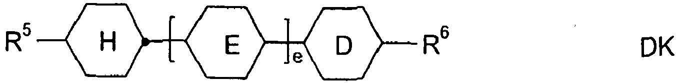 worin die einzelnen Reste bei jedem Auftreten gleich oder verschieden folgende Bedeutung haben:
   - R⁵ und R⁶: jeweils unabhängig voneinander eine der oben für R¹ angegebenen Bedeutungen, 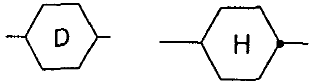 oder 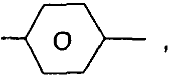 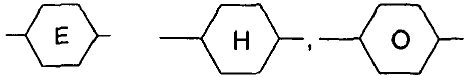 oder 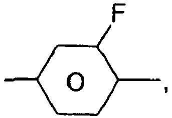 und
   e 1 oder 2.
   Die Verbindungen der Formel DK sind vorzugsweise ausgewählt aus der Gruppe bestehend aus folgenden Unterformeln: 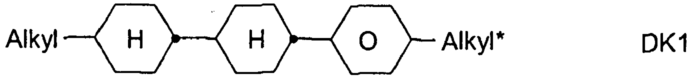 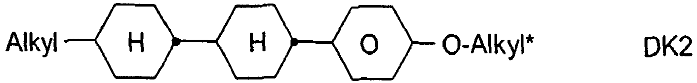 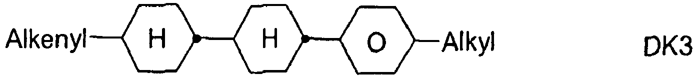 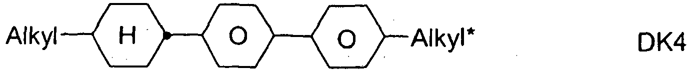 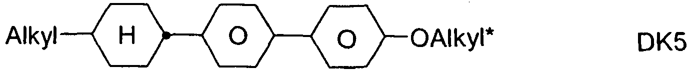 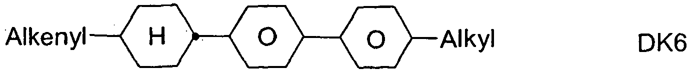 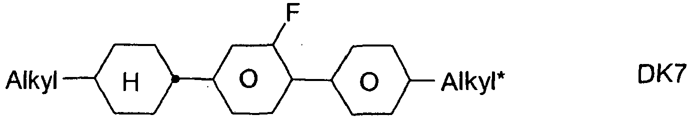 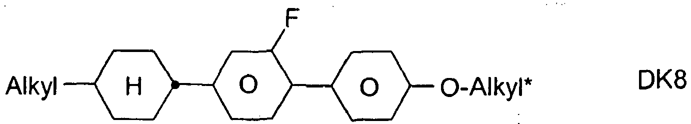 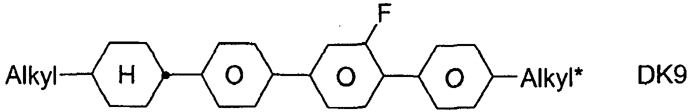 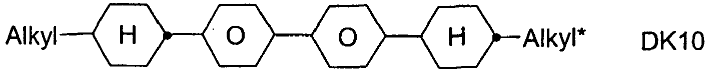 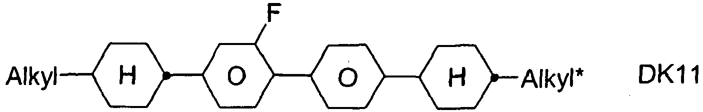 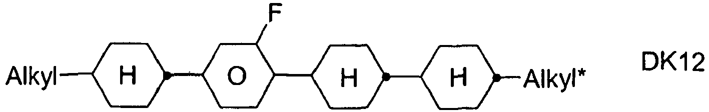 worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
d) FK-Medium, welches zusätzlich eine oder mehrere Verbindungen der folgenden Formel enthält: 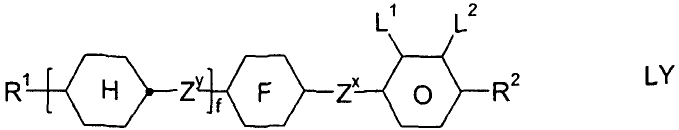 worin die einzelnen Reste folgende Bedeutung besitzen 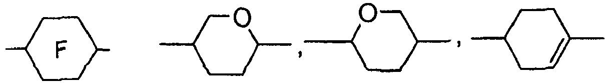 oder 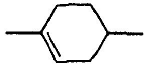
   - f: 0 oder 1,
   - R¹ und R²: jeweils unabhängig voneinander Alkyl mit 1 bis 12 C-Atomen, wobei auch eine oder zwei nicht benachbarte CH₂-Gruppen durch -O-, -CH=CH-, -CO-, -OCO- oder - COO- so ersetzt sein können, dass O-Atome nicht direkt miteinander verknüpft sind,
   - Z^{x} und Z^{y}: jeweils unabhängig voneinander -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, oder eine Einfachbindung, vorzugsweise eine Einfachbindung,
   - L¹ und L²: jeweils unabhängig voneinander F, Cl, OCF₃, CF₃, CH₃, CH₂F, CHF₂.
   Vorzugsweise bedeuten beide Reste L¹ und L² F oder einer der Reste L¹ und L² F und der andere Cl.
   Die Verbindungen der Formel LY sind vorzugsweise ausgewählt aus der Gruppe bestehend aus folgenden Unterformeln 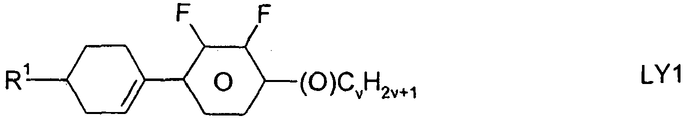 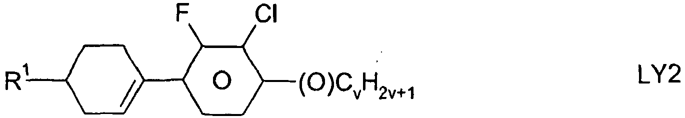 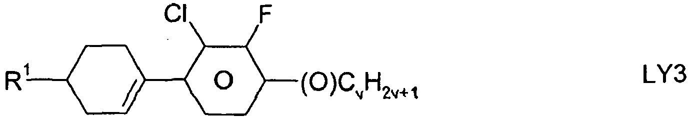 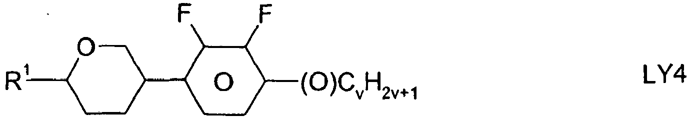 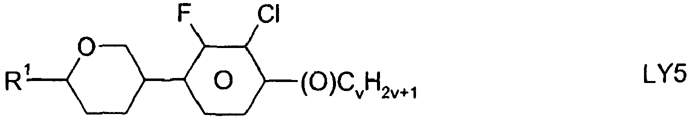 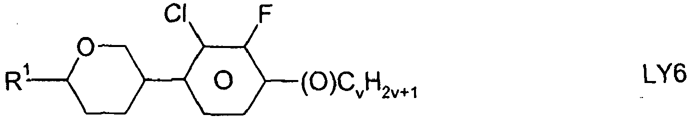 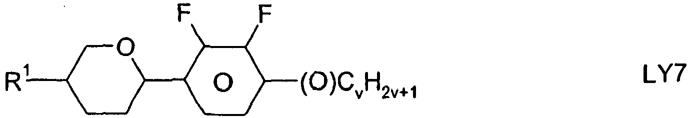 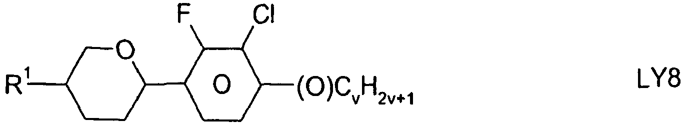 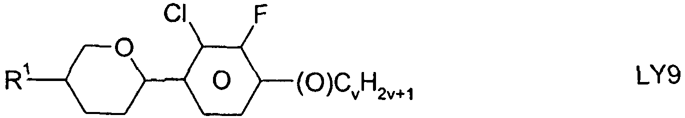 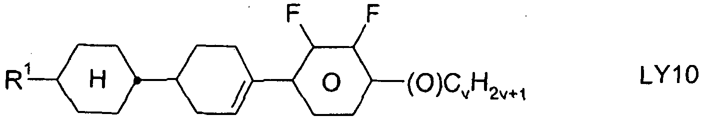 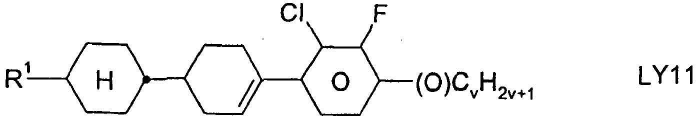 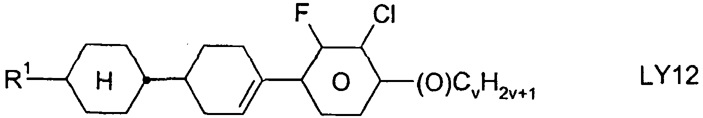 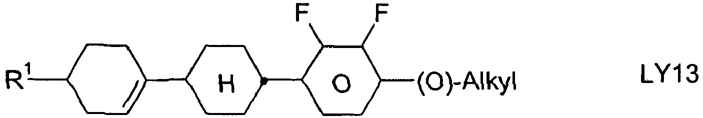 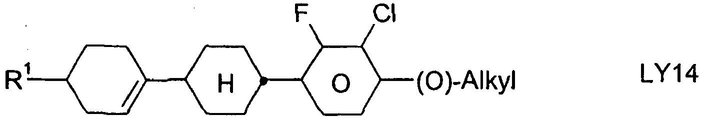 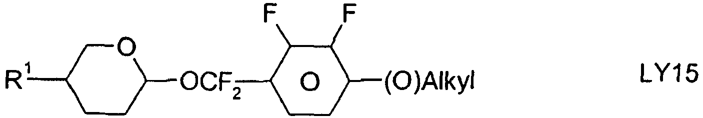 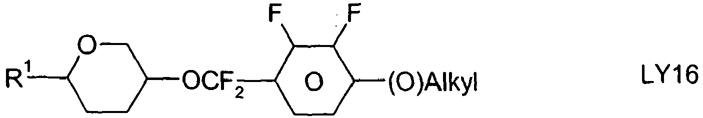 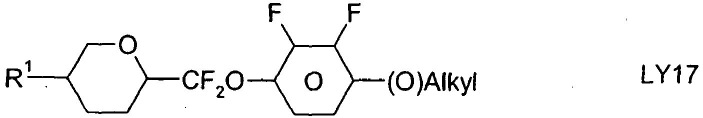 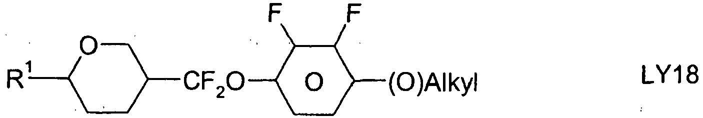 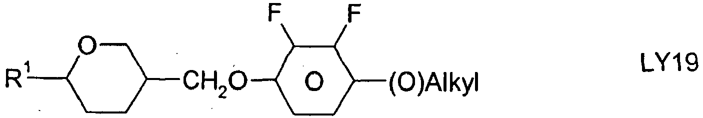 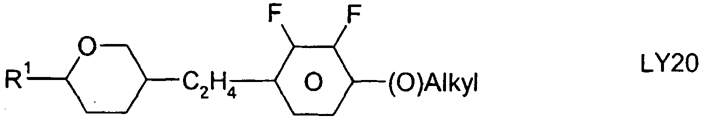 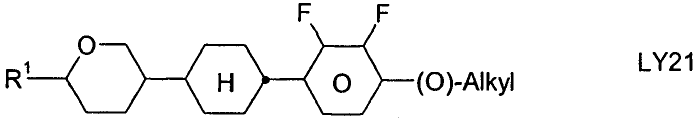 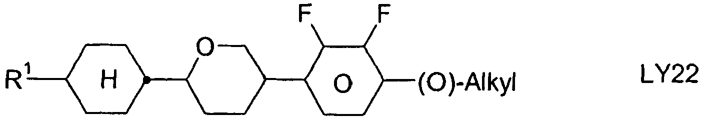 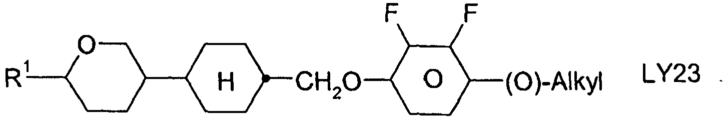 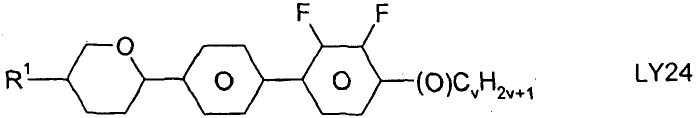 worin R¹ die oben angegebene Bedeutung hat, Alkyl einen geradkettigen Alkylrest mit 1-6 C-Atomen, (O) ein Sauerstoffatom oder eine Einfachbindung und v eine ganze Zahl von 1 bis 6 bedeuten. R¹ bedeutet vorzugsweise geradkettiges Alkyl mit 1 bis 6 C-Atomen oder geradkettiges Alkenyl mit 2 bis 6 C-Atomen, insbesondere CH₃, C₂H₅, n-C₃H₇, n-C₄H₉, n-C₅H₁₁, CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
e) FK-Medium, welches zusätzlich eine oder mehrere Verbindungen ausgewählt aus der Gruppe bestehend aus folgenden Formeln enthält: 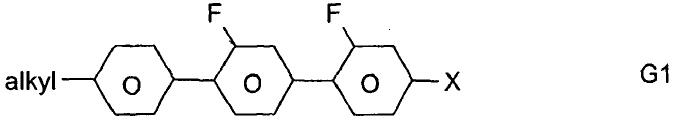 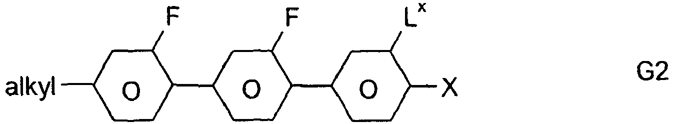 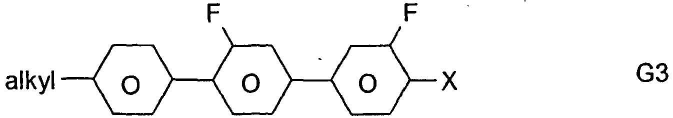 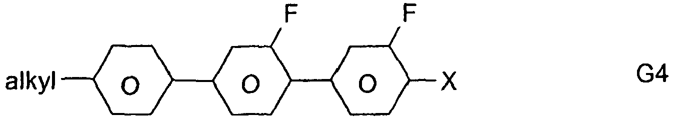 worin alkyl C₁₋₆-alkyl, L^{x} H oder F und X F, Cl, OCF₃, OCHF₂ oder OCH=CF₂ bedeutet. Besonders bevorzugt sind Verbindungen der Formel G1, worin X F bedeutet.
f) FK-Medium, welches zusätzlich eine oder mehrere Verbindungen ausgewählt aus der Gruppe bestehend aus folgenden Formeln enthält: 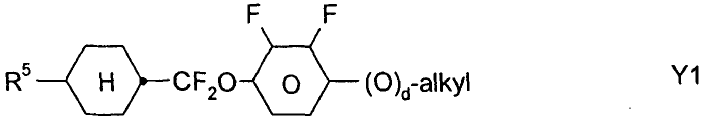 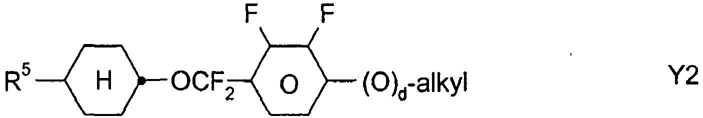 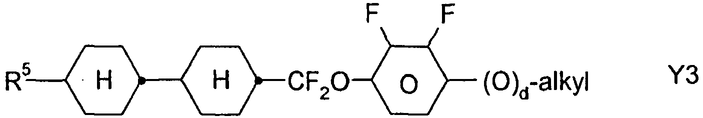 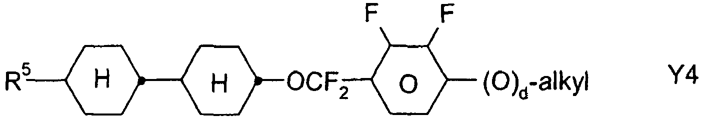 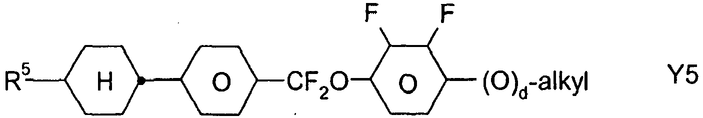 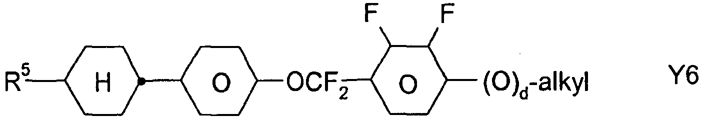 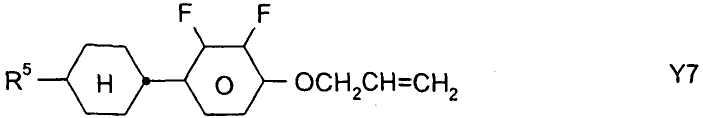 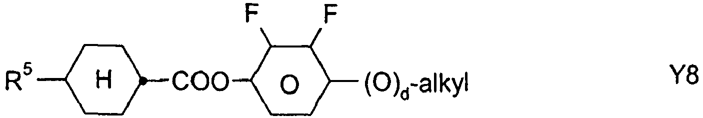 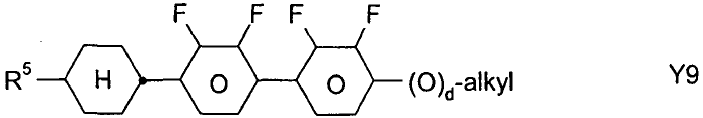 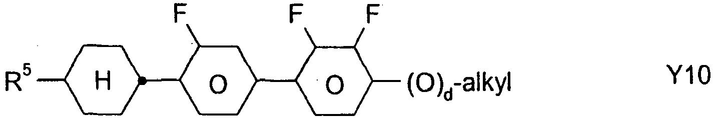 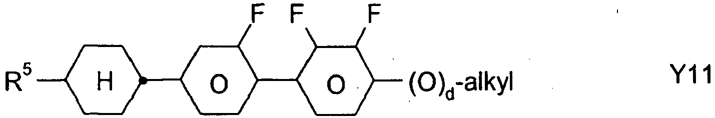 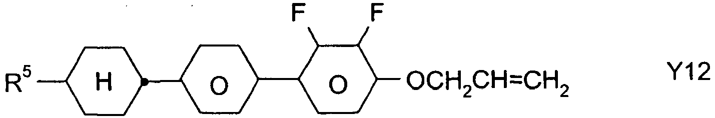 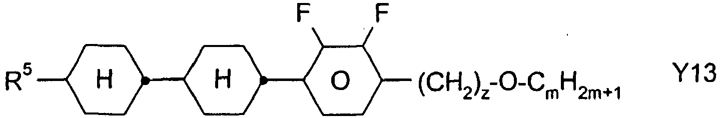 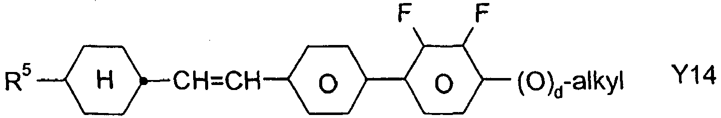 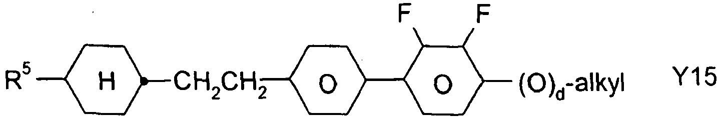 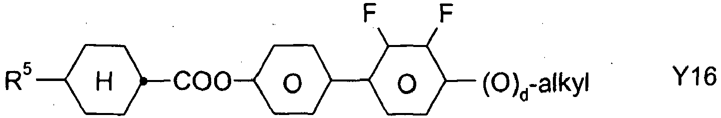 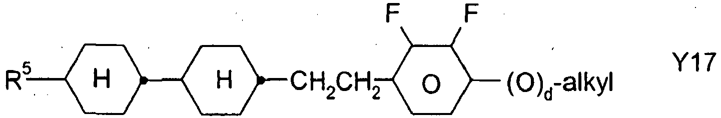 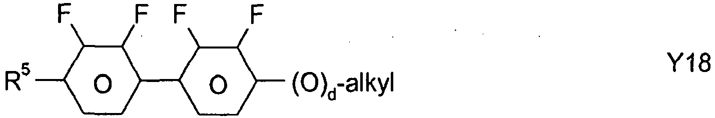 worin R⁵ eine der oben für R¹ angegebenen Bedeutungen besitzt, alkyl C₁₋₆-alkyl, d 0 oder 1, und z und m jeweils unabhängig voneinander eine ganze Zahl von 1 bis 6 bedeuten. R⁵ ist in diesen Verbindungen besonders bevorzugt C₁₋₆-alkyl oder -alkoxy oder C₂₋₆-alkenyl, d ist vorzugsweise 1. Vorzugsweise enthält das erfindungsgemäße FK-Medium eine oder mehrere Verbindungen der oben genannten Formeln in Mengen von ≥ 5 Gew.%.
g) FK-Medium, welches zusätzlich eine oder mehrere Biphenylverbindungen ausgewählt aus der Gruppe bestehend aus folgenden Formeln enthält: 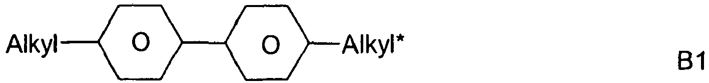 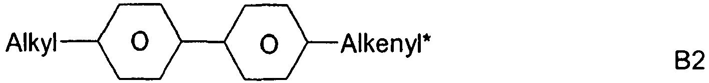 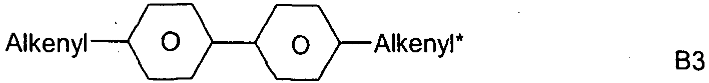 worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
   Der Anteil der Biphenyle der Formeln B1 bis B3 in der FK-Mischung beträgt vorzugsweise mindestens 3 Gew.%, insbesondere ≥ 5 Gew.%.
   Die Verbindungen der Formel B2 sind besonders bevorzugt.
   Die Verbindungen der Formel B1 bis B3 sind vorzugsweise ausgewählt aus der Gruppe bestehend aus folgenden Unterformeln 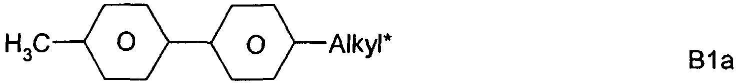 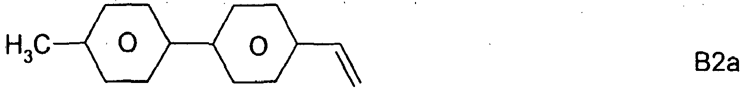 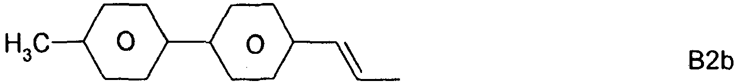 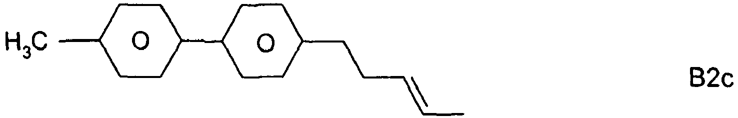 worin Alkyl* einen Alkylrest mit 1-6 C-Atomen bedeutet. Insbesondere bevorzugt enthält das erfindungsgemäße Medium eine oder mehrere Verbindungen der Formeln B1a und/oder B2c.
h) FK-Medium, welches zusätzlich eine oder mehrere Terphenylverbindungen der folgenden Formel enthält: 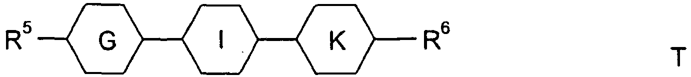 worin R⁵ und R⁶ jeweils unabhängig voneinander eine der oben für R¹ angegebenen Bedeutungen besitzen und 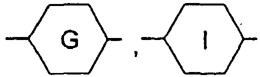 und 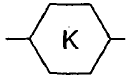 jeweils unabhängig voneinander 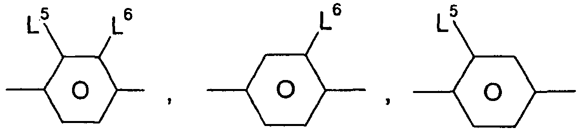 oder 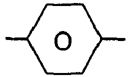 bedeuten, worin L⁵ F oder Cl, vorzugsweise F, und L⁶ F, Cl, OCF₃, CF₃, CH₃, CH₂F oder CHF₂, vorzugsweise F, bedeuten.
   Die Verbindungen der Formel T sind vorzugsweise ausgewählt aus der Gruppe bestehend aus folgenden Unterformeln 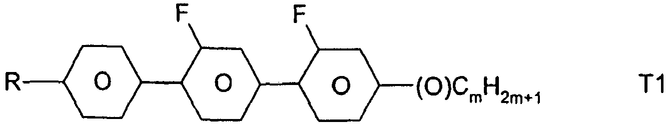 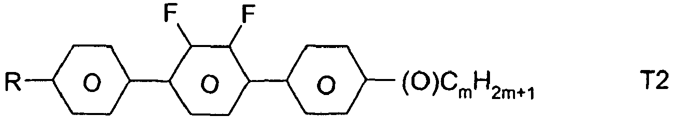 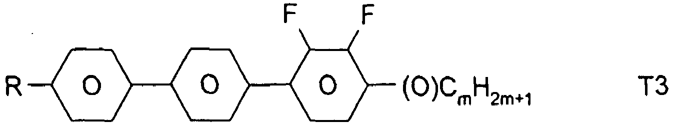 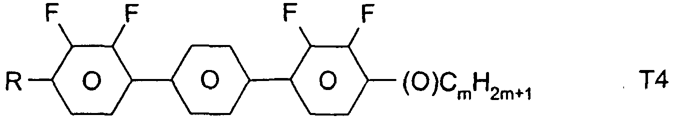 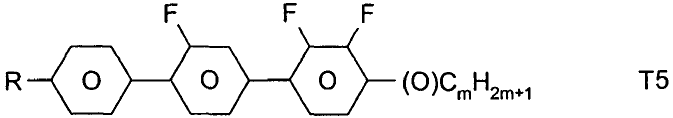 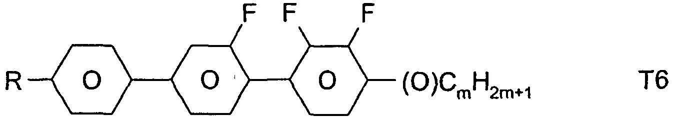 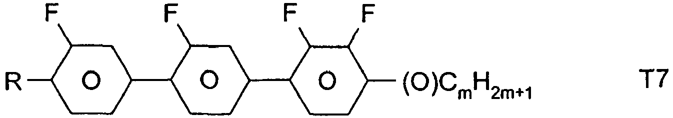 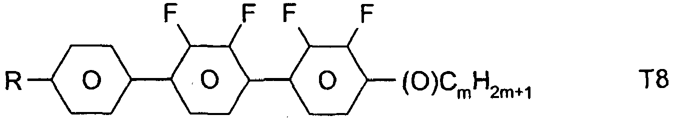 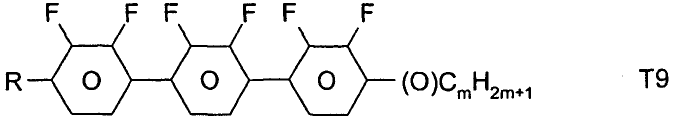 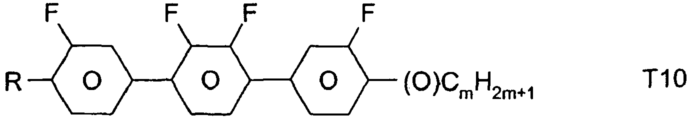 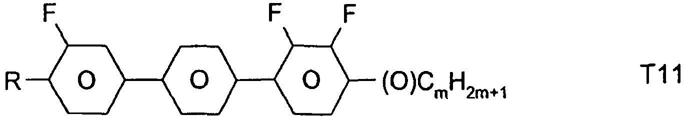 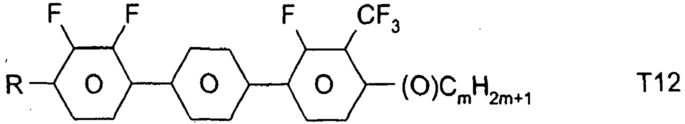 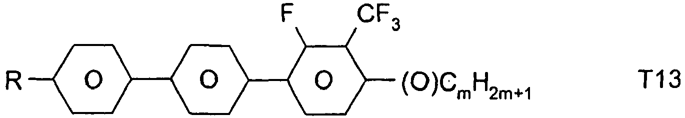 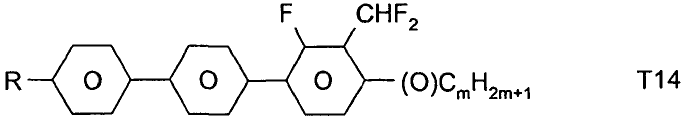 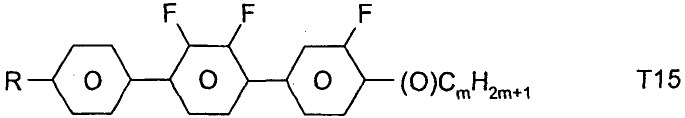 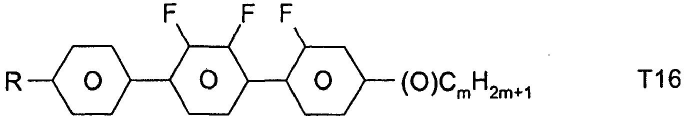 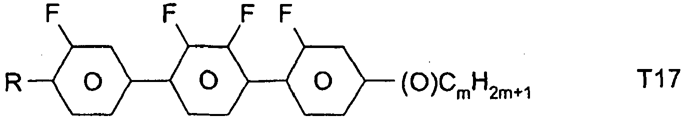 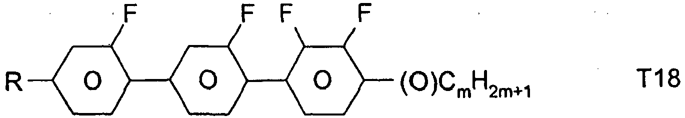 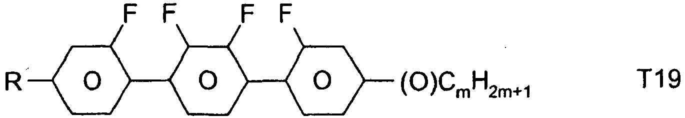 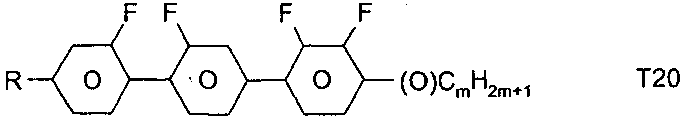 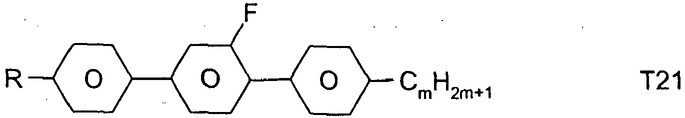 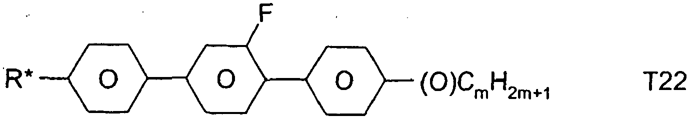 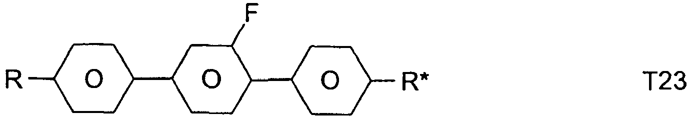 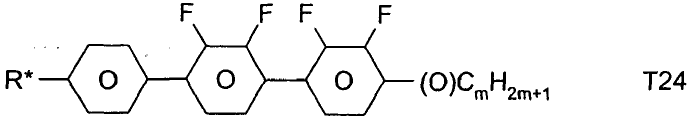 worin R einen geradkettigen Alkyl- oder Alkoxyrest mit 1-7 C-Atomen, R* einen geradkettigen Alkenylrest mit 2-7 C-Atomen, (O) ein Sauerstoffatom oder eine Einfachbindung, und m eine ganze Zahl von 1 bis 6 bedeutet. R* bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
   Vorzugsweise bedeutet R Methyl, Ethyl, Propyl, Butyl, Pentyl, Hexyl Methoxy, Ethoxy, Propoxy, Butoxy oder Pentoxy.
   Das erfindungsgemäße FK-Medium enthält die Terphenyle der Formeln T und deren bevorzugte Unterformeln vorzugsweise in einer Menge von 0,5-30 Gew.%, insbesondere von 1-20 Gew.%.
   Besonders bevorzugt sind Verbindungen der Formeln T1, T2, T3 und T21. In diesen Verbindungen bedeutet R vorzugsweise Alkyl, ferner Alkoxy jeweils mit 1-5 C-Atomen.
   Vorzugsweise werden die Terphenyle in erfindungsgemäßen Mischungen eingesetzt, wenn der Δn-Wert der Mischung ≥ 0,1 sein soll. Bevorzugte Mischungen enthalten 2-20 Gew.% einer oder mehrerer Terphenyl-Verbindungen der Formel T, vorzugsweise ausgewählt aus der Gruppe der Verbindungen T1 bis T22.
i) FK-Medium, welches zusätzlich eine oder mehrere Verbindungen ausgewählt aus der Gruppe bestehend aus folgenden Formeln enthält: 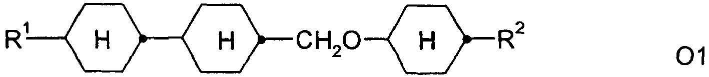 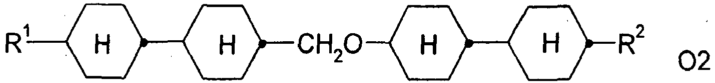 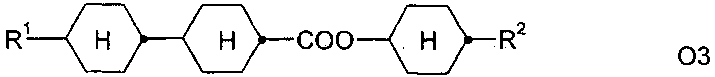 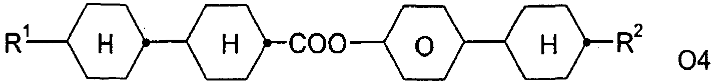 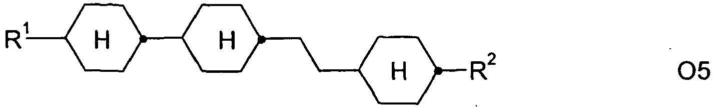 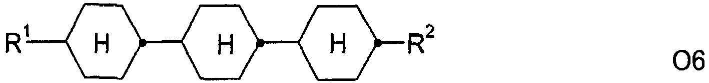 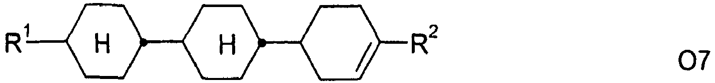 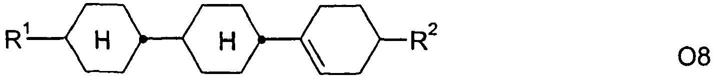 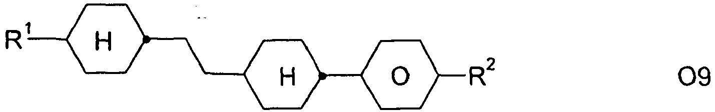 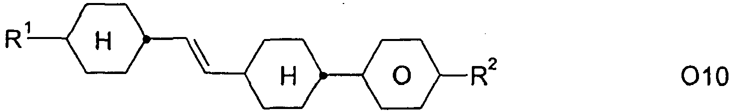 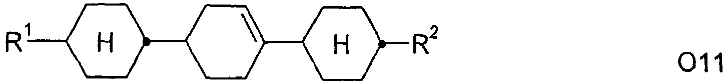 worin R¹ und R² die oben angegebenen Bedeutungen haben, und vorzugsweise jeweils unabhängig voneinander geradkettiges Alkyl mit 1 bis 6 C-Atomen oder geradkettiges Alkenyl mit 2 bis 6 C-Atomen bedeuten.
   Bevorzugte Medien enthalten eine oder mehrere Verbindungen ausgewählt aus den Formeln O1, 03 und 04.
k) FK-Medium, welches zusätzlich eine oder mehrere Verbindungen der folgenden Formel enthält: 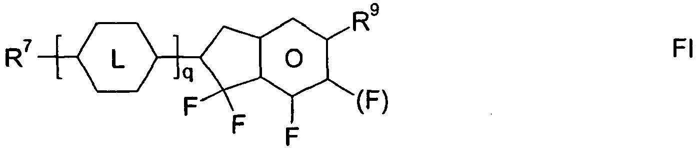 worin 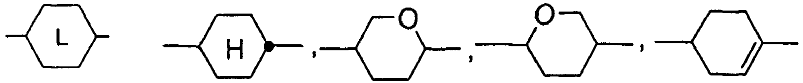 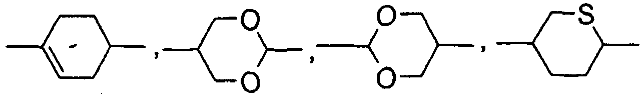 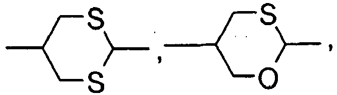 R⁹ H, CH₃, C₂H₅ oder n-C₃H₇, (F) einen optionalen Fluorsubstituenten und q 1, 2 oder 3 bedeutet, und R⁷ eine der für R¹ angegebenen Bedeutungen hat, vorzugsweise in Mengen von > 3 Gew.%, insbesondere ≥ 5 Gew.%, und ganz besonders bevorzugt von 5-30 Gew.%.
   Besonders bevorzugte Verbindungen der Formel IF sind ausgewählt aus der Gruppe bestehend aus folgenden Unterformeln: 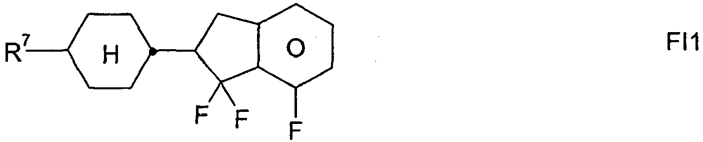 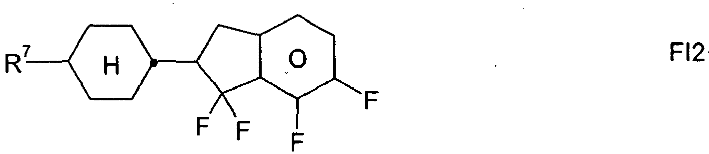 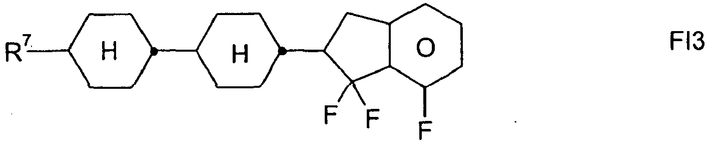 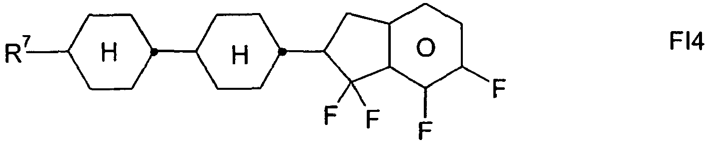 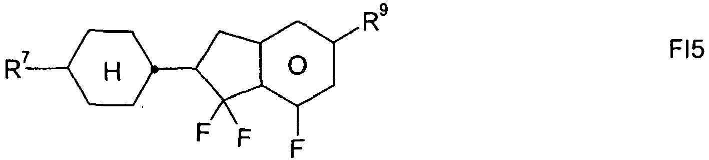 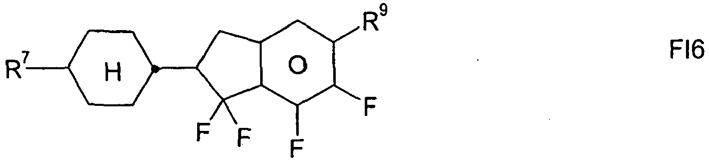 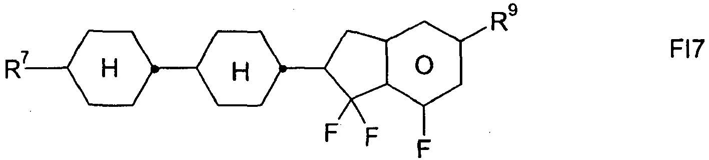 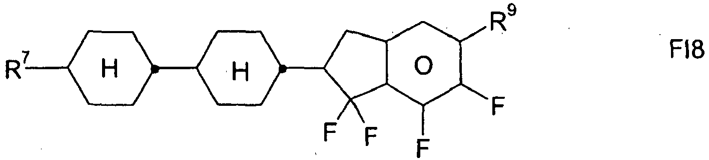 worin R⁷ vorzugsweise geradkettiges Alkyl bedeutet und R⁹ CH₃, C₂H₅ oder n-C₃H₇ bedeutet. Besonders bevorzugt sind die Verbindungen der Formel FI1, FI2 und FI3.
m) FK-Medium, welches zusätzlich eine oder mehrere Verbindungen ausgewählt aus der Gruppe bestehend aus folgenden Formeln enthält: 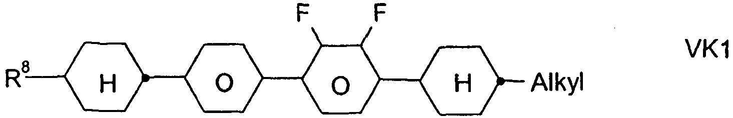 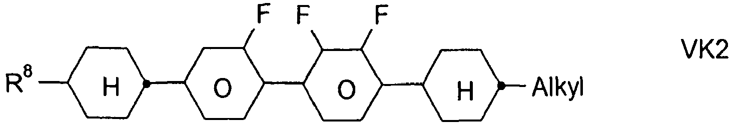 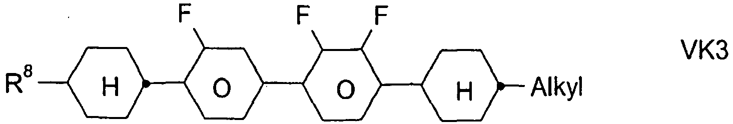 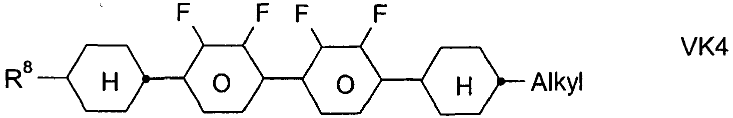 worin R⁸ die für R¹ angegebene Bedeutung hat und Alkyl einen geradkettigen Alkylrest mit 1-6 C-Atomen bedeutet.
n) FK-Medium, welches zusätzlich eine oder mehrere Verbindungen enthält, die eine Tetrahydronaphthyl- oder Naphthyl-Einheit aufweisen, wie z.B. die Verbindungen ausgewählt aus der Gruppe bestehend aus folgenden Formeln: 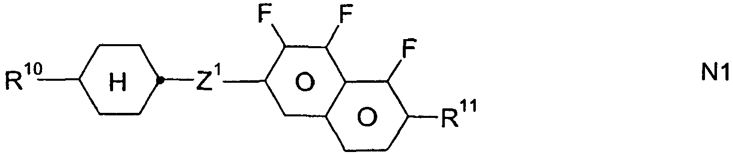 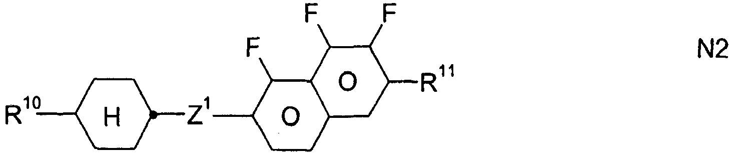 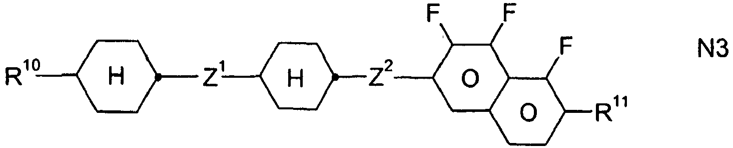 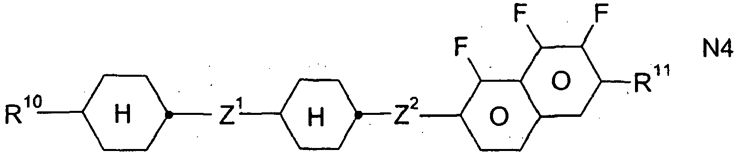 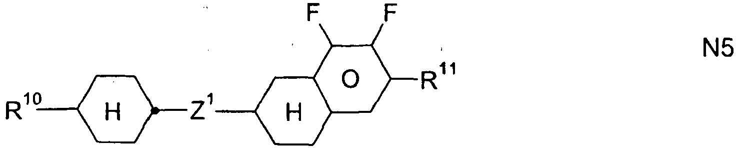 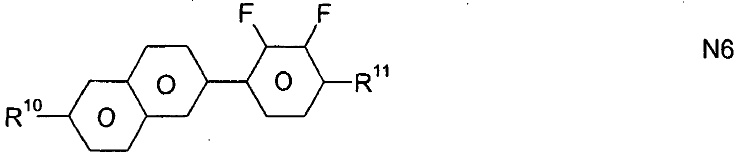 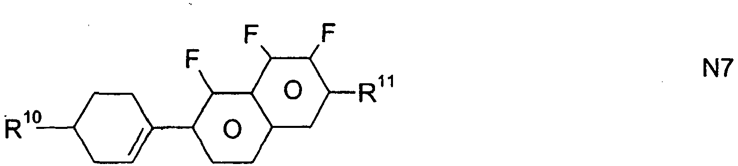 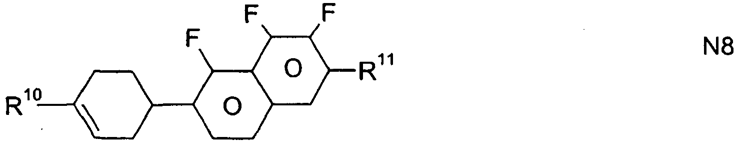 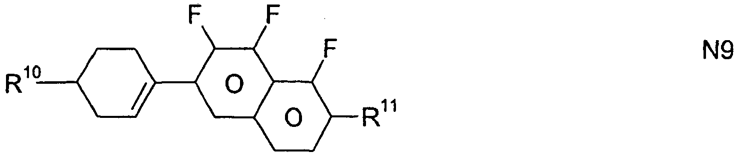 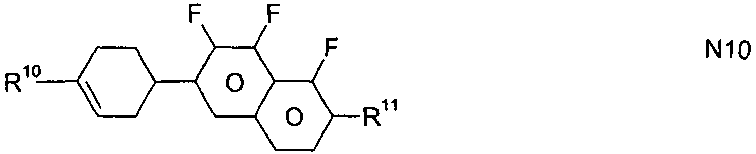 worin R¹⁰ und R¹¹ jeweils unabhängig voneinander eine der für R¹ angegebenen Bedeutungen haben, vorzugsweise geradkettiges Alkyl oder Alkoxy mit 1 bis 6 C-Atomen oder geradkettiges Alkenyl mit 2 bis 6 C-Atomen bedeuten, und Z¹ und Z² jeweils unabhängig voneinander -C₂H₄-, -CH=CH-, -(CH₂)₄-, -(CH₂)₃O-, -O(CH₂)₃-, -CH=CH-CH₂CH₂-, -CH₂CH₂CH=CH-, -CH₂O-, -OCH₂-, -CO-O-, -O-CO-, -C₂F₄-, -CF=CF-, -CF=CH-, -CH=CF-, -CH₂- oder eine Einfachbindung bedeuten.
o) FK-Medium, welches zusätzlich eine oder mehrere eine oder mehrere Difluordibenzochromane und/oder Chromane der folgenden Formeln enthält: 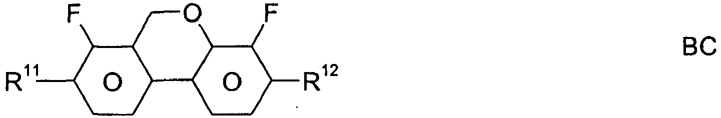 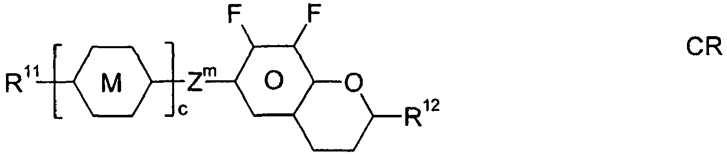 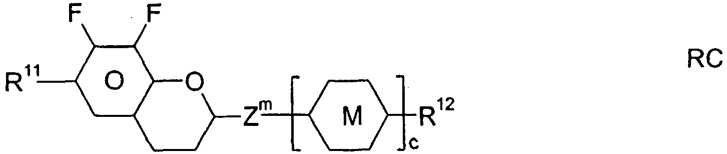 worin
   - R¹¹ und R¹²: jeweils unabhängig voneinander die oben angegebene Bedeutung aufweisen,
   - Ring M: trans-1,4-Cyclohexylen oder 1,4-Phenylen bedeutet,
   - Z^{m}: -C₂H₄-, -CH₂O-, -OCH₂-, -CO-O- oder -O-CO-,
   - c: 0 oder 1 bedeutet,
   vorzugsweise in Mengen von 3 bis 20 Gew.%, insbesondere in Mengen von 3 bis 15 Gew.%.
   Besonders bevorzugte Verbindungen der Formeln BC, CR und RC sind ausgewählt aus der Gruppe bestehend aus folgenden Unterformeln: 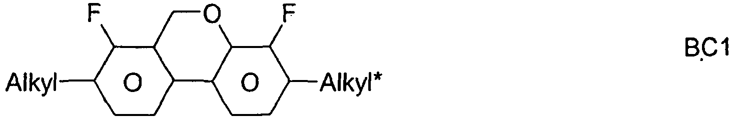 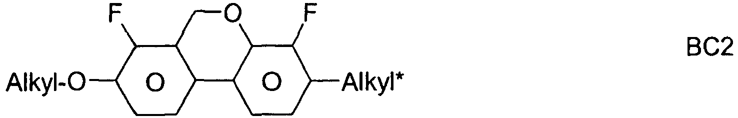 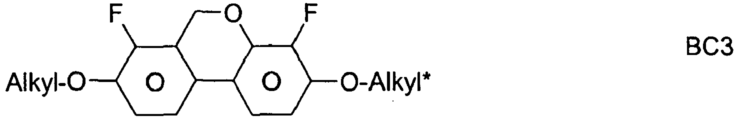 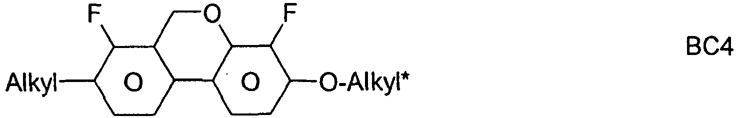 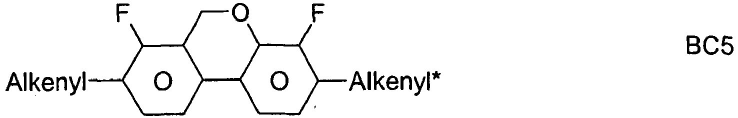 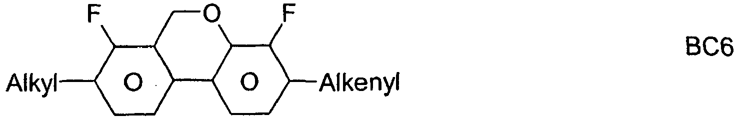 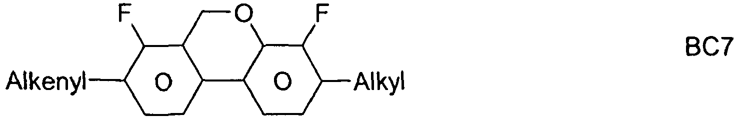 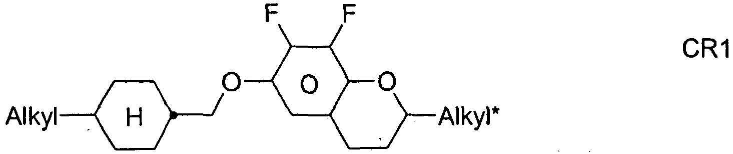 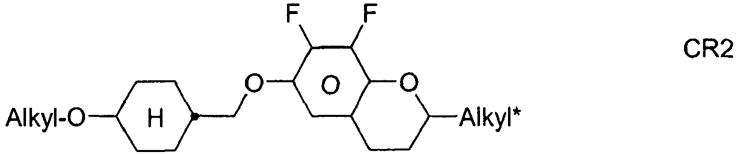 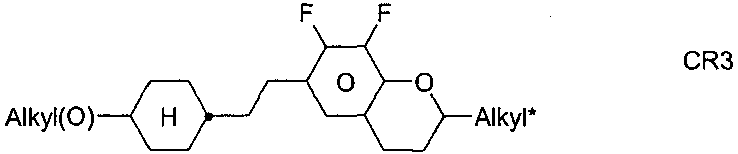 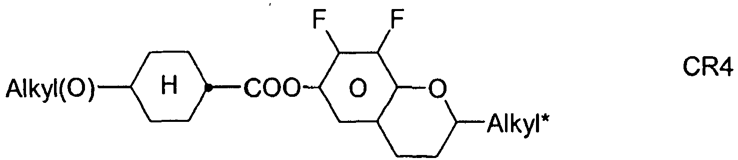 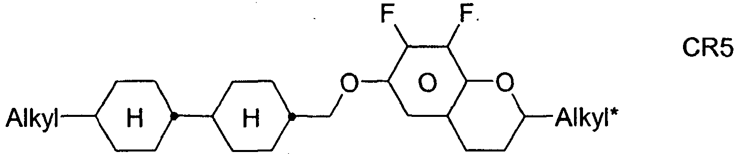 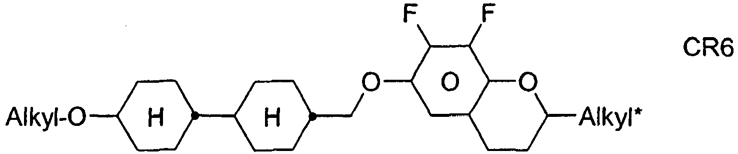 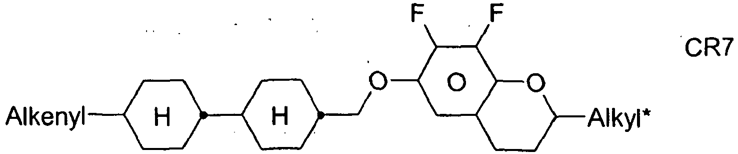 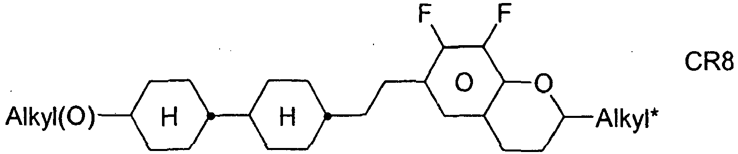 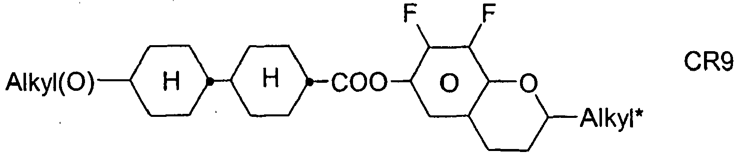 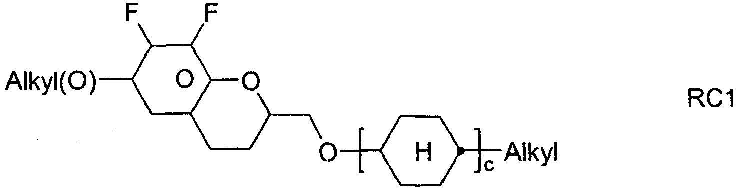 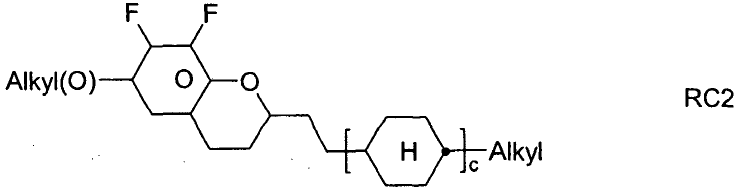 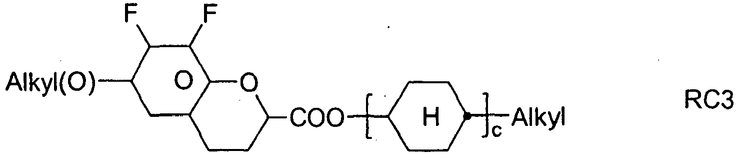 worin Alkyl und Alkyl* jeweils unabhängig voneinander einen geradkettigen Alkylrest mit 1-6 C-Atomen, (O) ein Sauerstoffatom oder eine Einfachbindung, c 1 oder 2, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
   Ganz besonders bevorzugt sind Mischungen enthaltend eine, zwei oder drei Verbindungen der Formel BC-2.
p) FK-Medium, welches zusätzlich eine oder mehrere fluorierte Phenanthrene und/oder Dibenzofurane der folgenden Formeln enthält: 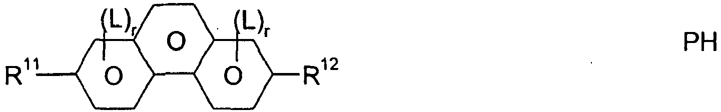 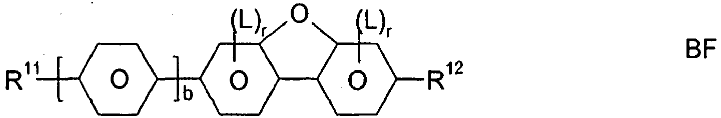 worin R¹¹ und R¹² jeweils unabhängig voneinander die oben angegebenen Bedeutungen besitzen, b 0 oder 1, L F und r 1, 2 oder 3 bedeutet.
   Besonders bevorzugte Verbindungen der Formeln PH und BF sind ausgewählt aus der Gruppe bestehend aus folgenden Unterformeln: 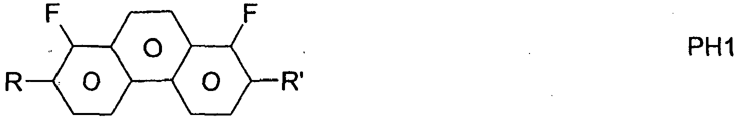 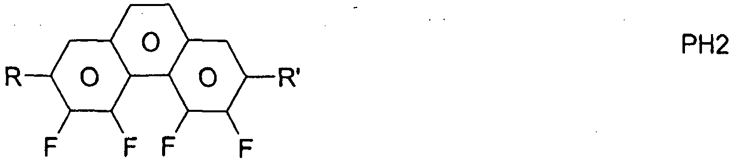 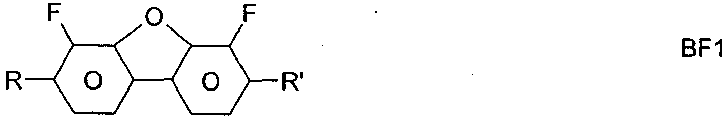 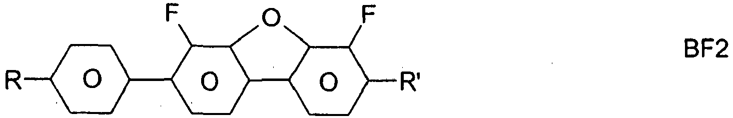 worin R und R' jeweils unabhängig voneinander einen geradkettigen Alkyl- oder Alkoxyrest mit 1-7 C-Atomen bedeuten.
q) FK-Medium, welches außer den erfindungsgemäßen polymerisierbaren Verbindungen, insbesondere der Formel I oder deren Unterformeln, sowie den Comonomeren, keine Verbindungen enthält, die eine endständige Vinyloxygruppe (-O-CH=CH₂) aufweisen.
r) FK-Medium, welches 1 bis 5, vorzugsweise 1, 2 oder 3 polymerisierbare Verbindungen, vorzusgweise ausgewählt aus erfindungsgemäßen polymerisierbare Verbindungen, insbesondere der Formel I oder deren Unterformeln, enthält.
s) FK-Medium, worin der Anteil an polymerisierbaren Verbindungen, insbesondere der Formel I oder deren Unterformeln, im Gesamtgemisch 0,05 bis 5 %, vorzugsweise 0,1 bis 1 % beträgt.
t) FK-Medium, welches 1 bis 8, vorzugsweise 1 bis 5 Verbindungen der Formel CY1, CY2, PY1 und/oder PY2 enthält. Der Anteil dieser Verbindungen im Gesamtgemisch beträgt vorzugsweise 5 bis 60 %, besonders bevorzugt 10 bis 35 %. Der Gehalt dieser einzelnen Verbindungen beträgt vorzugsweise jeweils 2 bis 20 %.
u) FK-Medium, welches 1 bis 8, vorzugsweise 1 bis 5 Verbindungen der Formel CY9, CY10, PY9 und/oder PY10 enthält. Der Anteil dieser Verbindungen im Gesamtgemisch beträgt vorzugsweise 5 bis 60 %, besonders bevorzugt 10 bis 35 %. Der Gehalt dieser einzelnen Verbindungen beträgt vorzugsweise jeweils 2 bis 20 %.
v) FK-Medium, welches 1 bis 10, vorzugsweise 1 bis 8 Verbindungen der Formel ZK enthält, insbesondere Verbindungen der Formel ZK1, ZK2 und/oder ZK6. Der Anteil dieser Verbindungen im Gesamtgemisch beträgt vorzugsweise 3 bis 25 %, besonders bevorzugt 5 bis 45 %. Der Gehalt dieser einzelnen Verbindungen beträgt vorzugsweise jeweils 2 bis 20 %.
w) FK-Medium, worin der Anteil an Verbindungen der Formel CY, PY und ZK im Gesamtgemisch mehr als 70 %, vorzugsweise mehr als 80 % beträgt,
x) PSA-VA-Anzeige, worin der pretilt-Winkel vorzugsweise ≤ 85°, besonderes bevorzugt ≤ 80° beträgt.

In einer zweiten bevorzugten Ausführungsform enthält das FK-Medium eine FK-Host-Mischung basierend auf Verbindungen mit positiver dielektrischer Anisotropie. Solche FK-Medien eignen sich besonders zur Verwendung in PSA-OCB-, PSA-TN-, PSA-Posi-VA-, PSA-IPS- oder PSA-FFS-Anzeigen.

Besonders bevorzugt ist ein FK-Medium dieser zweiten bevorzugten Ausführungsform, welches eine oder mehrere Verbindungen ausgewählt aus der Gruppe bestehend aus den Verbindungen der Formeln AA und BB enthält 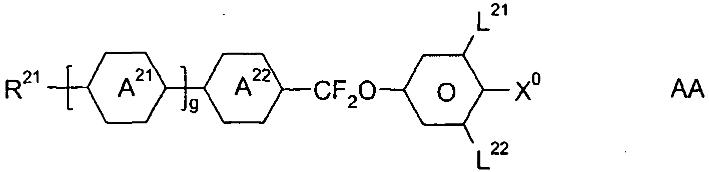 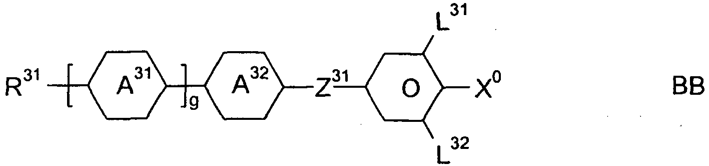 und gegebenenfalls zusätzlich zu den Verbindungen der Formel AA und/oder BB eine oder mehrere Verbindungen der Formel CC enthält 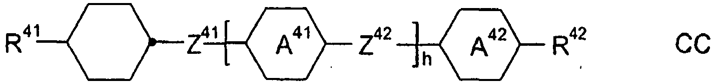 worin die einzelnen Reste folgende Bedeutung besitzen: 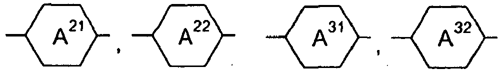 jeweils unabhängig voneinander, und bei jedem Auftreten gleich oder verschieden 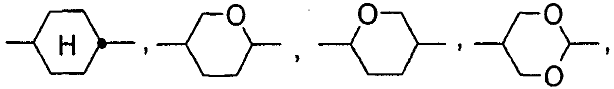 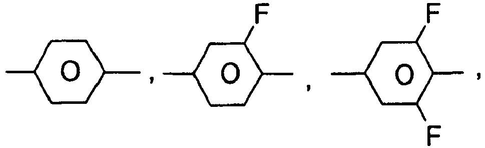 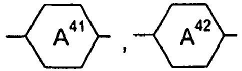 jeweils unabhängig voneinander, und bei jedem Auftreten gleich oder verschieden 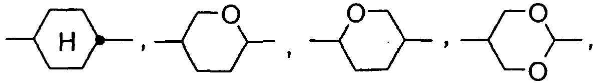 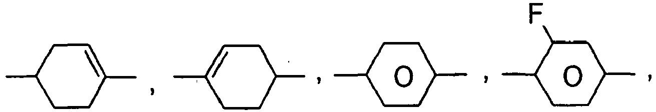
- R²¹, R³¹, R⁴¹, R⁴²: jeweils unabhängig voneinander Alkyl, Alkoxy, Oxaalkyl oder Fluoralkyl mit 1 bis 9 C-Atomen oder Alkenyl mit 2 bis 9 C-Atomen,
- X⁰: F, Cl, halogeniertes Alkyl oder Alkoxy mit 1 bis 6 C-Atomen oder halogeniertes Alkenyl oder Alkenyloxy mit 2 bis 6 C-Atomen,
- Z³¹: -CH₂CH₂-, -CF₂CF₂-, -COO-, *trans-* -CH=CH-, *trans-*-CF=CF-, -CH₂O- oder eine Einfachbindung, vorzugsweise -CH₂CH₂-, -COO-, *trans-* -CH=CH- oder eine Einfachbindung, besonders bevorzugt -COO-, *trans-*-CH=CH- oder eine Einfachbindung,
- Z⁴¹, Z⁴²: -CH₂CH₂-, -COO-, *trans-* -CH=CH-, *trans-* -CF=CF-, - CH₂O-, -CF₂O-, -C≡C- oder eine Einfachbindung, vorzugsweise eine Einfachbindung,
- L²¹, L²², L³¹, L³²: H oder F,
- g: 1, 2 oder 3,
- h: 0, 1, 2 oder 3.
X⁰ ist vorzugsweise F, Cl, CF₃, CHF₂, OCF₃, OCHF₂, OCFHCF₃, OCFHCHF₂, OCFHCHF₂, OCF₂CH₃, OCF₂CHF₂, OCF₂CHF₂, OCF₂CF₂CHF₂, OCF₂CF₂CHF₂, OCFHCF₂CF₃, OCFHCF₂CHF₂, OCF₂CF₂CF₃, OCF₂CF₂CClF₂, OCClFCF₂CF₃ oder CH=CF₂, besonders bevorzugt F oder OCF₃.

Die Verbindungen der Formel AA sind vorzugsweise ausgewählt aus der Gruppe bestehend aus folgenden Formeln: 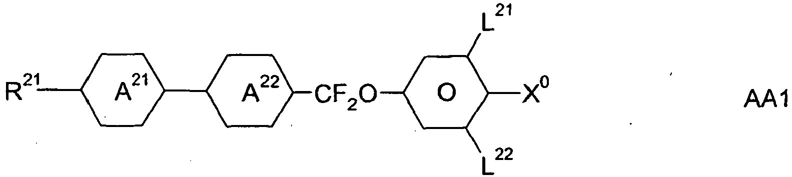 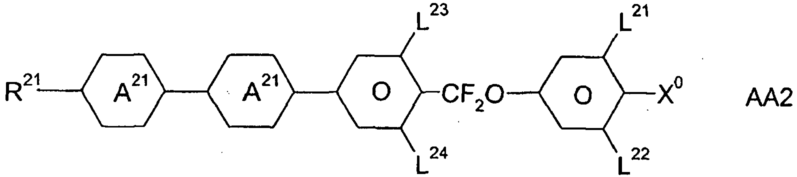 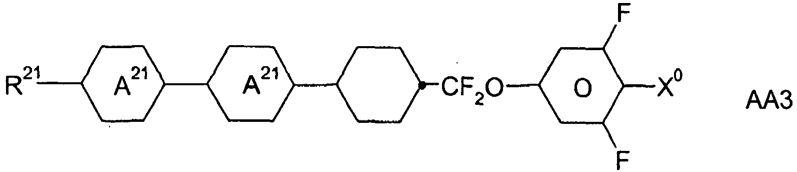 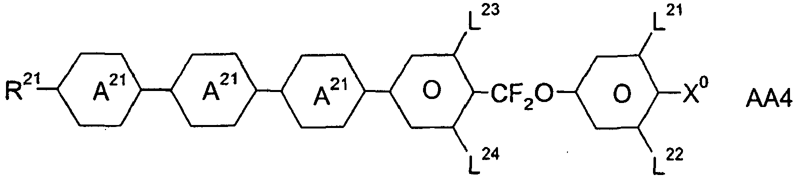 worin A²¹, R²¹, X⁰, L²¹ und L²² die in Formel AA angegebene Bedeutung besitzen, L²³ und L²⁴ jeweils unabhängig voneinander H oder F bedeuten, und X⁰ vorzugsweise F bedeutet. Besonders bevorzugt sind Verbindungen der Formeln AA1 und AA2.

Besonders bevorzugte Verbindungen der Formel AA1 sind ausgewählt aus der Gruppe bestehend aus den folgenden Unterformeln: 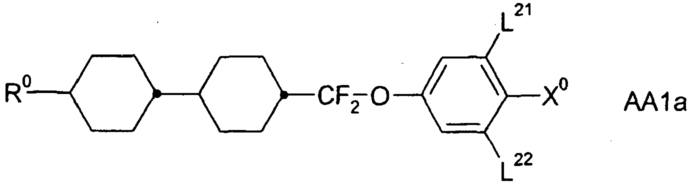 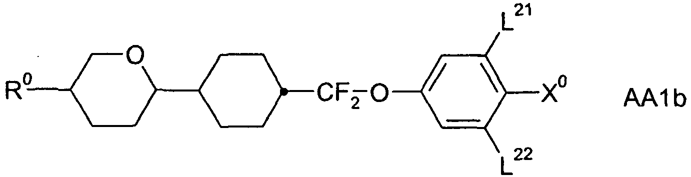 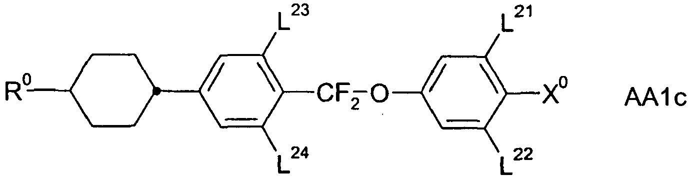 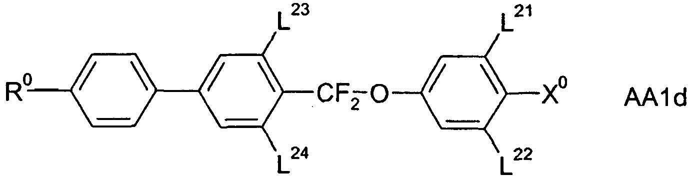 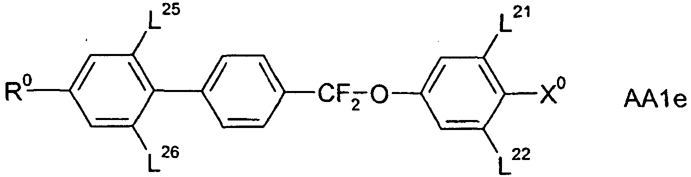 worin R⁰ die für R²¹ in Formel AA1 angegebene Bedeutung besitzt, X⁰, L²¹ und L²² die in Formel AA1 angegebene Bedeutung besitzen, L²³, L^{24,} L²⁵ und L²⁶ jeweils unabhängig voneinander H oder F bedeuten, und X⁰ vorzugsweise F bedeutet.

Ganz besonders bevorzugte Verbindungen der Formel AA1 sind ausgewählt aus der Gruppe bestehend aus den folgenden Unterformeln: 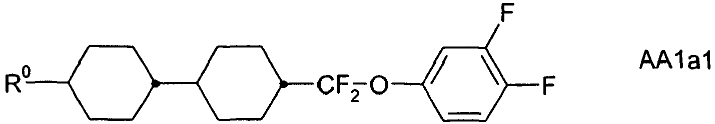 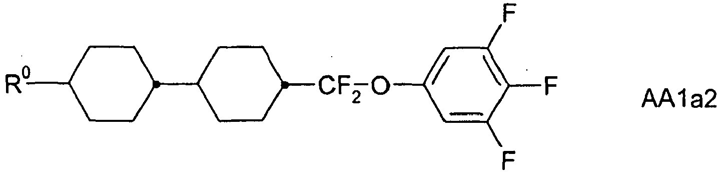 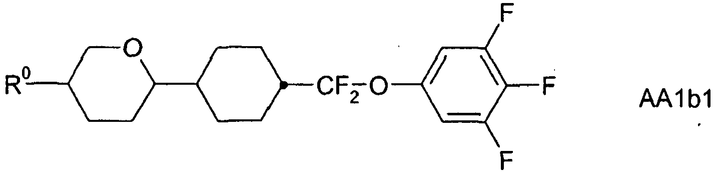 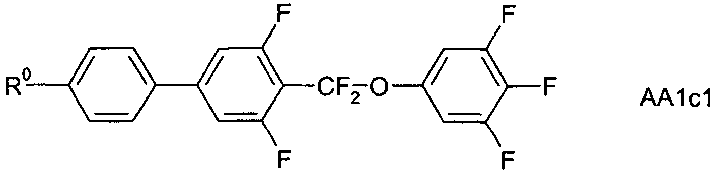 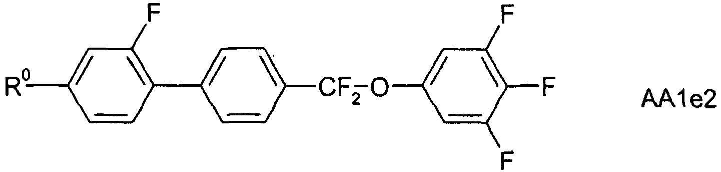 worin R⁰ die für R²¹ in Formel AA1 angegebene Bedeutung besitzt.

Besonders bevorzugte Verbindungen der Formel AA2 sind ausgewählt aus der Gruppe bestehend aus den folgenden Unterformeln: 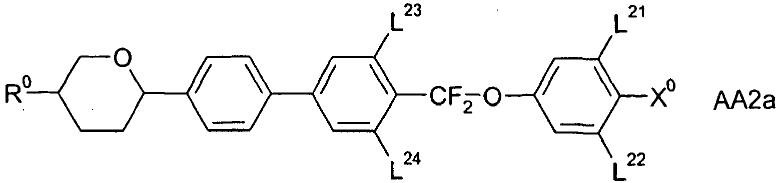 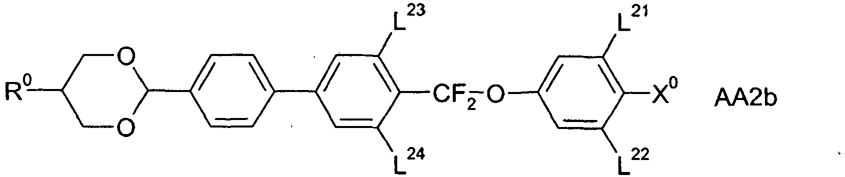 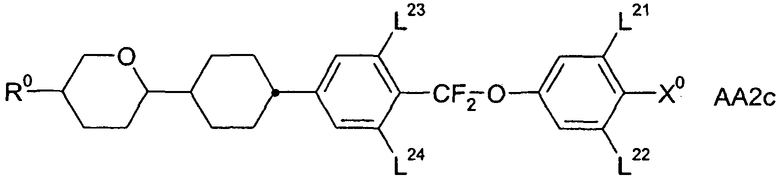 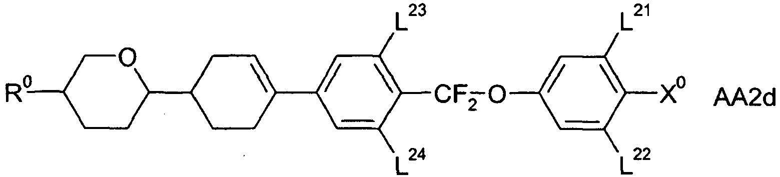 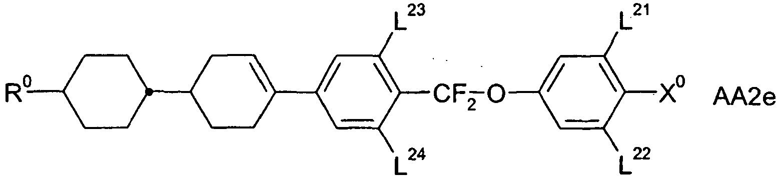 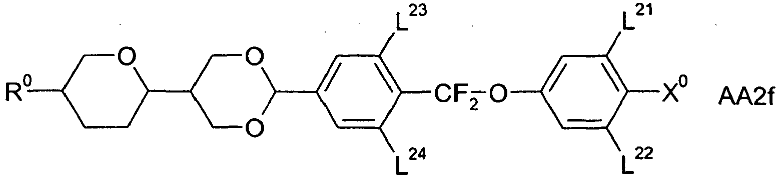 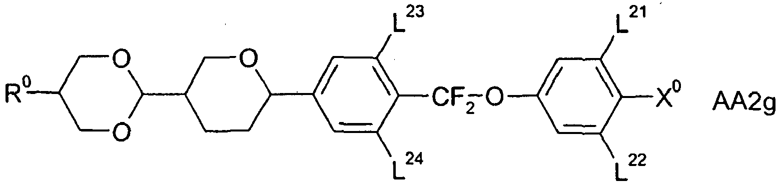 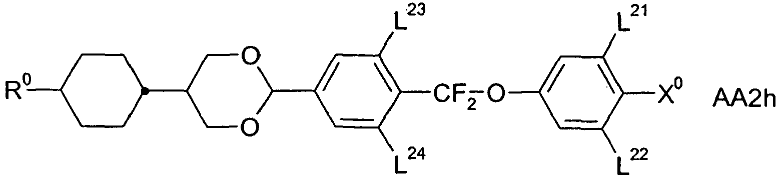 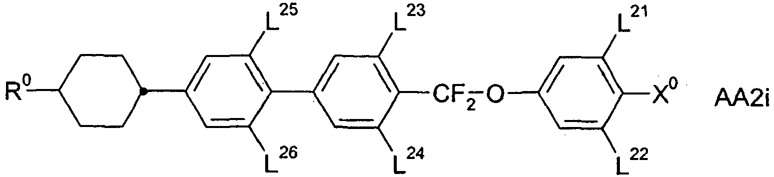 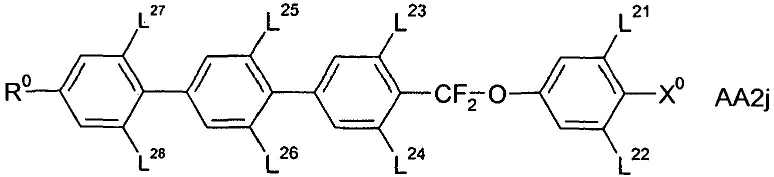 worin R⁰ die für R²¹ in Formel AA1 angegebene Bedeutung besitzt, X⁰, L²¹ und L²² die in Formel AA angegebene Bedeutung besitzen, L²³, L²⁴, L²⁵ und L²⁶ jeweils unabhängig voneinander H oder F bedeuten und X⁰ vorzugsweise F bedeutet.

Ganz besonders bevorzugte Verbindungen der Formel AA2 sind ausgewählt aus der Gruppe bestehend aus den folgenden Unterformeln: 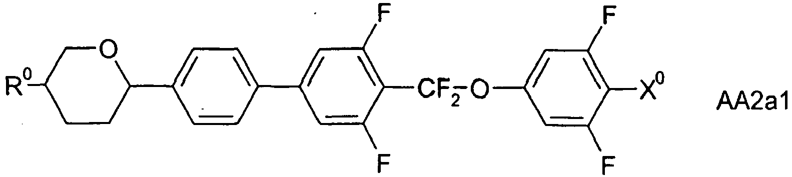 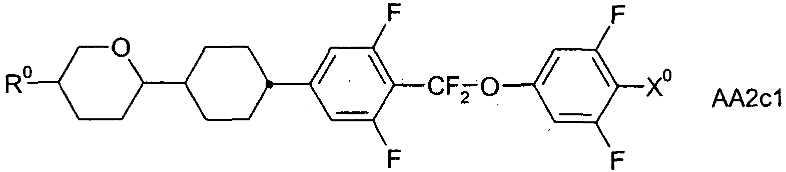 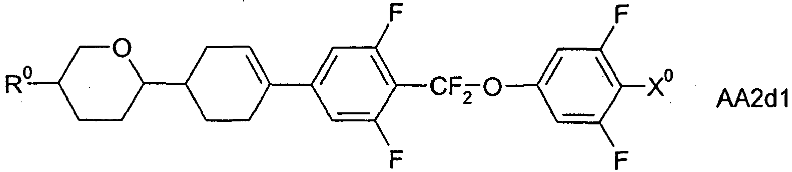 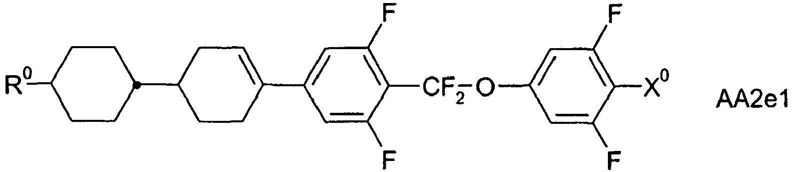 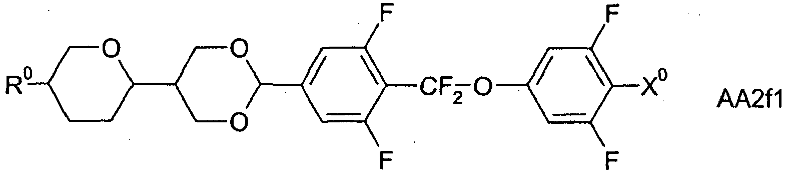 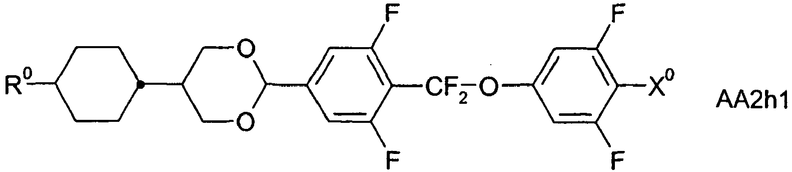 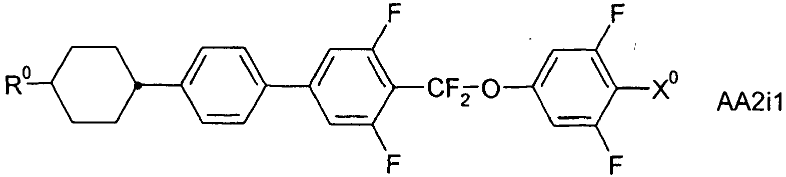 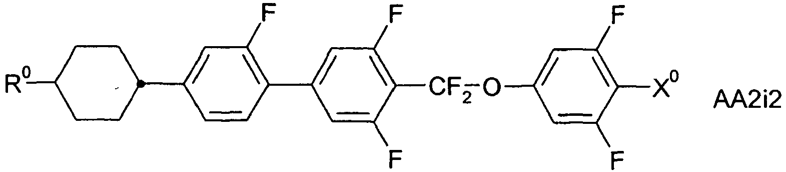 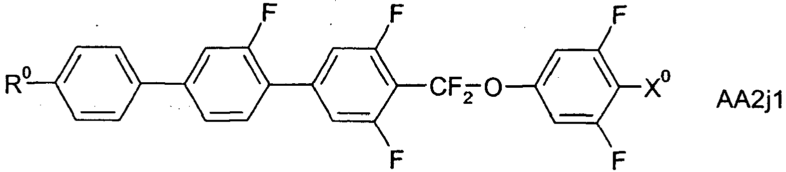 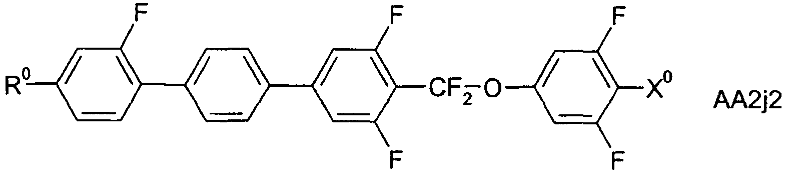 worin R⁰ die für R²¹ in Formel AA1 angegebene Bedeutung besitzt.

Besonders bevorzugte Verbindungen der Formel AA3 sind ausgewählt aus der Gruppe bestehend aus den folgenden Unterformeln: 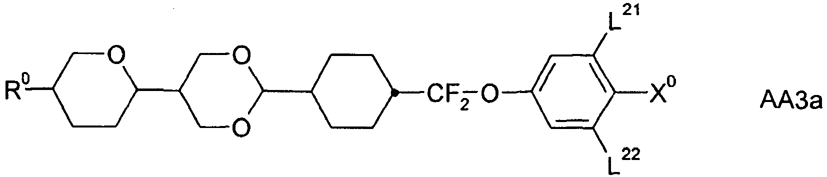 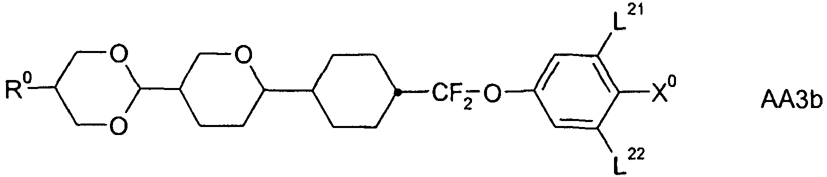 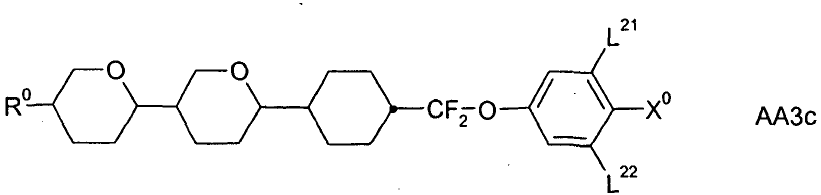 worin R⁰ die für R²¹ in Formel AA1 angegebene Bedeutung besitzt, X⁰, L²¹ und L²² die in Formel AA3 angegebene Bedeutung besitzen, und X⁰ vorzugsweise F bedeutet.

Besonders bevorzugte Verbindungen der Formel AA4 sind solche der folgenden Unterformel: 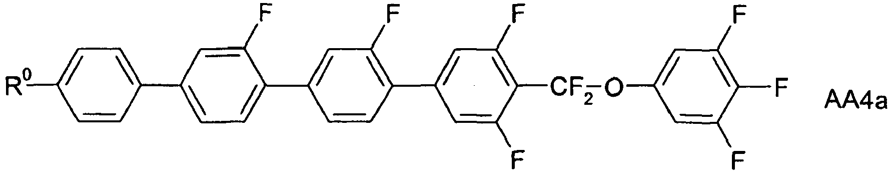 worin R⁰ die für R²¹ in Formel AA1 angegebene Bedeutung besitzt.

Die Verbindungen der Formel BB sind vorzugsweise ausgewählt aus der Gruppe bestehend aus folgenden Formeln: 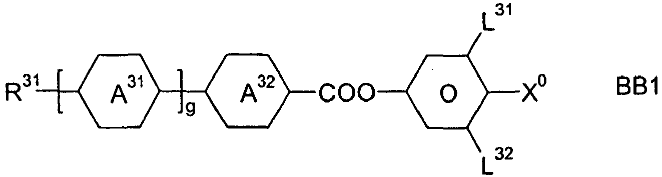 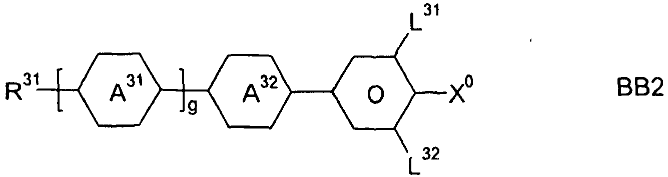 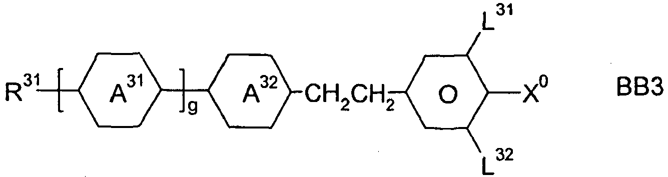 worin A³¹, A³², R³¹, X⁰, L³¹, L³² und g die in Formel BB angegebene Bedeutung besitzen, und X⁰ vorzugsweise F bedeutet. Besonders bevorzugt sind Verbindungen der Formeln BB1 und BB2.

Besonders bevorzugte Verbindungen der Formel BB1 sind ausgewählt aus der Gruppe bestehend aus den folgenden Unterformeln: 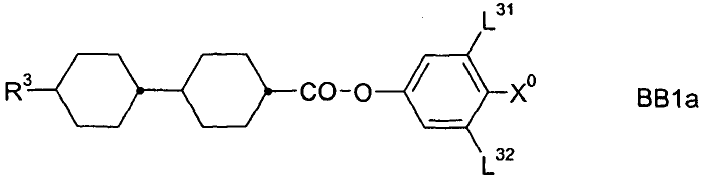 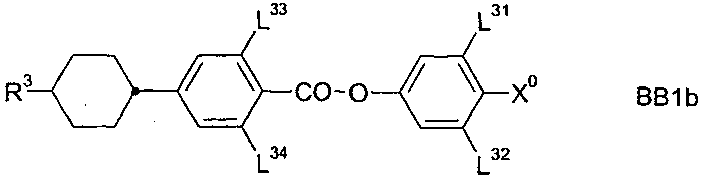 worin R³ die für R³¹ in Formel BB1 angegebene Bedeutung besitzt, X⁰, L³¹ und L³² die in Formel BB1 angegebene Bedeutung besitzen, und X⁰ vorzugsweise F bedeutet.

Ganz besonders bevorzugte Verbindungen der Formel BB1a sind ausgewählt aus der Gruppe bestehend aus den folgenden Unterformeln: 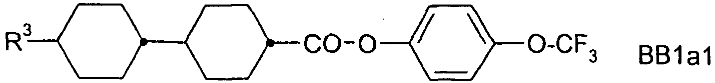 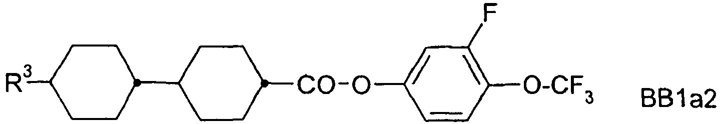 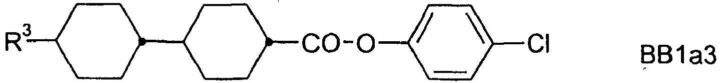 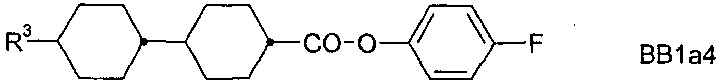 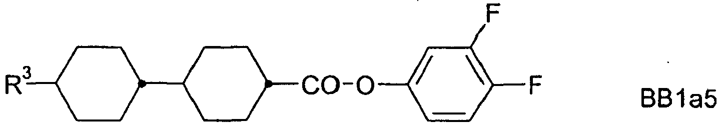 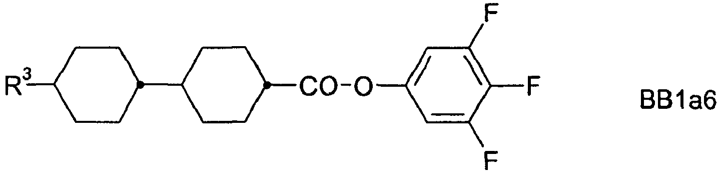 worin R³ die für R³¹ in Formel BB1 angegebene Bedeutung besitzt.

Ganz besonders bevorzugte Verbindungen der Formel BB1b sind ausgewählt aus der Gruppe bestehend aus den folgenden Unterformeln: 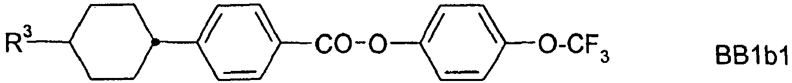 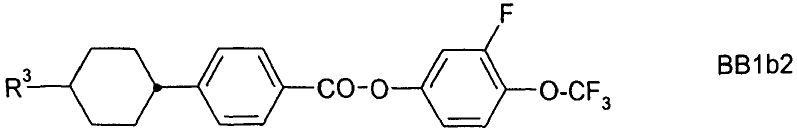 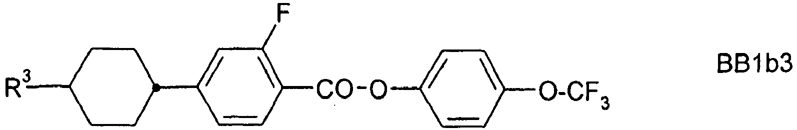 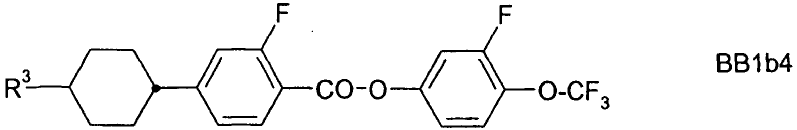 worin R³ die für R³¹ in Formel BB1 angegebene Bedeutung besitzt.

Besonders bevorzugte Verbindungen der Formel BB2 sind ausgewählt aus der Gruppe bestehend aus den folgenden Unterformeln: 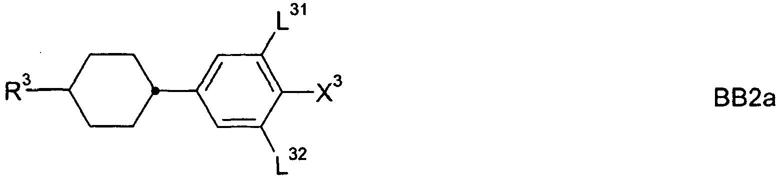 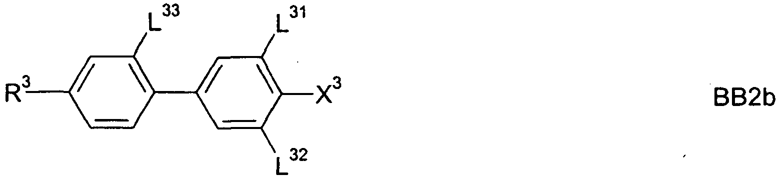 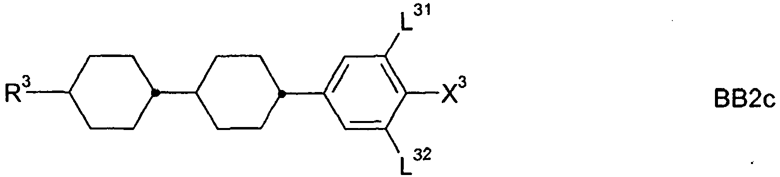 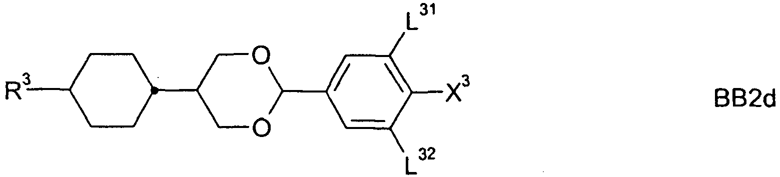 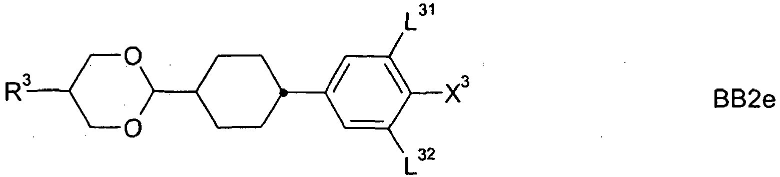 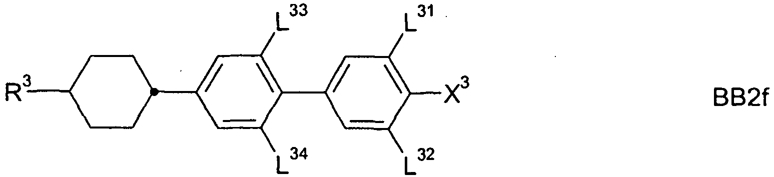 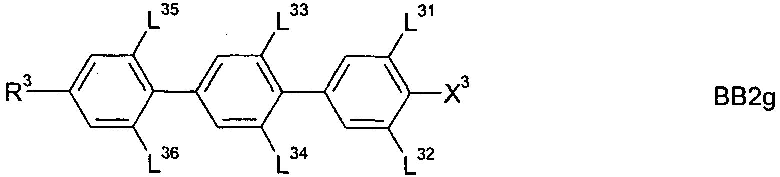 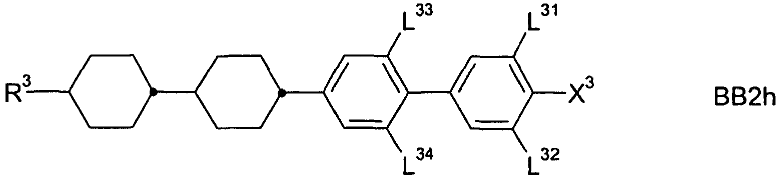 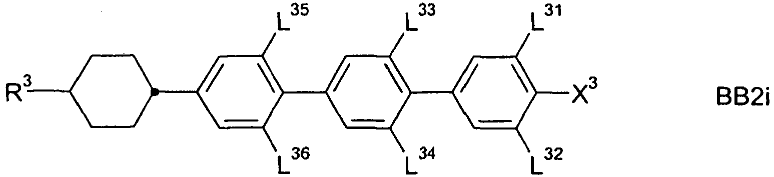 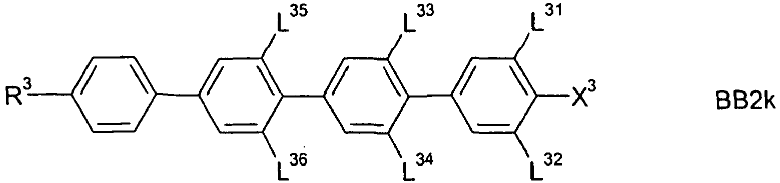 worin R⁰ die für R³¹ in Formel BB2 angegebene Bedeutung besitzt, X⁰, L³¹ und L³² die in Formel BB2 angegebene Bedeutung besitzen, L³³, L³⁴, L³⁵ und L³⁶ jeweils unabhängig voneinander H oder F bedeuten, und X⁰ vorzugsweise F bedeutet. Besonders bevorzugt sind Verbindungen der Formeln BB1 und BB2.

Ganz besonders bevorzugte Verbindungen der Formel BB2a sind ausgewählt aus der Gruppe bestehend aus den folgenden Unterformeln: 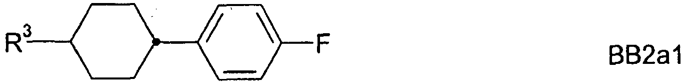 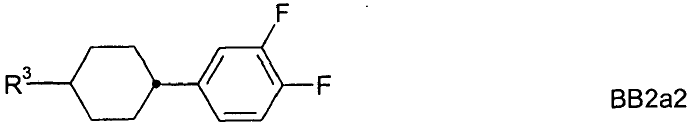 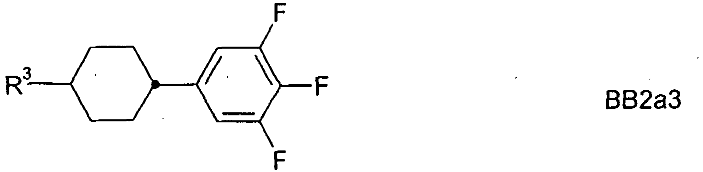 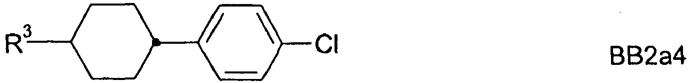 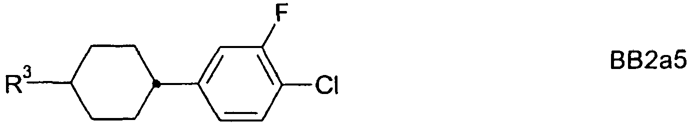 worin R³ die für R³¹ in Formel BB2 angegebene Bedeutung besitzt.

Ganz besonders bevorzugte Verbindungen der Formel BB2b sind ausgewählt aus der Gruppe bestehend aus den folgenden Unterformeln: 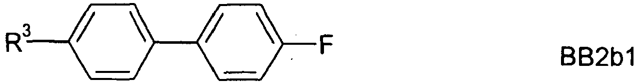 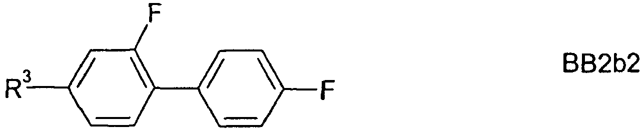 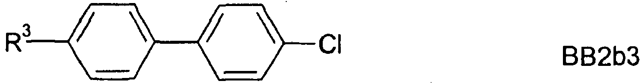 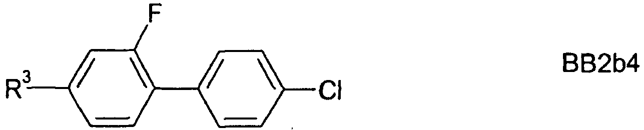 worin R³ die für R³¹ in Formel BB2 angegebene Bedeutung besitzt.

Ganz besonders bevorzugte Verbindungen der Formel BB2c sind ausgewählt aus der Gruppe bestehend aus den folgenden Unterformeln: 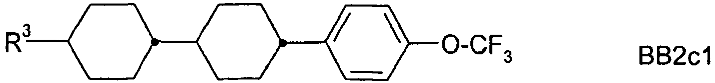 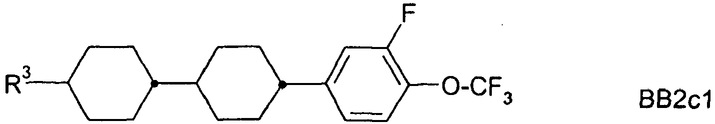 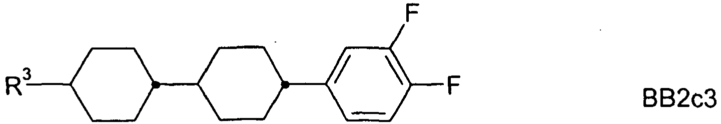 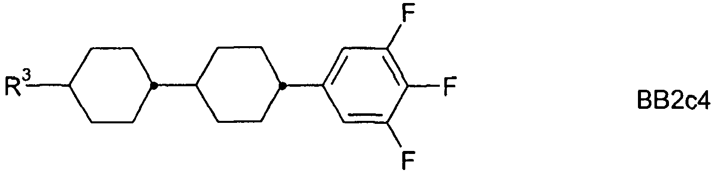 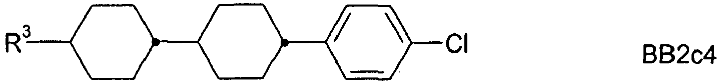 worin R³ die für R³¹ in Formel BB2 angegebene Bedeutung besitzt.

Ganz besonders bevorzugte Verbindungen der Formel BB2d und BB2e sind ausgewählt aus der Gruppe bestehend aus den folgenden Unterformeln: 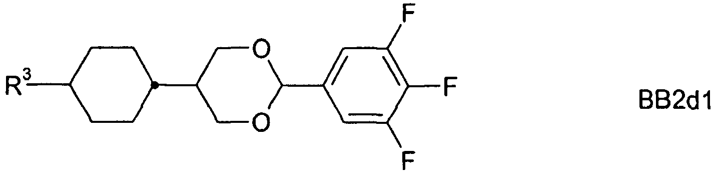 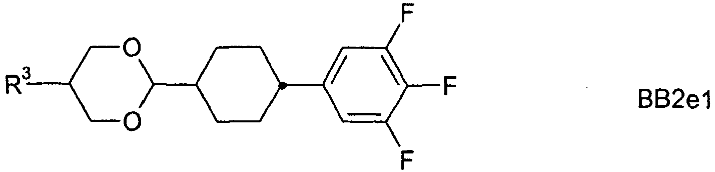 worin R³ die für R³¹ in Formel BB2 angegebene Bedeutung besitzt.

Ganz besonders bevorzugte Verbindungen der Formel BB2f sind ausgewählt aus der Gruppe bestehend aus den folgenden Unterformeln: 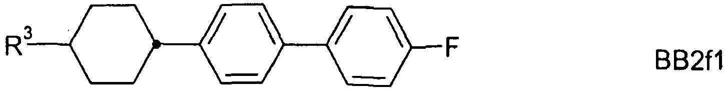 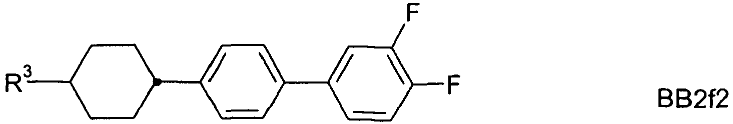 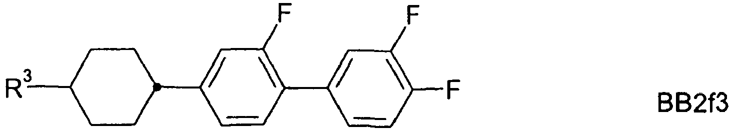 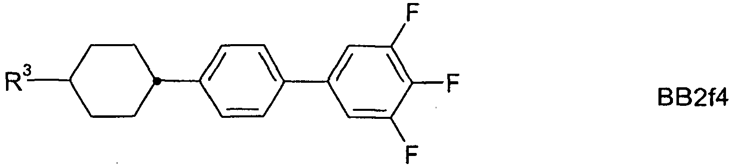 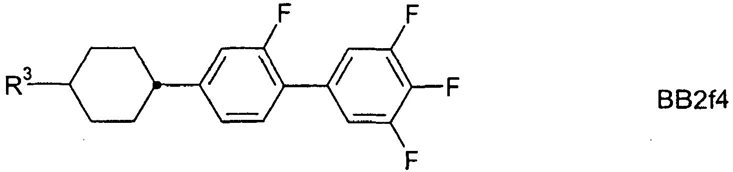 worin R³ die für R³¹ in Formel BB2 angegebene Bedeutung besitzt.

Ganz besonders bevorzugte Verbindungen der Formel BB2g sind ausgewählt aus der Gruppe bestehend aus den folgenden Unterformeln: 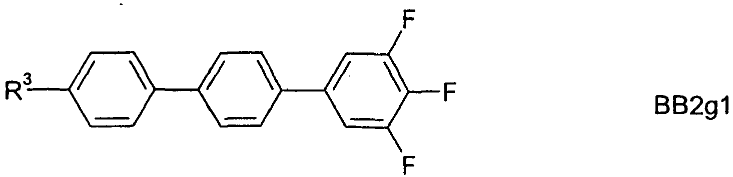 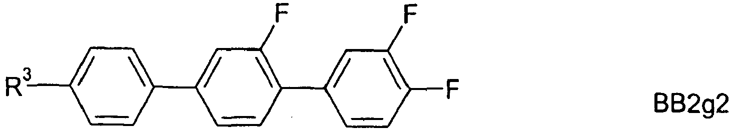 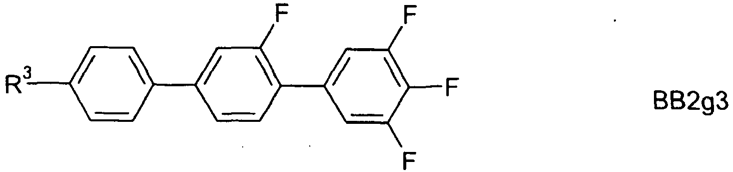 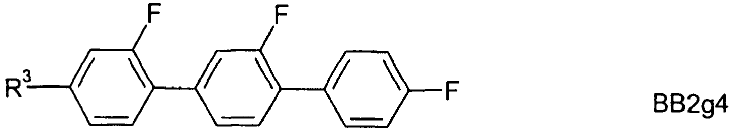 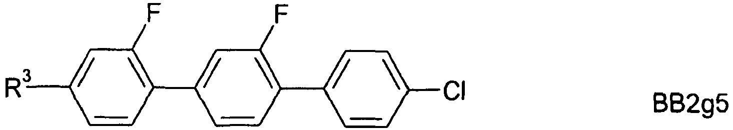 worin R³ die für R³¹ in Formel BB2 angegebene Bedeutung besitzt.

Ganz besonders bevorzugte Verbindungen der Formel BB2h sind ausgewählt aus der Gruppe bestehend aus den folgenden Unterformeln: 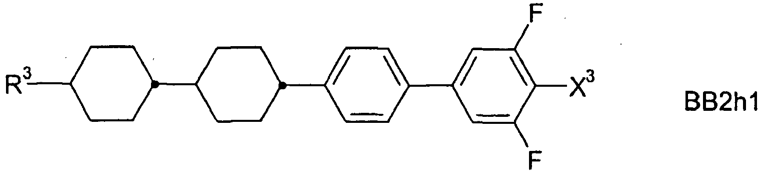 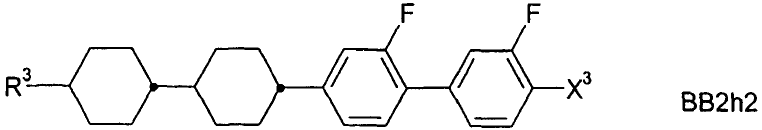 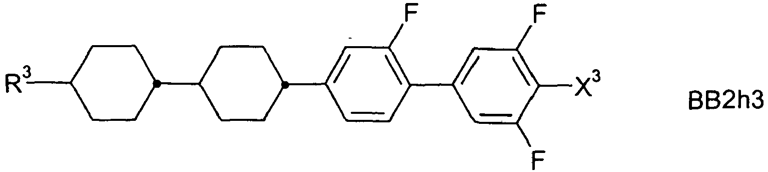 worin R³ die für R³¹ in Formel BB2 angegebene Bedeutung besitzt.

Ganz besonders bevorzugte Verbindungen der Formel BB2i sind ausgewählt aus der Gruppe bestehend aus den folgenden Unterformeln: 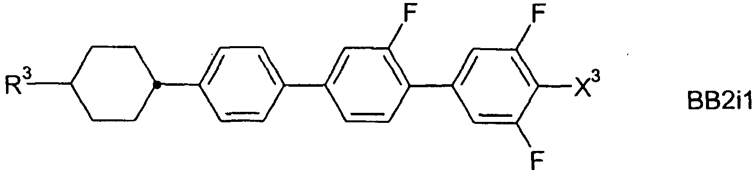 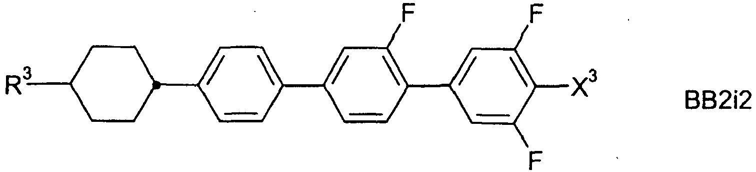 worin R³ die für R³¹ in Formel BB2 angegebene Bedeutung besitzt.

Ganz besonders bevorzugte Verbindungen der Formel BB2k sind ausgewählt aus der Gruppe bestehend aus den folgenden Unterformeln: 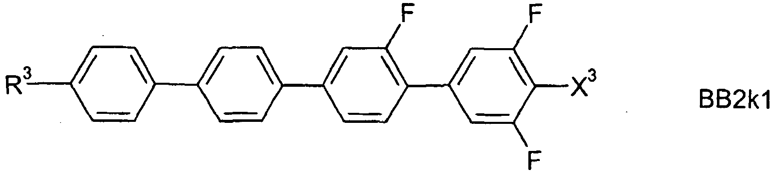 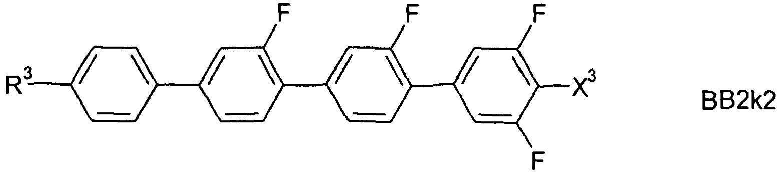 worin R³ die für R³¹ in Formel BB2 angegebene Bedeutung besitzt.

Alternativ oder zusätzlich zu den Verbindungen der Formeln BB1 und/oder BB2 können die FK-Medien eine oder mehrere Verbindungen der Formel BB3 wie oben definiert enthalten.

Besonders bevorzugte Verbindungen der Formel BB3 sind ausgewählt aus der Gruppe bestehend aus den folgenden Unterformeln: 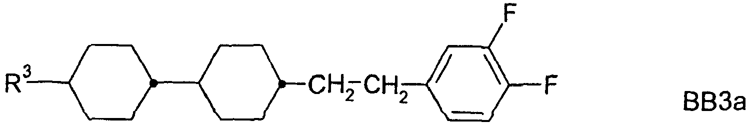 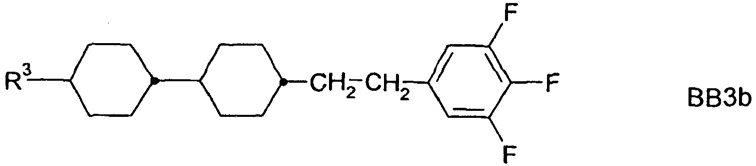 worin R³ die für R³¹ in Formel BB3 angegebene Bedeutung.

Vorzugsweise enthalten die FK-Medien dieser zweiten bevorzugten Ausführungsform zusätzlich zu den Verbindungen der Formel AA und/oder BB eine oder mehrere dielektrisch neutrale Verbindungen mit einer dielektrischen Anisotropie im Bereich von -1,5 bis +3, bevorzugt ausgewählt aus der Gruppe der Verbindungen der Formel CC wie oben definiert.

Besonders bevorzugte Verbindungen der Formel CC sind ausgewählt aus der Gruppe bestehend aus den folgenden Unterformeln: 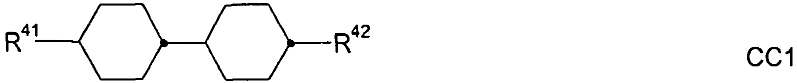 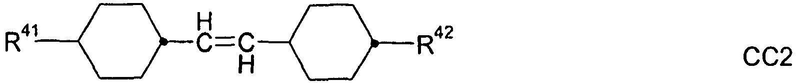 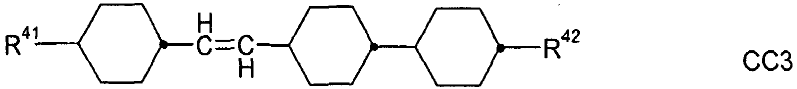 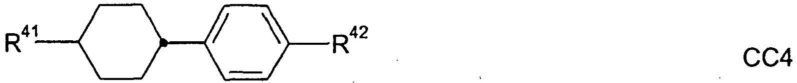 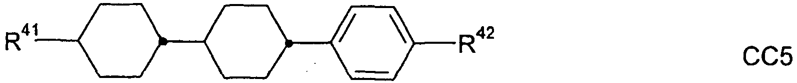 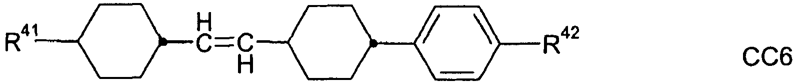 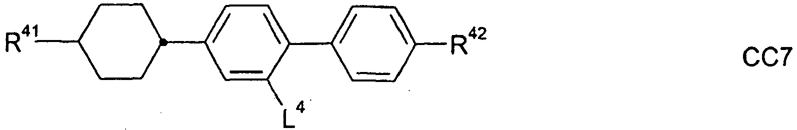 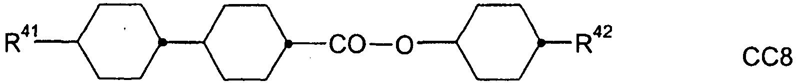 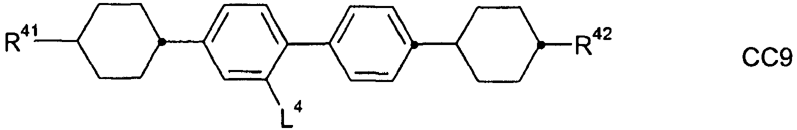 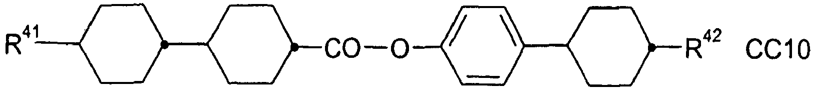 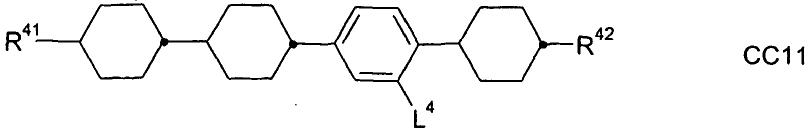 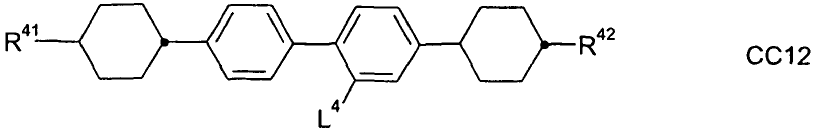 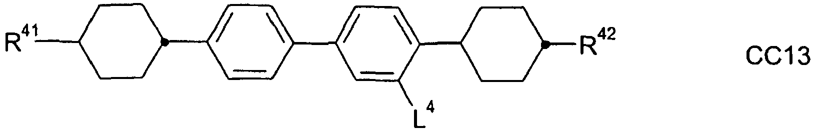 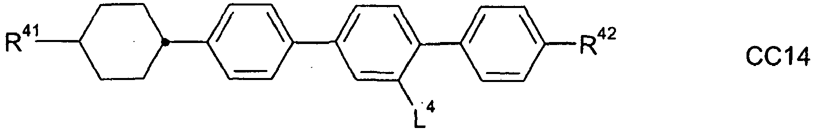 worin R⁴¹ und R⁴² die in Formel CC angegbenen Bedeutungen besitzen, und vorzugsweise jeweils unabhängig voneinander Alkyl, Alkoxy, fluoriertes Alkyl oder fluoriertes Alkoxy mit 1 bis 7 C-Atomen, Alkenyl, Alkenyloxy, Alkoxyalkyl oder fluoriertes Alkenyl mit 2 bis 7 C-Atomen bedeuten und L⁴ H oder F bedeutet.

Vorzugsweise enthalten die FK-Medien dieser zweiten bevorzugten Ausführungsform, zusätzlich oder alternativ zu den dielektrisch neutralen Verbindungen der Formel CC, eine oder mehrere dielektrisch neutrale Verbindungen mit einer dielektrischen Anisotropie im Bereich von -1,5 bis +3, ausgewählt aus der Gruppe der Verbindungen der Formel DD 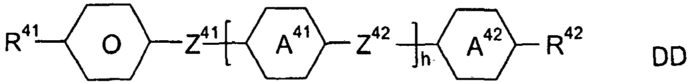 worin A⁴¹, A⁴², Z⁴¹, Z⁴², R⁴¹, R⁴² und h die in Formel CC angegebene Bedeutung besitzen.

Besonders bevorzugte Verbindungen der Formel DD sind ausgewählt aus der Gruppe bestehend aus den folgenden Unterformeln: 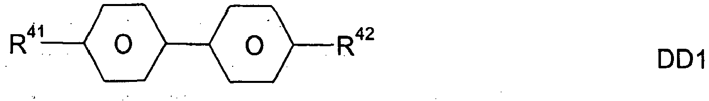 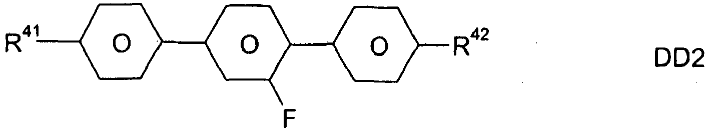 worin R⁴¹ und R⁴² die in Formel DD angegebenen Bedeutungen besitzen und R⁴¹ vorzugsweise Alkyl bedeutet und in Formel DD1 R⁴² vorzugsweise Alkenyl, vorzugsweise -(CH₂)₂-CH=CH-CH₃, und in Formel DD2 R⁴² vorzugsweise Alkyl -(CH₂)₂-CH=CH₂ oder -(CH₂)₂-CH=CH-CH₃ bedeutet.

Die Verbindungen der Formeln AA und BB werden vorzugsweise in einer Konzentration von 2 % bis 60 %, stärker bevorzugt von 3 % bis 35 % und ganz besonders bevorzugt von 4 % bis 30 % der Gesamtmischung verwendet.

Die Verbindungen der Formeln CC und DD werden vorzugsweise in einer Konzentration von 2 % bis 70 %, stärker bevorzugt von 5 % bis 65 %, noch stärker bevorzugt von 10 % bis 60 % und ganz besonders bevorzugt von 10 %, bevorzugt von 15 %, bis 55 % der Gesamtmischung verwendet.

Die Kombination von FK-Host-Mischungen und FK-Verbindungen der oben genannten bevorzugten Ausführungsformen mit den oben beschriebenen polymerisierten Verbindungen bewirkt in den erfindungsgemäßen FK-Medien niedrige Schwellenspannungen, niedrige Rotationsviskositäten und sehr gute Tieftemperaturstabilitäten bei gleichbleibend hohen Klärpunkten und hohen HR-Werten, und erlaubt die schnelle Einstellung eines besonders niedrigen pretilt-Winkels in PSA-Anzeigen. Insbesondere zeigen die FK-Medien in PSA-Anzeigen im Vergleich zu den Medien aus dem Stand der Technik deutlich verringerte Schaltzeiten, insbesondere auch der Graustufenschaltzeiten.

Vorzugsweise weist die Flüssigkristallmischung einen nematischen Phasenbereich von mindestens 80 K, besonders bevorzugt von mindestens 100 K, und eine Rotationsviskosität von nicht mehr als 250, vorzugsweise nicht mehr als 200 mPa·s, bei 20°C auf.

In den erfindungsgemäßen Anzeigen des VA-Typs sind die Moleküle in der Schicht des FK-Mediums im ausgeschalteten Zustand senkrecht zu den Elektrodenflächen (homöotrop) oder gekippt homöotrop (engl. "tilted") orientiert. Bei Anlegen einer elektrischen Spannung an die Elektroden findet eine Umorientierung der FK-Moleküle mit den Moleküllängsachsen parallel zu den Elektrodenflächen statt.

Erfindungsgemäße FK-Medien basierend auf Verbindungen mit negativer dielektrischer Anisotropie gemäß der ersten bevorzugten Ausführungsform, insbesondere zur Verwendung in Anzeigen des PSA-VA-Typs, weisen eine negative dielektrische Anisotropie Δε auf, vorzugsweise von etwa -0,5 bis -10, insbesondere von etwa -2,5 bis -7,5 bei 20°C und 1 kHz.

Die Doppelbrechung Δn in erfindungsgemäßen FK-Medien zur Verwendung in Anzeigen des VA-Typs liegt vorzugsweise unter 0,16, besonders bevorzugt zwischen 0,06 und 0,14, insbesondere zwischen 0,07 und 0,12.

In den erfindungsgemäßen Anzeigen des OCB-Typs sind die Moleküle in der Schicht des FK-Mediums eine "bend"-Orientierung auf. Bei Anlegen einer elektrischen Spannung findet eine Umorientierung der FK-Moleküle mit den Moleküllängsachsen senkrecht zu den Elektrodenflächen statt. Erfindungsgemäße FK-Medien, zur Verwendung in Anzeigen des PSA-OCB-Typs sind vorzugsweise solche basierend auf Verbindungen mit positiver dielektrischer Anisotropie gemäß der zweiten bevorzugten Ausführungsform, und weisen vorzugsweise eine positive dielektrische Anisotropie Δε von etwa +4 bis +17 bei 20°C und 1 kHz auf.

Die Doppelbrechung Δn in erfindungsgemäßen FK-Medien zur Verwendung in Anzeigen des OCB-Typs liegt vorzugsweise zwischen 0,14 und 0,22, insbesondere zwischen 0,16 und 0,22.

Erfindungsgemäße FK-Medien, basierend auf Verbindungen mit positiver dielektrischer Anisotropie gemäß der zweiten bevorzugten Ausführungsform, zur Verwendung in Anzeigen des PSA-TN-, PSA-Posi-VA-, PSA-IPS- oder PSA-FFS-Typs haben vorzugsweise eine positive dielektrische Anisotropie zwischen +2 und +30, insbesondere zwischen +3 und +20.

Die Doppelbrechung Δn in erfindungsgemäßen FK-Medien zur Verwendung in Anzeigen des PSA-TN-, PSA-Posi-VA-, PSA-IPS- oder PSA-FFS-Typs liegt vorzugsweise zwischen 0,07 und 0,15, insbesondere zwischen 0,08 und 0,13.

Die erfindungsgemäßen FK-Medien können auch weitere, dem Fachmann bekannte und in der Literatur beschriebene Zusätze oder Additive enthalten, wie beispielsweise Polymerisationsinitiatoren, Inhibitoren, Stabilisatoren, oberflächenaktive Substanzen, Nanopartikel oder chirale Dotierstoffe. Diese können polymerisierbar oder unpolymerisierbar sein. Polymerisierbare Additive werden dementsprechend der polymerisierbaren Komponente oder Komponente A) zugerechnet. Unpolymerisierbare Additive werden dementsprechend der unpolymerisierbaren Komponente oder Komponente B) zugerechnet.

Die FK.Medien können beispielsweise einen oder mehrere chirale Dotierstoffe enthalten, vorzusgweise solche ausgewählt aus der Gruppe bestehend aus Verbindungen der nachfolgenden Tabelle B.

Ferner können den FK.Medien beispielsweise 0 bis 15 Gew.-% pleochroitische Farbstoffe zugesetzt werden, ferner Nanopartikel, Leitsalze, vorzugsweise Ethyl-dimethyldodecylammonium-4-hexoxybenzoat, Tetrabutylammoniumtetraphenylborat oder Komplexsalze von Kronenethern (vgl. z.B. Haller et al., Mol. Cryst. Liq. Cryst. 24, 249-258 (1973)) zur Verbesserung der Leitfähigkeit, oder Substanzen zur Veränderung der dielektrischen Anisotropie, der Viskosität und/oder der Orientierung der nematischen Phasen. Derartige Substanzen sind z.B. in DE-A 22 09 127, 22 40 864, 23 21 632, 23 38 281, 24 50 088, 26 37 430 und 28 53 728 beschrieben.

Die einzelnen Komponenten der bevorzugten Ausführungsformen a)-z) der erfindungsgemäßen FK-Medien sind entweder bekannt, oder ihre Herstellungsweisen sind für den einschlägigen Fachmann aus dem Stand der Technik ohne weiteres abzuleiten, da sie auf in der Literatur beschriebenen Standardverfahren basieren. Entsprechende Verbindungen der Formel CY werden beispielsweise in EP-A-0 364 538 beschrieben. Entsprechende Verbindungen der Formel ZK werden beispielsweise in DE-A-26 36 684 und DE-A-33 21 373 beschrieben.

Die Herstellung der erfindungsgemäß verwendbaren FK-Medien erfolgt in an sich üblicher Weise, beispielsweise indem man eine oder mehrere der oben genannten Verbindungen mit einer oder mehreren polymerisierbaren Verbindungen wie oben definiert, und ggf. mit weiteren flüssigkristallinen Verbindungen und/oder Additiven mischt. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Das Verfahren zur Herstellung der erfindungsgemäßen FK-Medien ist ein weiterer Gegenstand der Erfindung.

Es versteht sich für den Fachmann von selbst, daß die erfindungsgemäßen FK-Medien auch Verbindungen enthalten können, worin beispielsweise H, N, O, Cl, F durch die entsprechenden Isotope ersetzt sind.

Der Aufbau der erfindungsgemäßen FK-Anzeigen entspricht der für PSA-Anzeigen üblichen Geometrie, wie er im eingangs zitierten Stand der Technik beschrieben ist. Es sind Geometrien ohne Protrusions bevorzugt, insbesondere diejenigen, bei denen darüber hinaus die Elektrode auf der Colour Filter-Seite unstrukturiert ist und lediglich die Elektrode auf der TFT-Seite Schlitze aufweist. Besonders geeignete und bevorzugte Elektrodenstrukturen für PSA-VA-Anzeigen sind beispielsweise in US 2006/0066793 A1 beschrieben.

Die folgenden Beispiele erläutern die vorliegende Erfindung ohne sie zu begrenzen. Sie zeigen dem Fachmann jedoch bevorzugte Mischungskonzepte mit bevorzugt einzusetzenden Verbindungen und deren jeweiligen Konzentrationen sowie deren Kombinationen miteinander. Außerdem illustrieren die Beispiele, welche Eigenschaften und Eigenschftskombinationen zugänglich sind.

Folgende Abkürzungen werden verwendet:
(n, m, z: jeweils unabhängig voneinander 1, 2, 3, 4, 5 oder 6)

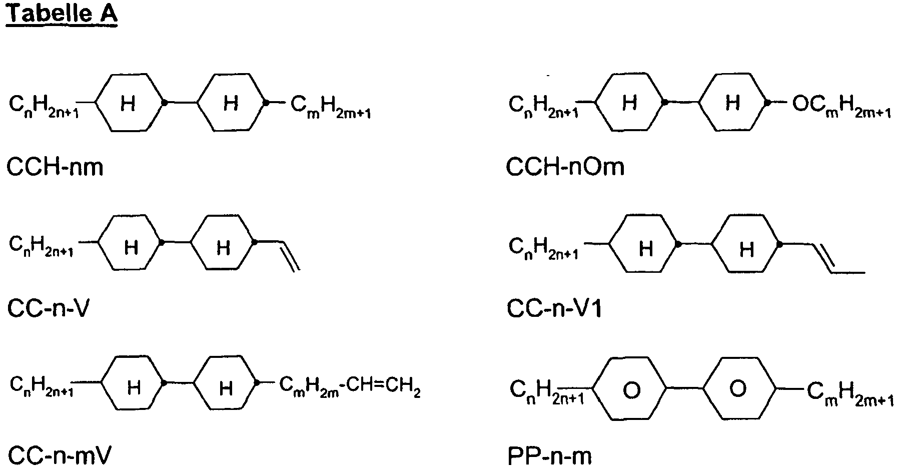

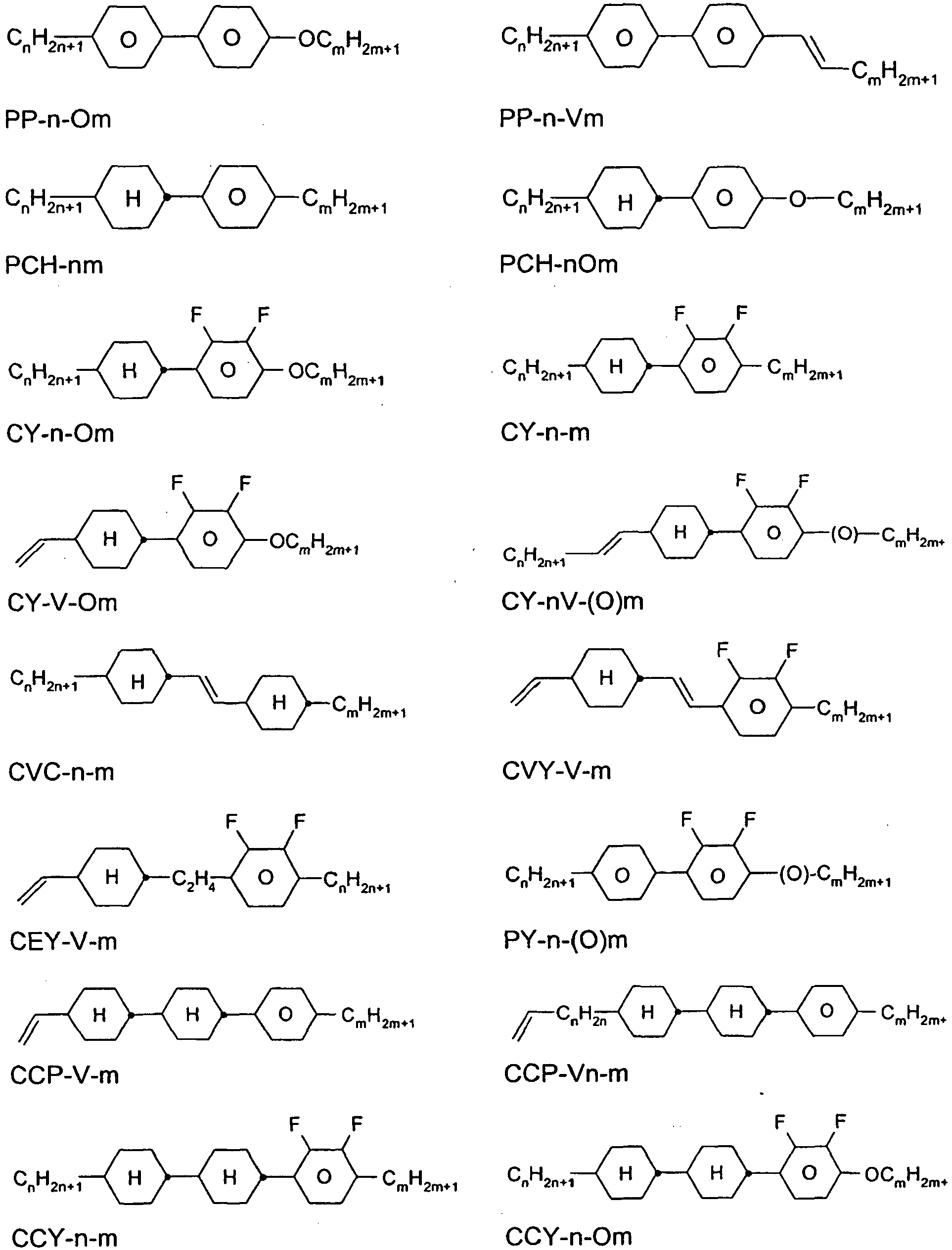

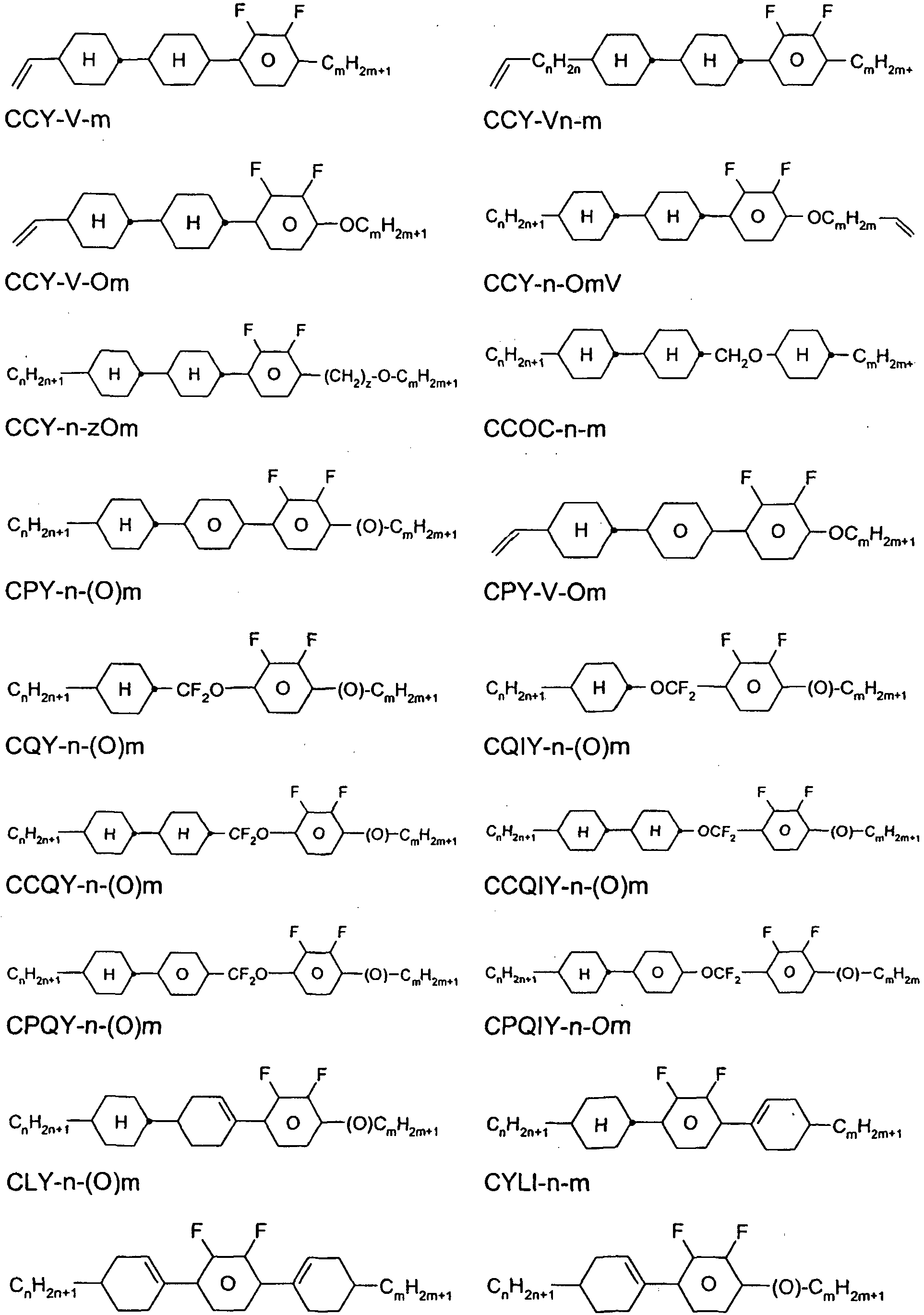

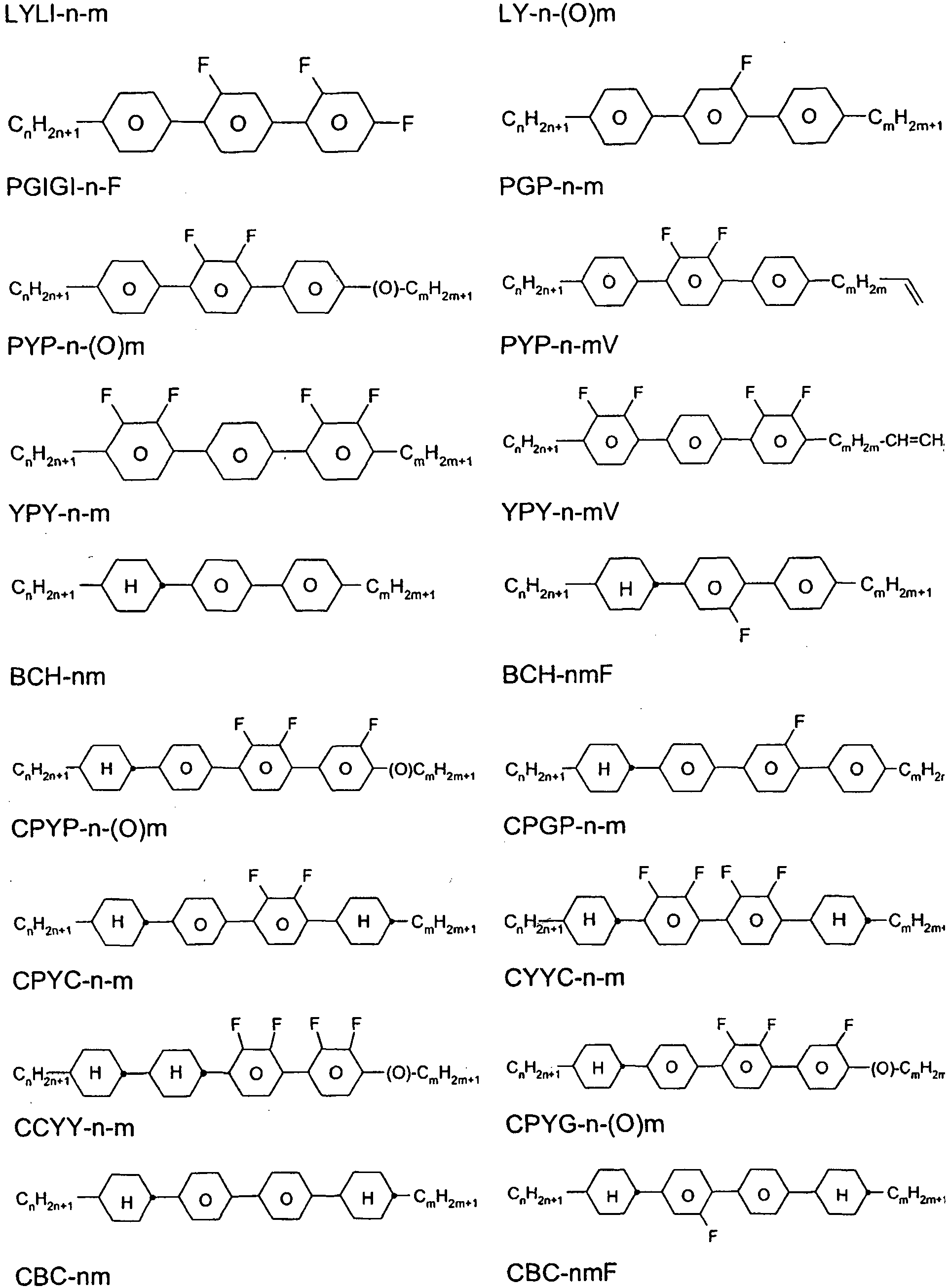

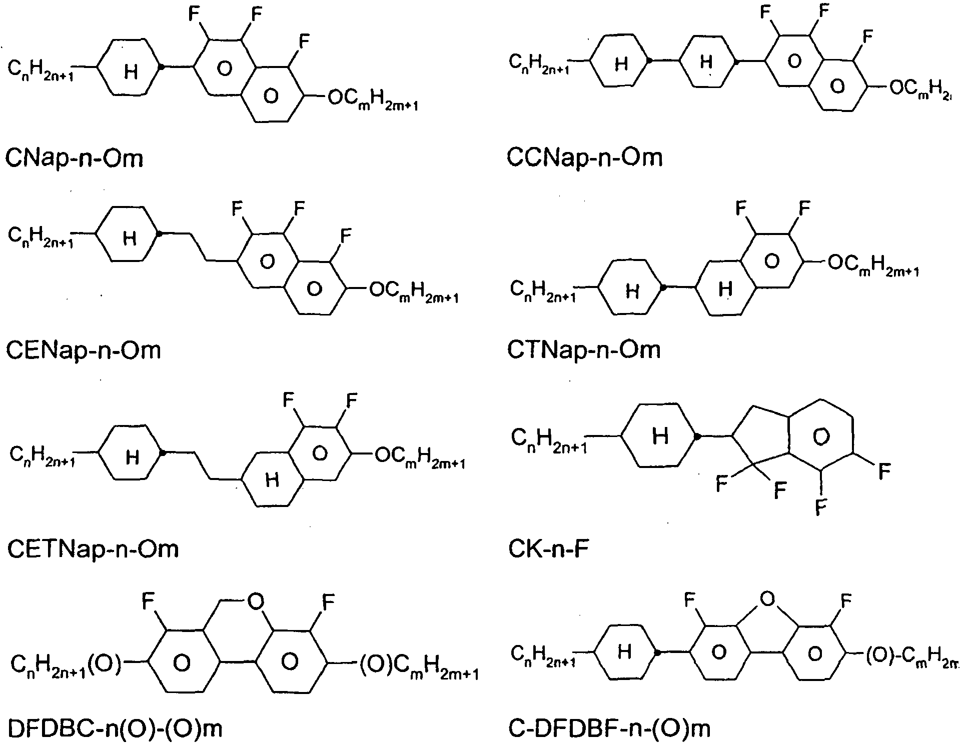

In einer bevorzugten Ausführungsform der vorliegenden Erfindung enthalten die erfindungsgemäßen FK-Medien eine oder mehrere Verbindungen ausgewählt aus der Guppe bestehend aus Verbindungen der Tabelle A.

**Tabelle B**

| In der Tabelle B werden mögliche chirale Dotierstoffe angegeben, die den erfindungsgemäßen FK-Medien zugesetzt werden können. | |
|---|---|
| 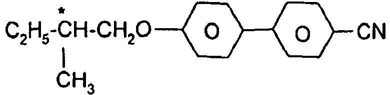 | 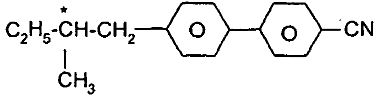 |
| **C 15** | **CB 15** |
| 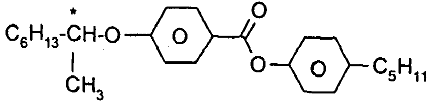 | 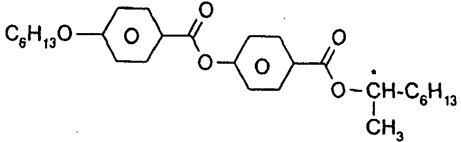 |
| **CM 21** | **R/S-811** |
| 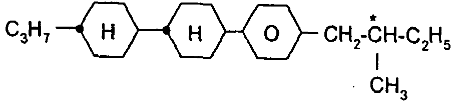 | 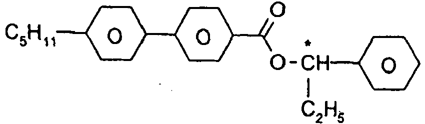 |
| **CM 44** | **CM 45** |
| 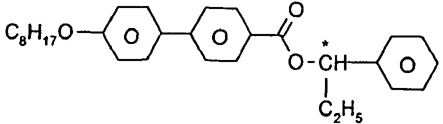 | 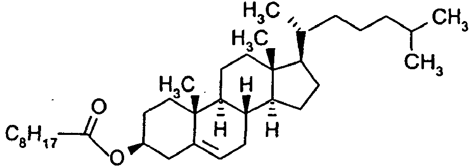 |
| **CM 47** | **CM** |
| 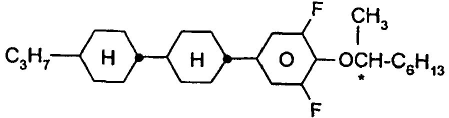 | 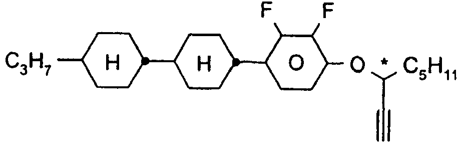 |
| **R/S-2011** | **R/S-3011** |
| 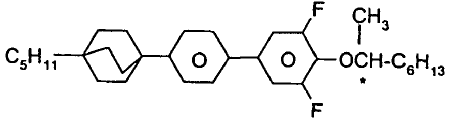 | 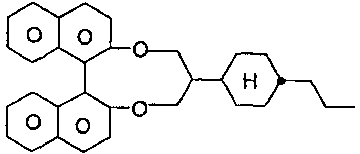 |
| **R/S-4011** | **R/S-5011** |
| 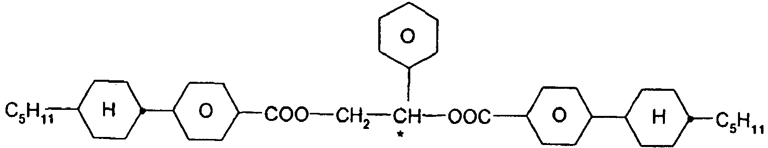 | |
| **R/S-1011** | |

Vorzugsweise enthalten die FK-Medien 0 bis 10 Gew.%, insbesondere 0,01 bis 5 Gew.%, besonders bevorzugt 0,1 bis 3 Gew.% an Dotierstoffen. Vorzugsweise enthalten die FK-Medien einen oder mehrere Dotierstoffe ausgewählt aus der Guppe bestehend aus Verbindungen der Tabelle B.

**Tabelle C**

| | |
|---|---|
| In der Tabelle C werden mögliche Stabilisatoren angegeben, die den erfindungsgemäßen FK-Medien zugesetzt werden können. | |
| (n bedeutet hier eine ganze Zahl von 1 bis 12, vorzugsweise 1, 2, 3, 4, 5, 6, 7 oder 8, endständige Methylgruppen sind nicht gezeigt). | |
| 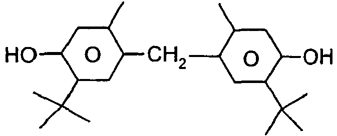 | 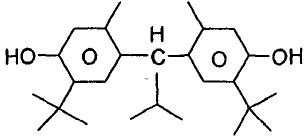 |
| 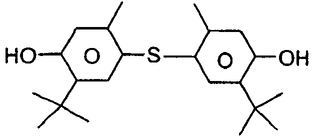 | 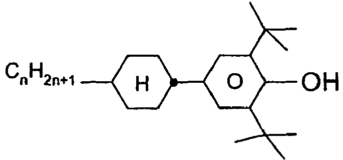 |
| 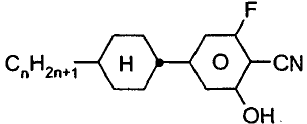 | 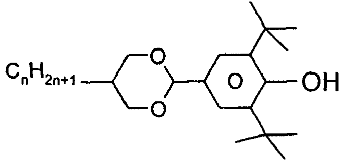 |
| 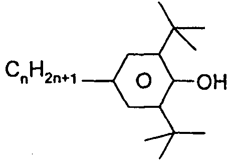 | 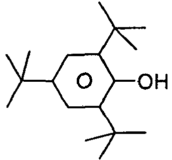 |
| 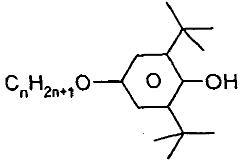 | 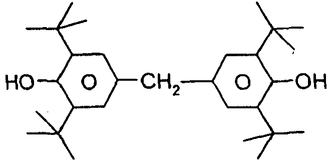 |
| 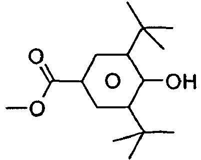 | 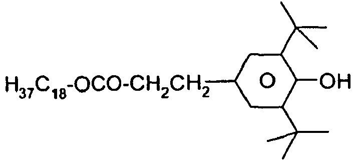 |
| 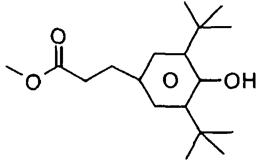 | 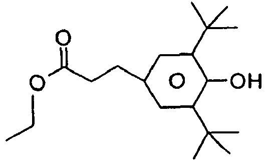 |
| 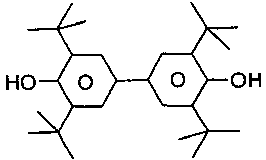 | 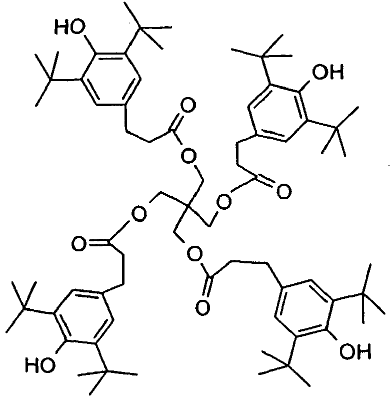 |
| 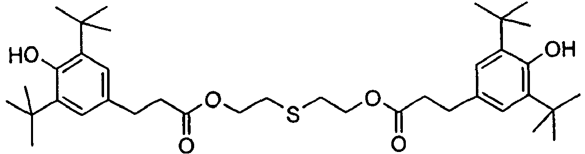 | |
| 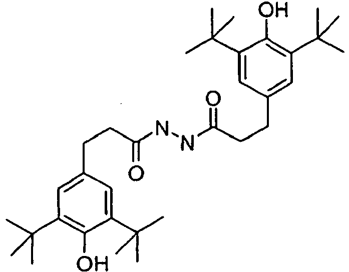 | 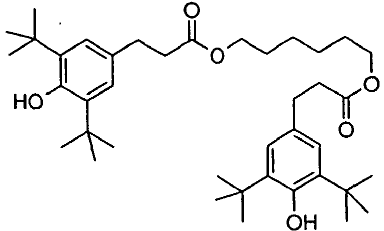 |
| 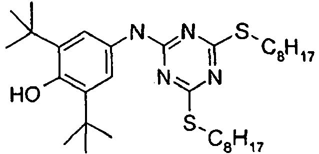 | 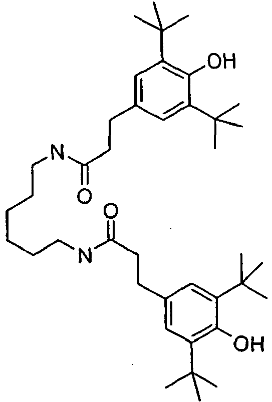 |
| 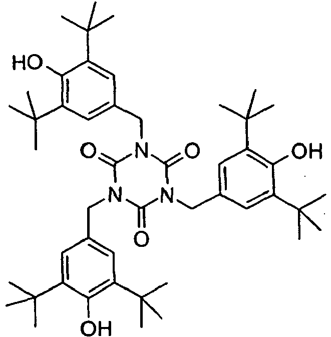 | 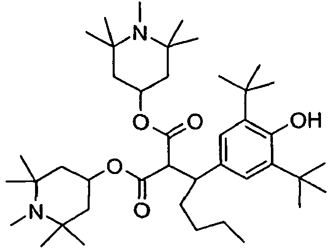 |
| 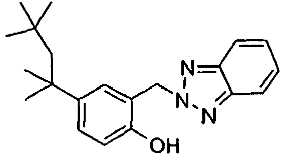 | 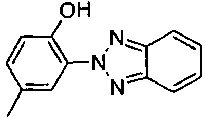 |
| 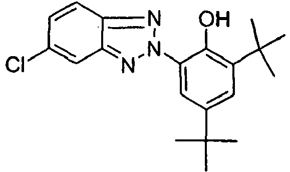 | 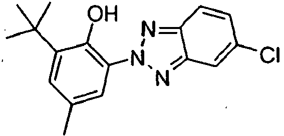 |
| 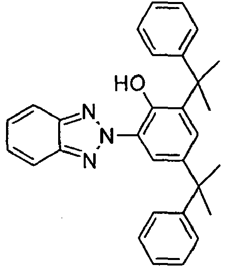 | 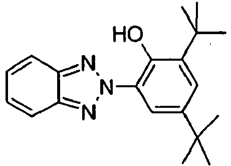 |
| 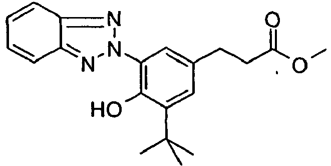 | 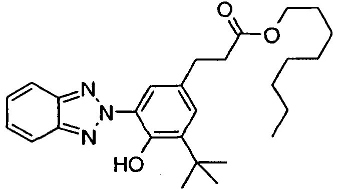 |
| 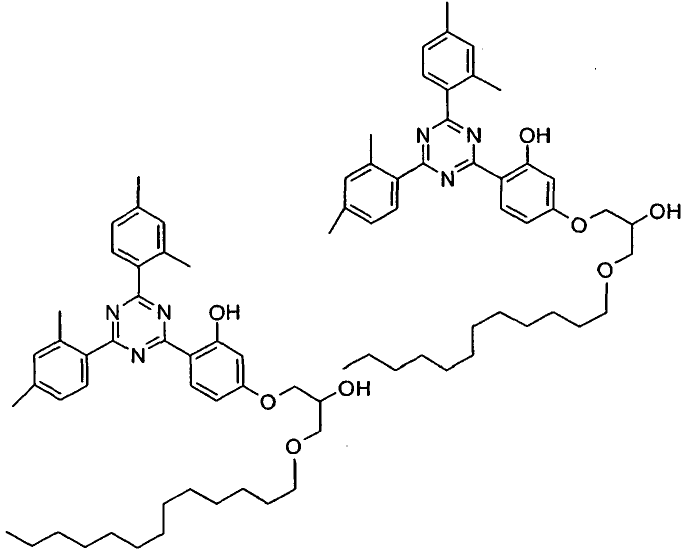 | |
| 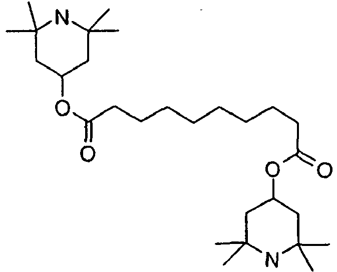 | 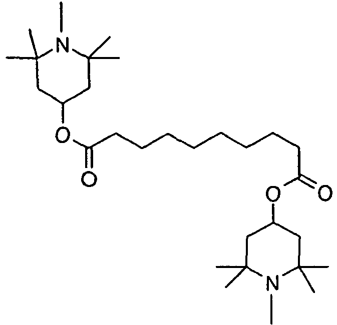 |

Vorzugsweise enthalten die FK-Medien 0 bis 10 Gew.%, insbesondere 1 ppm bis 5 Gew.%, besonders bevorzugt 1 ppm bis 1 Gew.% an Stabilisatoren. Vorzugsweise enthalten die FK-Medien einen oder mehrere Stabilisatoren ausgewählt aus der Gruppe bestehend aus Verbindungen der Tabelle C.

**Tabelle D**

| In der Tabelle D sind Beispielverbindungen zusammengestellt, die in den FK-Medien gemäß der vorliegenden Erfindung vorzugsweise als reaktive mesogene Verbindungen verwendet werden können. |
|---|
| 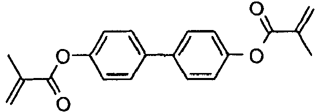 |
| **RM-1** |
| 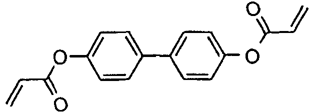 |
| **RM-2** |
| 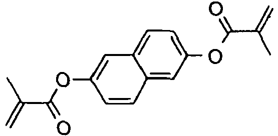 |
| **RM-3** |
| 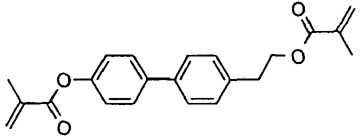 |
| **RM-4** |
| 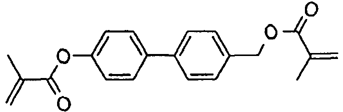 |
| **RM-5** |
| 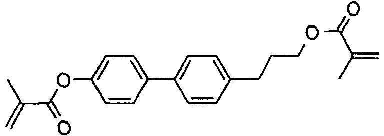 |
| **RM-6** |
| 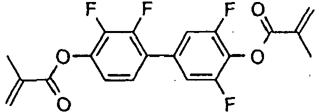 |
| **RM-7** |
| 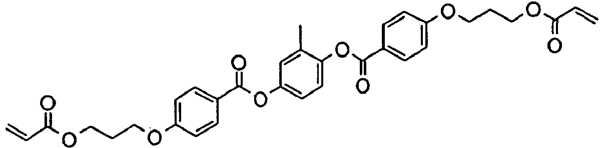 |
| **RM-8** |
| 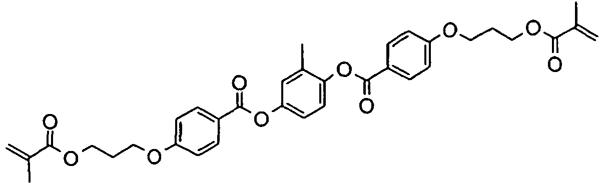 |
| **RM-9** |
| 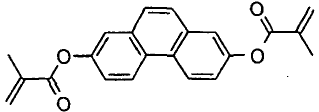 |
| **RM-10** |
| 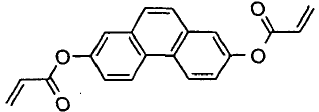 |
| **RM-11** |
| 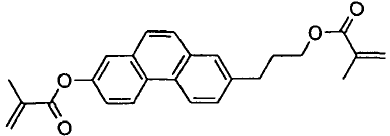 |
| **RM-12** |
| 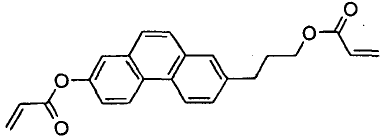 |
| **RM-13** |
| 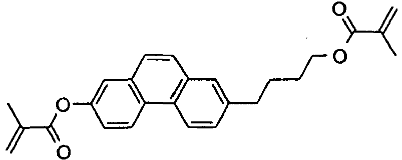 |
| **RM-14** |
| 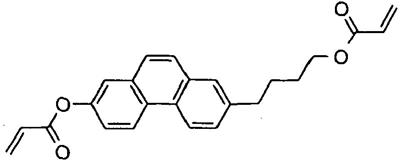 |
| **RM-15** |
| 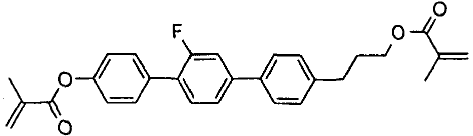 |
| **RM-16** |
| 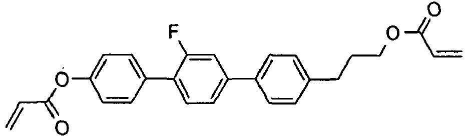 |
| **RM-17** |
| 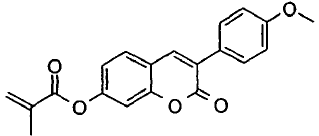 |
| **RM-18** |
| 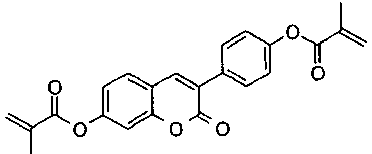 |
| **RM-19** |
| 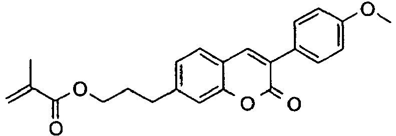 |
| 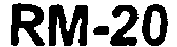 |
| **RM-20** |
| 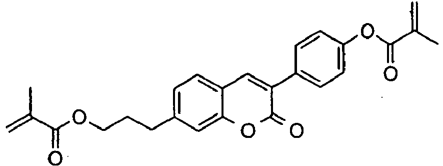 |
| **RM-21** |
| 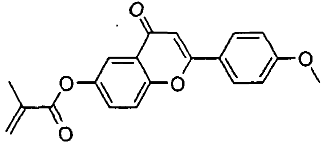 |
| **RM-22** |
| 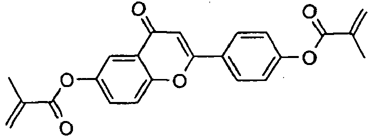 |
| **RM-23** |
| 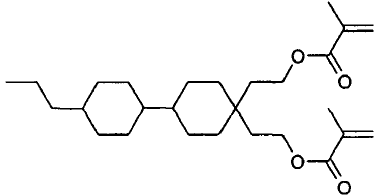 |
| **RM-24** |
| 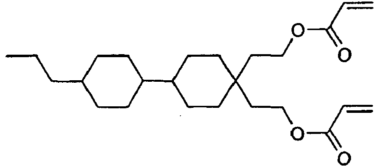 |
| **RM-25** |
| 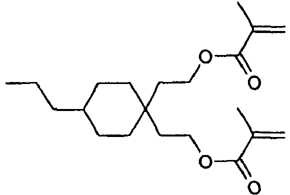 |
| **RM-26** |
| 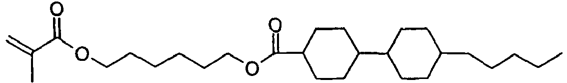 |
| **RM-27** |
| 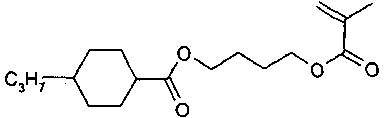 |
| **RM-28** |
| 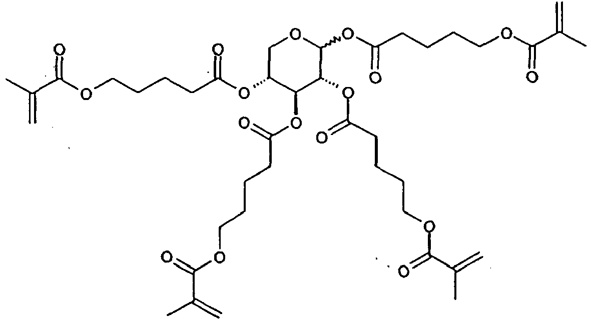 |
| **RM-29** |
| 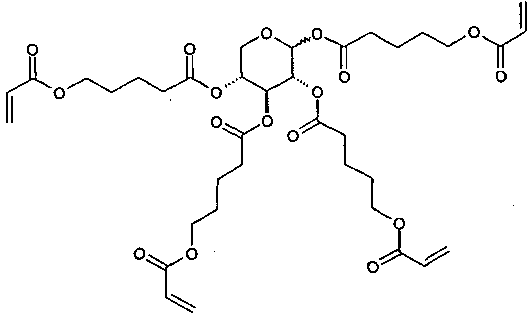 |
| **RM-30** |
| 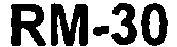 |
| 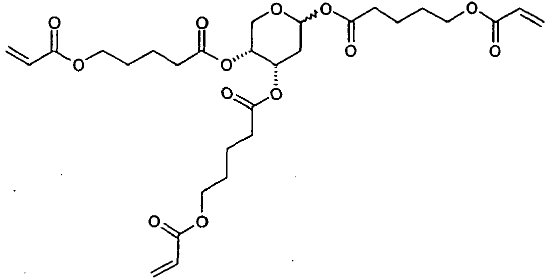 |
| **RM-31** |
| 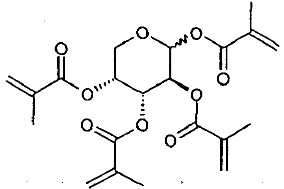 |
| **RM-32** |

In einer bevorzugten Ausführungsform der vorliegenden Erfindung enthalten die mesogenen Medien eine oder mehrere Verbindungen ausgewählt aus der Gruppe der Verbindungen der Tabelle D.

Außerdem werden folgende Abkürzungen und Symbole verwendet:

| | |
|---|---|
| Vₒ | Schwellenspannung, kapazitiv [V] bei 20°C, |
| nₑ | außerordentlicher Brechungsindex bei 20°C und 589 nm, |
| nₒ | ordentlicher Brechungsindex bei 20°C und 589 nm, |
| Δn | optische Anisotropie bei 20°C und 589 nm, |
| ε_{⊥} | dielektrische Permittivität senkrecht zum Direktor bei 20°C und 1 kHz, |
| ε_{∥} | dielektrische Permittivität parallel zum Direktor bei 20°C und 1 kHz, |
| Δε | dielektrische Anisotropie bei 20°C und 1 kHz, |
| Kp., T(N,I) | Klärpunkt [°C], |
| γ₁ | Rotationsviskosität bei 20°C [mPa·s], |
| K₁ | elastische Konstante, "splay"-Deformation bei 20°C [pN], |
| K₂ | elastische Konstante, "twist"-Deformation bei 20°C [pN], |
| K₃ | elastische Konstante, "bend"-Deformation bei 20°C [pN]. |

Soweit nicht explizit anders vermerkt, sind in der vorliegenden Anmeldung alle Konzentrationen in Gewichtsprozent angegeben und beziehen sich auf die entsprechende Gesamtmischung, enthaltend alle festen oder flüssigkristallinen Komponenten, ohne Lösungsmittel.

Soweit nicht explizit anders vermerkt, sind in der vorliegenden Anmeldung alle angegebenen Werte für Temperaturen, wie z. B. der Schmelzpunkt T(C,N), der Übergang von der smektischen (S) zur nematischen (N) Phase T(S,N) und der Klärpunkt T(N,I), in Grad Celsius (°C) angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar.

Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20°C und Δn wird bei 589 nm und Δε bei 1 kHz bestimmt, sofern nicht jeweils explizit anders angegeben.

Der Begriff "Schwellenspannung" bezieht sich für die vorliegende Erfindung auf die kapazitive Schwelle (V₀), auch Freedericksz-Schwelle genannt, sofern nicht explizit anders angegeben. In den Beispielen kann auch, wie allgemein üblich, die optische Schwelle für 10 % relativen Kontrast (V₁₀) angegeben werden.

Die zur Messung der kapazitiven Schwellenspannung verwendete Anzeige besteht aus zwei planparallelen Glasträgerplatten im Abstand von 20 µm, welche auf den Innenseiten jeweils ein Elektrodenschicht sowie eine darüberliegende, ungeriebene Orientierungsschicht aus Polyimid aufweisen, die eine homöotrope Randorientierung der Flüssigkristallmoleküle bewirken.

Die zur Messung der Tiltwinkel verwendete Anzeige bzw. Testzelle besteht aus zwei planparallelen Glasträgerplatten im Abstand von 4 µm, welche auf den Innenseiten jeweils eine Elektrodenschicht sowie eine darüberliegende Orientierungsschicht aus Polyimid aufweisen, wobei die beiden Polyimidschichten antiparallel zueinander gerieben werden und eine homöotrope Randorientierung der Flüssigkristallmoleküle bewirken.

Die polymerisierbaren Verbindungen werden in der Anzeige bzw. Testzelle durch Bestrahlung mit UVA-Licht (üblicherweise 365nm) einer definierten Intensität für eine vorgegebene Zeit polymerisiert, wobei gleichzeitig eine Spannung an die Anzeige angelegt wird (üblicherweise 10V bis 30V Wechselstrom, 1 kHz). In den Beispielen wird, falls nicht anders angegeben, eine Quecksilberdampflampe mit 50 mW/cm² verwendet, die Intensität wird mit einem Standard-UV-Meter (Fabrikat Ushio UNI meter) gemessen, der mit einem Bandpassfilter bei 365nm ausgerüstet ist.

Der Tiltwinkel wird per Drehkristall-Experiment (Autronic-Melchers TBA-105) bestimmt. Ein niedriger Wert (d.h. eine große Abweichung vom 90°-Winkel) entspricht dabei einem großen Tilt.

Der VHR -Wert wird wie folgt gemessen: Zur FK-Host-Mischung werden 0.3% einer polymerisierbaren monomeren Verbindung zugesetzt, und die dadurch entstandene Mischung in TN-VHR-Testzellen gefüllt (90° gerieben, Orientierungsschicht TN-Polyimid, Schichtdicke d ≈ 6 µm). Der HR-Wert wird nach 5min bei 100°C vor und nach 2h UV-Belastung (suntest) bei 1V, 60Hz, 64µs pulse bestimmt (Messgerät: Autronic-Melchers VHRM-105).

Zur Untersuchung der Tieftemperaturstabilität, auch als "LTS" (low temperature stability) bezeichnet, d.h. der Stabilität der FK-Mischung gegen spontane Auskristallisation einzelner Komponenten bei tiefen Temperaturen, werden Fläschchen mit 1g FK/RM-Mischung bei -10°C eingelagert und es wird regelmäßig überprüft, ob die Mischungen auskristallisiert waren.

### Beispiel 1

Verbindung 1 wird wie folgt hergestellt:

### 1.1 3-(4-Bromphenyl)-propan-1-ol

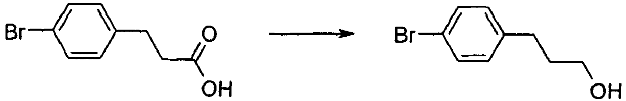

30,0 g (0,128 mol) 3-(4-Bromphenyl)-propionsäure werden in 300 ml THF vorgelegt und unter Eiskühlung tropfenweise mit 257 ml (0,257 mol) einer 1 M Lösung von Borwasserstoff in THF versetzt. Der Ansatz wird über Nacht bei Raumtemp. rühren gelassen, vorsichtig unter Kühlung hydrolysiert und dreimal mit MTB-Ether extrahiert. Die vereinigten org. Phasen werden mit ges. Natriumchloridlsg. gewaschen und über Natriumsulfat getrocknet. Das Lösungsmittel wird i.Vak. entfernt und der Rückstand mit MTB-Ether über Kieselgel filtriert. Man erhält 3-(4-Bromphenyl)-propan-1-ol, das ohne weitere Aufreinigung in der nächsten Stufe eingesetzt wird.

### 1.2 3-[-4,4,5,5-Tetramethyl-[1,3,2]dioxaborolan-2-yl)-phenyl]-propan-1-ol

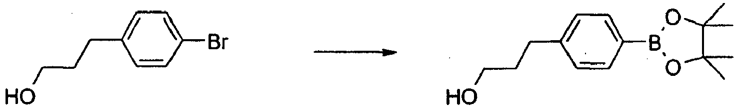

26,5 g (0,123 mol) 3-(4-Bromphenyl)-propan-1-ol und 36,0 g (0,139 mol) Bis(pinacolato)dibor werden in 400 ml Dioxan gelöst und nach Zugabe von 36,5 g (0,372 mol) Kaliumacetat und 2,80 (3,82 mmol) [1,1'-Bis-(diphenylphosphino)-ferrocen]-dichloropalladium(II) über Nacht unter Rückfluß erhitzt. Anschließend wird der Ansatz mit verd. Salzsäure angesäuert und dreimal mit MTB-Ether extrahiert. Die vereinigten org. Phasen werden mit Wasser gewaschen, über Natriumsulfat getrocknet und i. Vak. eingeengt. Der Rückstand wird mit Heptan/MTB-Ether (4:1) über Kieselgel filtriert und Reste Bis(pinacolato)dibor im Feinvakuum bei 160 °C entfernt. Man erhält 3-[4-(4,4,5,5-Tetramethyl-[1,3,2]dioxaborolan-2-yl)-phenyl]-propan-1-ol als gelbes Öl, das für weitere Umsetzungen rein genug ist.

### 1.3 3-(4'-Brom-3'-fluorbiphenyl-4-yl)-propan-1-ol

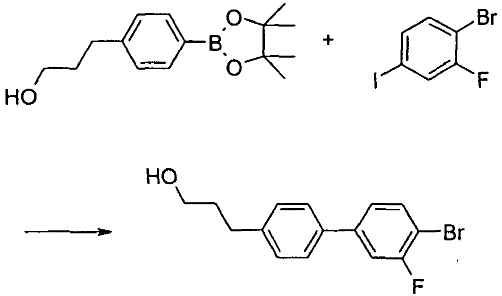

18 g (65 mmol) Natriummetaborat-Octahydrat und 2,8 g (3,9 mmol) Bis(triphenylphosphin)palladium(II)chlorid werden in 200 ml Wasser und 100 ml THF vorgelegt, 1-Brom-2-fluor-4-iodbenzol hinzugegeben und anschließend eine Lösung von 21,5 g (0,082 mol) 3-[4-(4,4,5,5-Tetramethyl-[1,3,2]dioxaborolan-2-yl)-phenyl]-propan-1-ol in 100 ml THF zutropfen gelassen. Der Ansatz wird über Nacht unter Rückfluß erhitzt, in MTB-Ether aufgenommen und dreimal mit Wasser gewaschen. Die org. Phase wird über Natriumsulfat getrocknet und das Lösungsmittel wird i. Vak. entfernt. Chromatographie des Rohproduktes mit Toluol/Essigester (7:1) an Kieselgel liefert 3-(4'-Brom-3'-fluorbiphenyl-4-yl)-propan-1-ol als farbloses Öl.

¹H-NMR (300 MHz, CDCl₃):
δ = 1,93 ppm (m_{c}, 2 H, CH₂), 2,76 (dd, J =7,4 Hz, J = 8,7 Hz, 2 H, Ar-C*H*₂-), 3,71 (dd, J = 6,1 Hz, J = 11,2 Hz, 2 H, -C*H*₂OH), 7,2-7,4 (m, 4 H, Ar-H), 7,47 (AB-m_{c}, 2 H, Ar-H), 7,57 (dd, J = 7,3 Hz, J = 8,3 Hz, 1 H, Ar-H).

### 1.4 2,2'-Difluor-4"-(3-hydroxy-propyl)-[1,1';4',1"]terphenyl-4-ol

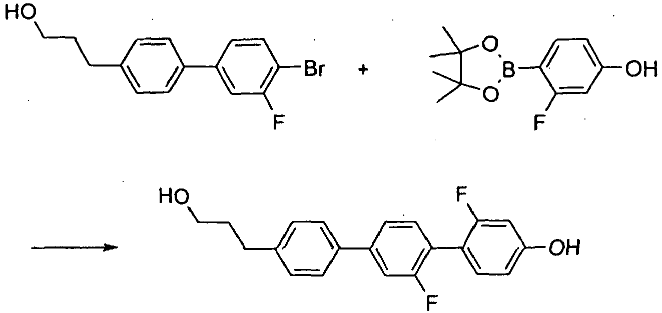

16,5 g (59 mmol) Natriummetaborat-Octahydrat und 1,3 g (1,8 mmol) Bis(triphenylphosphin)palladium(II)chlorid werden in 150 ml Wasser und 50 ml THF vorgelegt, 12,5 g (38,5 mmol) 3-(4'-Brom-3'-fluorbiphenyl-4-yl)-propan-1-ol hinzugegeben und anschließend eine Lösung von 12,5 g (0,053 mol) 3-Fluor-4-(4,4,5,5-tetramethyl-[1,3,2]dioxaborolan-2-yl)-phenol in 50 ml THF zutropfen gelassen. Der Ansatz wird über Nacht unter Rückfluß erhitzt, in MTB-Ether aufgenommen und dreimal mit Wasser gewaschen. Die org. Phase wird über Natriumsulfat getrocknet, das Lösungsmittel i. Vak. entfernt und das Rohprodukt aus Toluol/Essigester (9:1) umkristallisiert. Man erhält 2,2'-Difluor-4"-(3-hydroxy-propyl)-[1,1';4',1 "]terphenyl-4-ol als farblose Kristalle.

### 1.5 2-Methyl-acrylsäure-2,2'-difluor-4"-[3-(2-methyl-acryloyloxy)-propyl]-[1,1';4',1"]terphenyl-4-yl ester (1)

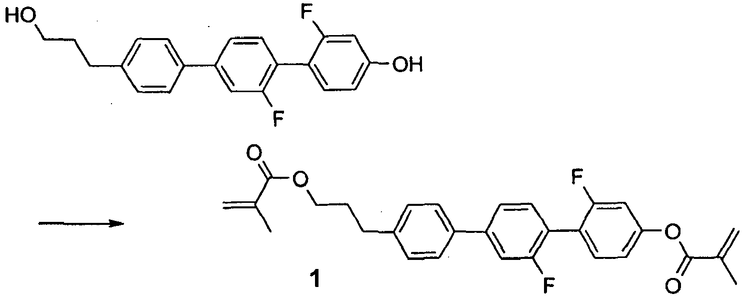

3,0 g (8,81 mmol) 2,2'-Difluor-4"-(3-hydroxy-propyl)-[1,1';4',1"]terphenyl-4-ol werden in 60 ml Dichlormethan vorgelegt, mit 4 ml Trethylamin versetzt und unter Eiskühlung wird tropfenweise eine Lösung von 2,2 ml (22,5 mmol) Acrylsäurechlorid in 5 ml Dichlormethan hinzugegeben. Die Kühlung wird entfernt und der Ansatz 3 h bei Raumtemp. rühren gelassen. Anschließend wird die Lösung über Kieselgel filtriert, eingeengt und der Rückstand aus Ethanol umkristallisiert. Man erhält 2-Methyl-acrylsäure-2,2'-difluor-4"-[3-(2-methyl-acryloyloxy)-propyl]-[1,1';4',1"]terphenyl-4-yl-ester (1) als farblose Kristalle vom Schmp. 67°C.

### Beispiel 2

Verbindung 2 wird wie folgt hergestellt:

### 2.1 2'-Fluor-4"-(3-hydroxy-propyl)-[1,1';4',1"]terphenyl-4-ol

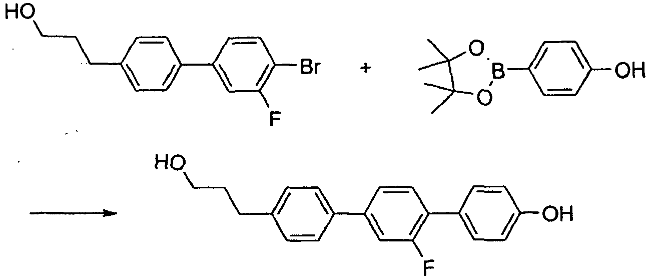

In Analogie zu der unter 1.4 beschriebenen Synthese erhält man aus 3-(4'-Brom-3'-fluorbiphenyl-4-yl)-propan-1-ol und 4-(4,4,5,5-Tetramethyl-[1,3,2]dioxaborolan-2-yl)-phenol das 2'-Fluor-4"-(3-hydroxy-propyl)-[1,1';4',1"]terphenyl-4-ol als farblosen Feststoff.

¹H-NMR (400 MHz, DMSO-d₆)
δ = 1,80 ppm (m_{c}, 2 H, CH₂), 2,71 (7, J =7,8 Hz, 2 H, Ar-CH₂-), 3,71 (7, J = 6,5 Hz, 2 H, -C*H₂*OH), 4,53 (s, br. 1 H, OH), 6,93 (AB-m_{c}, 2 H, Ar-H), 7,35 (d, J = 8,25, 2 H, Ar-H), 7,47 (AB-dd, J = 1,5 Hz, J = 8,6 Hz, 2 H, Ar-H), 7,54 - 7,64 (m, 3 H, Ar-H), 7,70 (d, J = 8,3 Hz, 2 H, Ar-H), 9,70 (s, 1 H, Ar-OH).

### 2.2 2-Methyl-acrylsäure-2-fluor-4"-[3-(2-methyl-acryloyloxy)-propyl]-[1,1';4',1"]terphenyl-4-ylester (2)

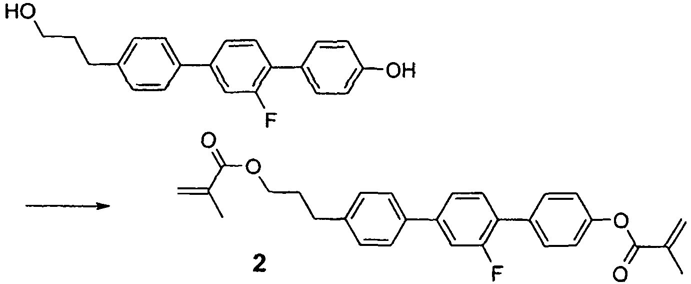

In Analogie zu Beispiel 1 erhält man aus 2'-Fluor-4"-(3-hydroxy-propyl)-[1,1';4',1"]terphenyl-4-ol den 2-Methyl-acrylsäure-2'-fluor-4"-[3-(2-methyl-acryloyloxy)-propyl]-[1,1';4',1"]terphenyl-4-ylester (2) als farblose Kristalle vom Schmp. 76 °C.

### Beispiel 3-7

In Analogie zu den in Beispiel 1 und 2 beschriebenen Verfahren werden die folgenden Verbindungen hergestellt: 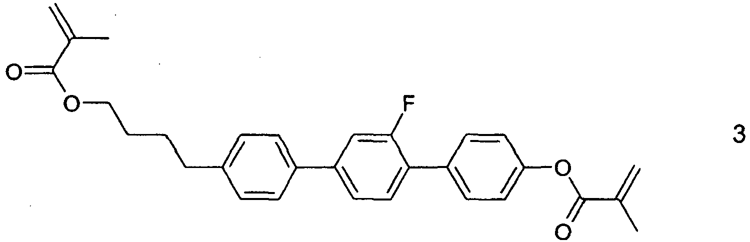 K 80 SmB 83 SmA 139 I 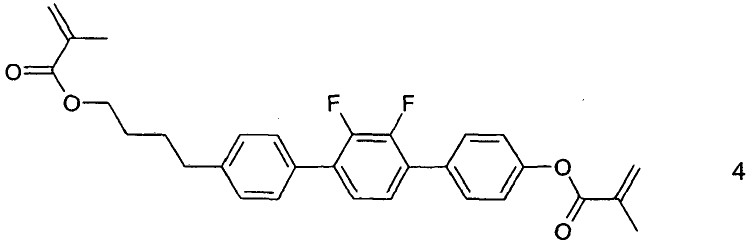 K 102 N 115.3 I 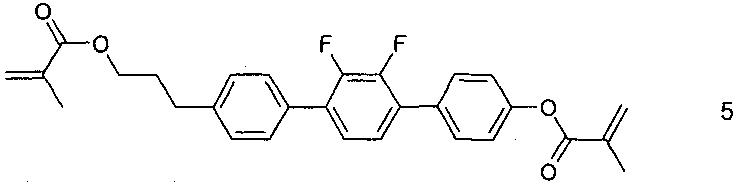 K 91 SmA (75) N 100.3 I 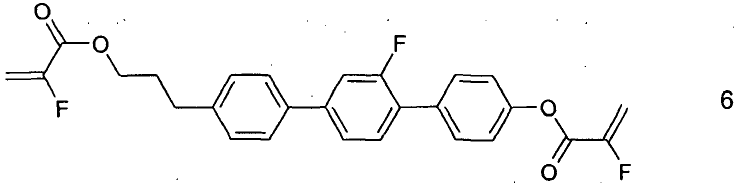 Tg K 95 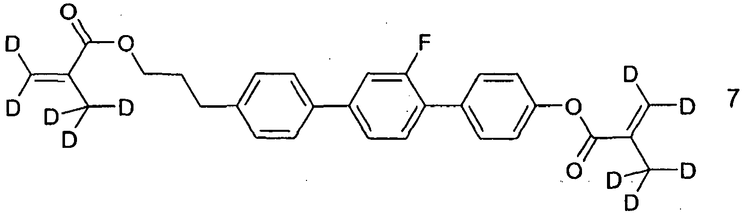 (worin D Deuterium bedeutet)

### Mischungsbeispiel A

Die nematische FK-Mischung N1 wird wie folgt formuliert

| | | | |
|---|---|---|---|
| CCH-501 | 9,00 % | Kp. | + 70,0 |
| CCH-35 | 14,00 % | Δn | 0,0825 |
| PCH-53 | 8,00 % | Δε | - 3,5 |
| CY-3-O4 | 14,00 % | ε_{\|\|} | 3,5 |
| CY-5-O4 | 13,00 % | K₃/K₁ | 1,00 |
| CCY-3-O2 | 8,00 % | γ₁ | 141 |
| CCY-5-O2 | 8,00 % | V₀ | 2,06 |
| CCY-2-1 | 9,00 % | | |
| CCY-3-1 | 9,00 % | | |
| CPY-2-O2 | 8,00 % | | |

Zur FK-Mischung N1 werden jeweils 0.3% eines polymerisierbaren Monomers der Formel 1, 2, 3, 4, 5 oder 6 aus Beispiel 1-6 zugesetzt. Die so hergestellten Mischungen werden in VA-e/o-Testzellen gefüllt (antiparallel gerieben, Orientierungsschicht VA-Polyimid, Schichtdicke d ≈ 4µm). Unter Anlegen einer Spannung von 24 V (Wechselstrom) werden die Zellen in der angegebenen Zeit mit UV-Licht der Intensität 50 mW/cm² bestrahlt, dadurch erfolgt Polymerisation der monomeren Verbindung. Vor und nach der UV-Bestrahlung wird per Drehkristall-Experiment (Autronic-Melchers TBA-105) der Tiltwinkel bestimmt.

Zur Bestimmung der Polymerisationsgeschwindigkeit wird der Restgehalt an unpolymerisiertem Monomer (in Gew.%) in den Testzellen nach verschiedenen Belichtungszeiten mit der HPLC-Methode gemessen. Dazu wird jede Mischung jeweils unter den angegebenen Bedingungen in der Testzelle polymerisiert. Danach wird die Mischung mit MEK (Methylethylketon) aus der Testzelle gespült und vermessen.

Für die FK-Medien enthaltend FK-Mischung N1 und das Monomer 1 bzw. 2 werden ausserdem die FK-Phaseneigenschaften durch DSC-Messung, sowie die LTS bei -10°C und die VHR-Werte vor und nach 2h suntest Belastung wie oben angegeben bestimmt.

Zu Vergleichszwecken werden die oben beschriebenen Versuche mit den aus dem Stand der Technik (z.B. WO 2009/030329 A1) bekannten polymerisierbaren Verbindungen V1 und V2 durchgeführt.

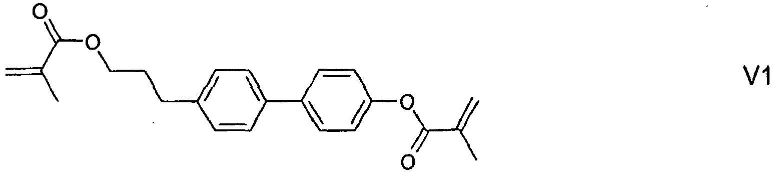

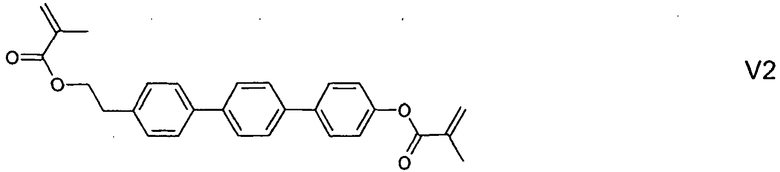

Die vergleichenden Tiltwinkelergebnisse für die Monomere 1-6 sowie V1 und V2 sind in Tabelle 1 aufgeführt.

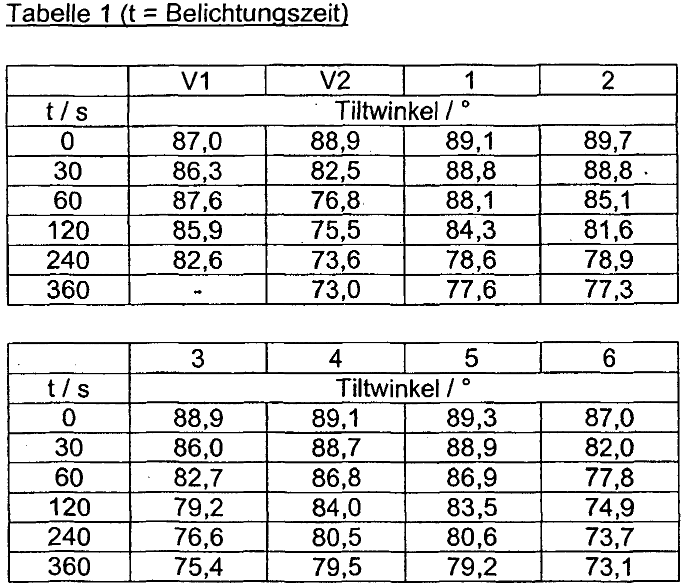

Wie aus Tabelle 1 ersichtlich ist, wird in PSA-Anzeigen enthaltend ein erfindungsgemäßes FK-Medium mit der FK-Mischung N1 und einem der Monomere 1-6, im Vergleich zum FK-Medium mit N1 und Monomer V1, eine deutlich schnellere Generierung des pretilt Winkels bei gleichen Bedingungen (50mW/cm², UVA; 24V) erzielt.

Die pretilt-Winkeländerung in einer PSA-Anzeige mit Monomer V1 in FK-Mischung N1 beträgt beispielsweise 1,1° nach Belichten mit 50mW/cm², 2min und 24V Spannung. Mit den gleichen Bedingungen wird bei einer PSA-Anzeige enthaltend das erfindungsgemäße Monomer 1 in N1 eine pretilt-Winkeländerung von 4,8° erreicht, also das Vierfache, und mit dem erfindungsgemäßen Monomer 2 in N1 eine pretilt-Winkeländerung von 8,1° erreicht, also das Siebenfache. Bei beiden Beispielen ist eine Sättigung des Tiltwinkels bereits nach 240s (4min) zu beobachten. Bei Monomer V1 ist dies nicht der Fall.

Die Monomerkonzentrationen in der Anzeige nach verschiedenen Belichtungszeiten sind in Tabelle 2 zusammengefasst.

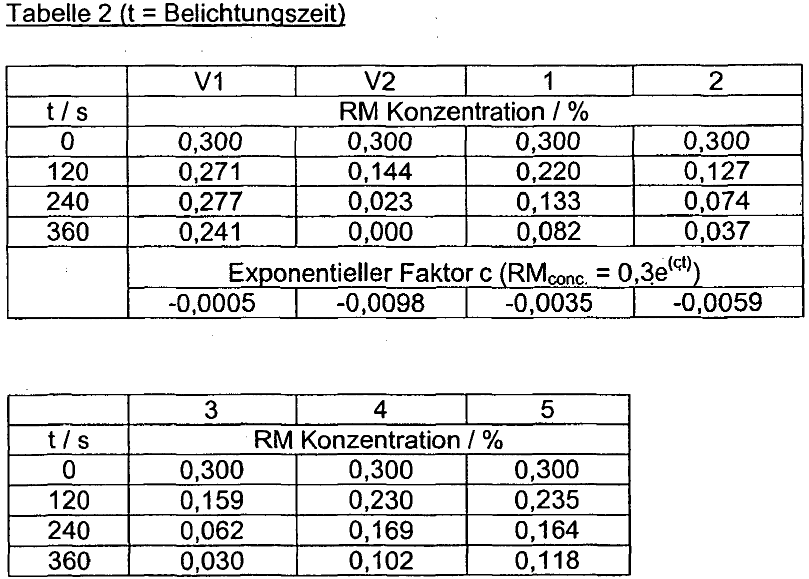

Sehr auffällig ist der Unterschied in den Restmonomerkonzentrationen. Wie aus Tabelle 2 ersichtlich ist, sind in PSA-Anzeigen mit Monomer V1 in N1 nach 360s (6min) Belichtung noch 0,241% von ursprünglich 0,3% Monomer erhalten. Nach derselben Belichtungszeit enthalten dagegen PSA-Anzeigen z.B. mit dem erfindungsgemäßen Monomer 1 in N1 nur noch 0,082%, und z.B. mit dem erfindungsgemäßen Monomer 2 in N1 nur noch 0,037% Restmonomer von ursprünglich 0,3%.

An eine Kurve der Messdaten des Monomergehalts, aufgetragen gegen die Belichtungszeit, kann eine e-Funktion angefittet werden. Aus dieser lässt sich ein exponentieller Faktor c ermitteln, der ein Maß für die Polymerisationsgeschwindigkeit ist (siehe Tabelle 2). Daraus folgt, dass Monomer 1 in N1 siebenmal schneller und Monomer 2 in N1 zwölfmal schneller polymerisiert als Monomer V1 in N1.

Das FK-Phasenverhalten sowie die LTS- und VHR-Werte der FK-Medien sind in Tabelle 3 zusammengefasst.

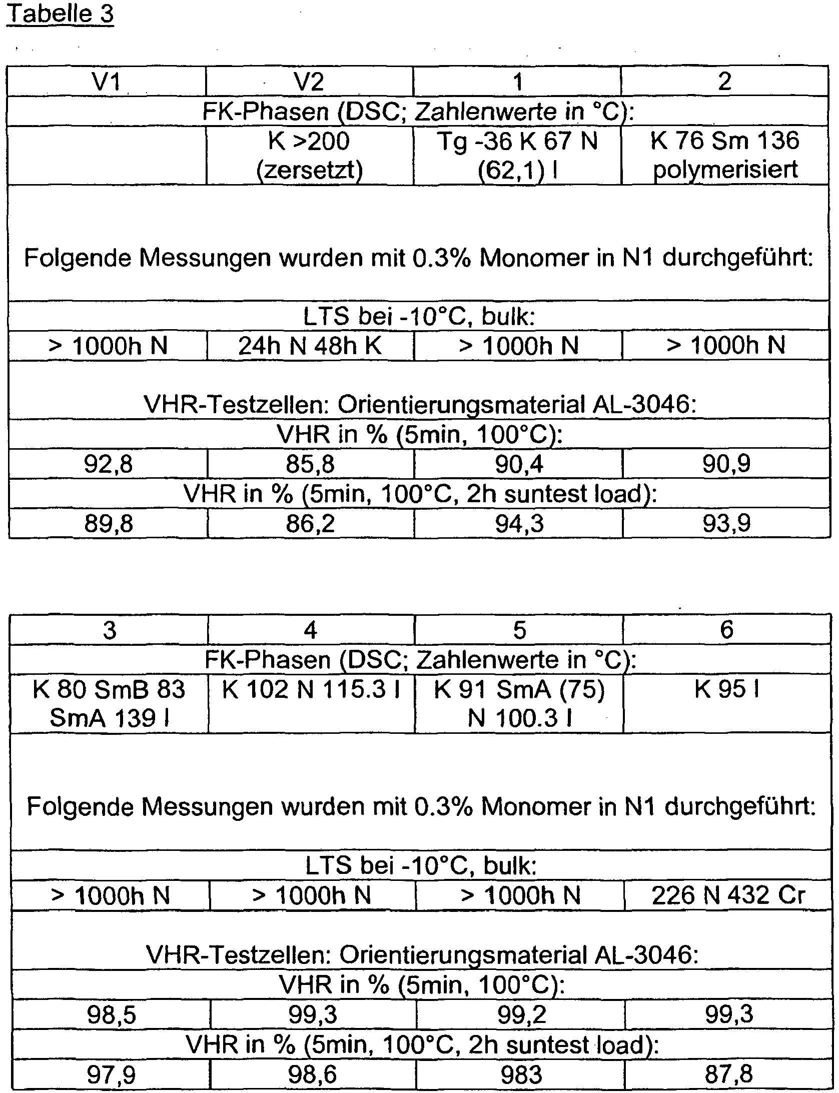

Wie aus Tabelle 3 ersichtlich ist, sind auch die LTS- und VHR- Werte der erfindungsgemäßen Monomere 1-6 in der FK-Mischung N1 sehr gut und vergleichbar oder sogar besser als die LTS- und VHR-Werte von Monomer V1 in N1.

Monomer V2 zeigt in FK-Mischung N1 zwar eine noch schnellere pretilt-Generierung (siehe Tabelle 1) und eine noch schnellere Polymerisationsgeschwindigkeit (siehe Tabelle 2) als die erfindungsgemäßen Monomere 1-6, ist aber sehr schwer löslich und führt zu schlechteren LTS- und VHR-Werten vor und nach suntest Belastung der FK/Monomer-Mischung, wie aus Tabelle 3 ersichtlich ist.

## Patentansprüche

1. Verwendung von polymerisierbaren Verbindungen, enthaltend eine Terphenylgruppe, die mindestens einfach durch ein Halogenatom substituiert ist, und eine oder mehrere polymerisierbare Gruppen, in FK-Medien und FK-Anzeigen des PS- oder PSA-Typs.

2. Verwendung nach Anpruch 1, **dadurch gekennzeichnet, dass** die polymerisierbaren Verbindungen zwei oder mehr als zwei polymerisierbare Gruppen enthalten, wobei mindestens eine dieser polymerisierbaren Gruppen über eine Abstandsgruppe mit der Terphenylgruppe verbunden ist und mindestens eine dieser polymerisierbaren Gruppen direkt mit der Terphenylgruppe verbunden ist.

3. Verwendung nach Anpruch 1 oder 2, **dadurch gekennzeichnet, dass** die polymerisierbaren Verbindungen aus Formel I ausgewählt sind 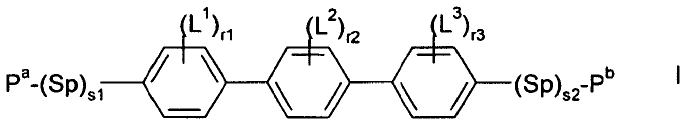 worin die einzelnen Reste folgende Bedeutung besitzen
P^{a}, P^{b} jeweils unabhängig voneinander eine polymerisierbare Gruppe,
Sp bei jedem Auftreten gleich oder verschieden eine Abstandsgruppe,
L¹ L², L³ jeweils unabhängig voneinander F oder Cl einer oder mehrere der Reste L¹,L² und L³ auch vollständig oder teilweise fluoriertes Alkyl oder Alkoxy mit 1, 2 oder 3 C-Atomen, P^{a} oder P^{a}-Sp-, wobei mindestens einer der Reste L¹,L² und L³ F oder Cl, vorzugsweise F, bedeutet,
r1, r2, r3 jeweils unabhängig voneinander 0, 1, 2, 3 oder 4, wobei r1+r2+r3 ≥ 1,
s1 und s2 jeweils unabhängig voneinander 0 oder 1.

4. Verwendung nach einem oder mehreren der Ansprüche 1 bis 3, **dadurch gekennzeichnet, dass** die Verbindungen der Formel I ausgewählt sind aus der Gruppe bestehend aus folgenden Unterformeln: 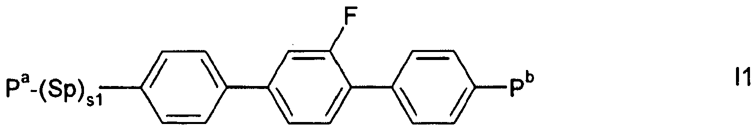 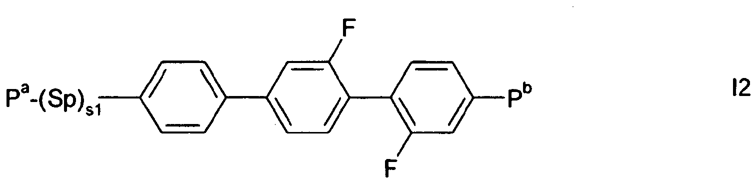 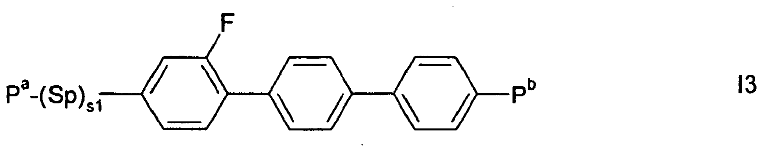 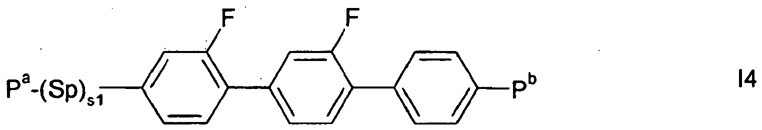 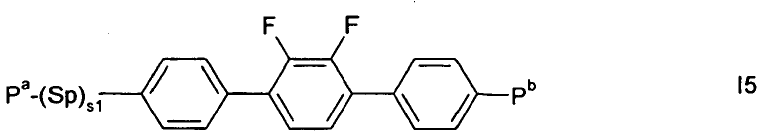 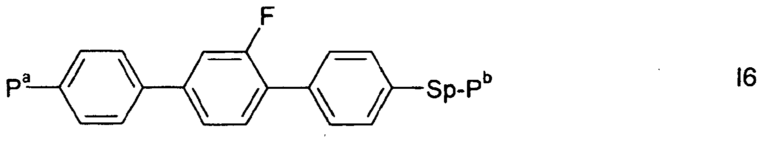 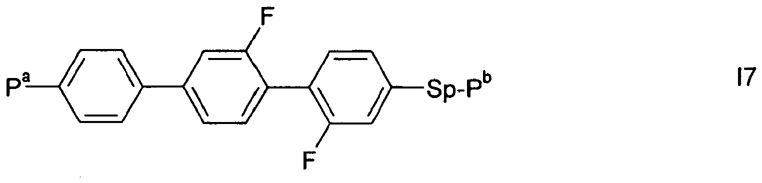 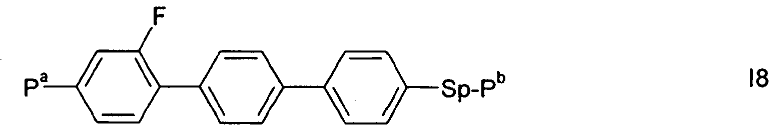 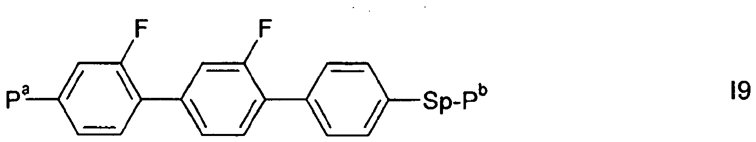 worin P^{a}, P^{b}, Sp und s1 die in Anspruch 3 angegebene Bedeutung besitzen.

5. Verwendung von Verbindungen nach einem oder mehreren der Ansprüche 1 bis 4 in FK-Anzeigen des PS- oder PSA-Typs, enthaltend eine FK-Zelle mit zwei Substraten und zwei Elektroden, wobei mindestens ein Substrat lichtdurchlässig ist und mindestens ein Substrat eine oder zwei Elektroden aufweist, sowie einer zwischen den Substraten befindlichen Schicht eines FK-Mediums enthaltend eine polymerisierte Komponente und eine niedermolekulare Komponente, wobei die polymerisierte Komponente erhältlich ist durch Polymerisation einer oder mehrerer polymerisierbarer Verbindungen zwischen den Substraten der FK-Zelle im FK-Medium, vorzugsweise unter Anlegen einer elektrischen Spannung an die Elektroden, wobei mindestens eine der polymerisierbaren Verbindungen eine polymerisierbare Verbindung nach einem oder mehreren der Ansprüche 1 bis 4 ist.

6. Verwendung nach einem oder mehreren der Ansprüche 1 bis 5, **dadurch gekennzeichnet, dass** das FK-Medium eine oder mehrere Verbindungen der Formel CY und/oder PY enthält: 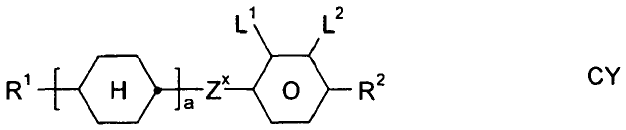 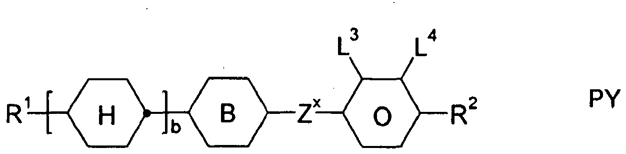 worin die einzelnen Reste folgende Bedeutung besitzen
a 1 oder 2,
b 0 oder 1, 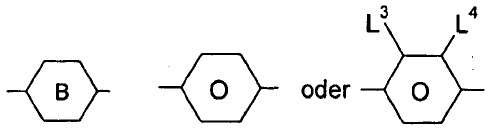
R¹ und R² jeweils unabhängig voneinander Alkyl mit 1 bis 12 C-Atomen, wobei auch eine oder zwei nicht benachbarte CH₂-Gruppen durch -O-, -CH=CH-, -CO-, -O-CO- oder -CO-O- so ersetzt sein können, dass O-Atome nicht direkt miteinander verknüpft sind,
Z^{x} -CH=CH-, -CH₂O-, -OCH₂-, -CF₂O-, -OCF₂-, -O-, -CH₂-, -CH₂CH₂-, oder eine Einfachbindung, vorzugsweise eine Einfachbindung,
L¹⁻⁴ jeweils unabhängig voneinander F, Cl, OCF₃, CF₃, CH₃, CH₂F, CHF₂.

7. Verwendung nach einem oder mehreren der Ansprüche 1 bis 6, **dadurch gekennzeichnet, dass** das FK-Medium eine oder mehrere Verbindungen der folgenden Formel enthält: 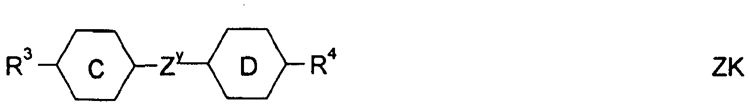 worin die einzelnen Reste folgende Bedeutung besitzen 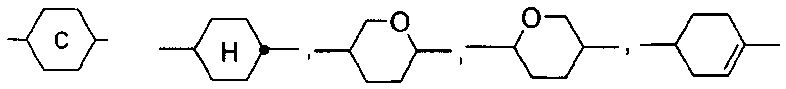 oder 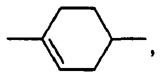 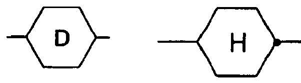 oder 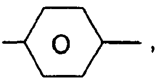
R³ und R⁴ jeweils unabhängig voneinander Alkyl mit 1 bis 12 C-Atomen, worin auch eine oder zwei nicht benachbarte CH₂-Gruppen durch -O-, -CH=CH-, -CO-, -O-CO- oder -CO-O- so ersetzt sein können, dass O-Atome nicht direkt miteinander verknüpft sind,
Z^{y} -CH₂CH₂-, -CH=CH-, -CF₂O-, -OCF₂-, -CH₂O-, -OCH₂-, -COO-, -OCO-, -C₂F₄-, -CF=CF- oder eine Einfachbindung.

8. FK-Anzeige nach einem oder mehreren der Ansprüche 1 bis 7.

9. FK-Anzeige nach Anspruch 8, **dadurch gekennzeichnet, dass** sie eine PSA-VA-, PSA-OCB-, PSA-IPS-, PSA-FFS-, PSA-Posi-VA- oder PSA-TN-Anzeige ist.

10. Verbindungen der Formel I 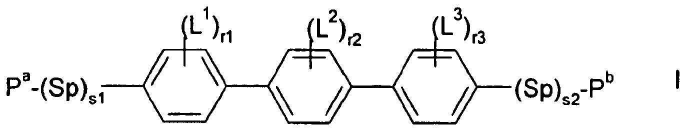 worin P^{a}, P^{b}, Sp, L¹, L², L³, r1, r2 und r3die in Anspruch 3 angegebene Bedeutung besitzen, und s1 1 und s2 0 bedeutet, oder s1 0 und s2 1 bedeutet.

11. Verbindungen nach Anspruch 10, ausgewählt aus der Gruppe bestehend aus folgenden Unterformeln: 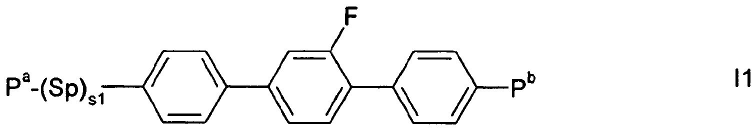 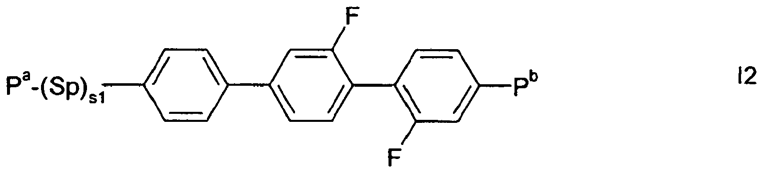 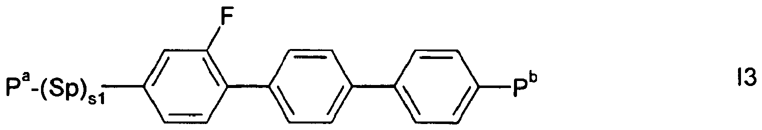 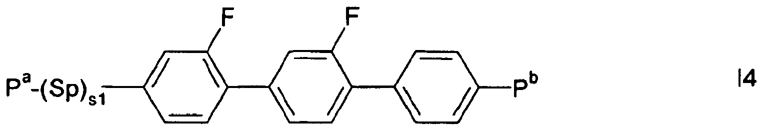 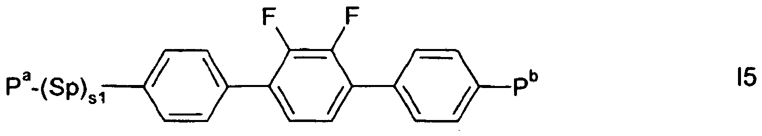 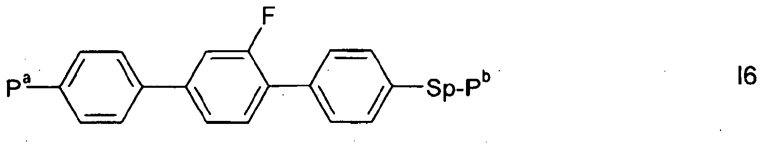 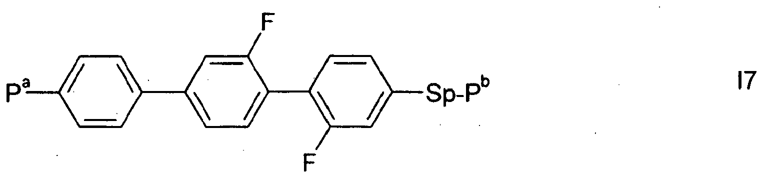 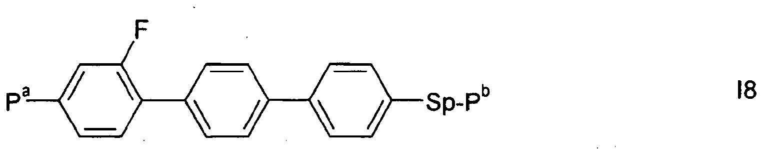 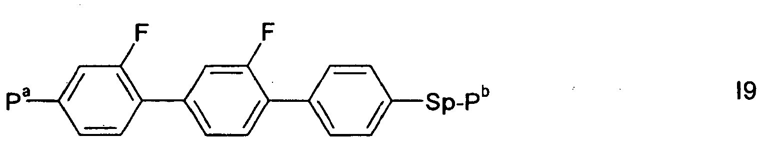 worin P^{a}, P^{b}, Sp und s1 die in Anspruch 13 angegebene Bedeutung besitzen, und s1 1 bedeutet.

12. Verbindungen nach Anspruch 10 oder 11, dadurch gekennnzeichnet, dass P^{a} und P^{b} jeweils unabhängig voneinander eine Vinyloxy-, Acrylat-, Methacrylat-, Fluoracrylat-, Chloracrylat-, Oxetan- oder Epoxygruppe bedeuten.

13. Verbindungen nach einem oder mehreren der Ansprüche 10 bis 12, dadurch gekennnzeichnet, dass Sp bei jedem Auftreten gleich oder verschieden ausgewählt ist aus der Formel Sp"-X", so dass der Rest " P^{a/b}-Sp-" " der Formel " P^{a/b}-Sp"-X"- " entspricht, wobei
Sp" Alkylen mit 1 bis 20, vorzugsweise 1 bis 12 C-Atomen bedeutet, welches optional durch F, Cl Br, I oder CN ein- oder mehrfach substituiert ist, und worin auch eine oder mehrere nicht benachbarte CH₂-Gruppen jeweils unabhängig voneinander so durch -O-, -S-, -NH-, -N(R⁰)-, -Si(R⁰⁰R⁰⁰⁰)-, -CO-, -CO-O-, -O-CO-, -O-CO-O-, -S-CO-, -CO-S-, -N(R⁰⁰)-CO-O-, -O-CO-N(R⁰⁰)-, -N(R⁰⁰)-CO-N(R⁰⁰)-, -CH=CH- oder -C=C- ersetzt sein können, dass O- und/oder S-Atome nicht direkt miteinander verknüpft sind,
X" -O-, -S-, -CO-, -CO-O-, -O-CO-, -O-CO-O-, -CO-N(R⁰⁰)-, - N(R⁰⁰)-CO-, -N(R⁰⁰)-CO-N(R⁰⁰)-, -OCH₂-, -CH₂O-, -SCH₂-, - CH₂S-, -CF₂O-, -OCF₂-, -CF₂S-, -SCF₂-, -CF₂CH₂-, -CH₂CF₂-, - CF₂CF₂-, -CH=N-, -N=CH-, -N=N-, -CH=CR⁰-, -CY²=CY³-, - C≡C-, -CH=CH-CO-O-, -O-CO-CH=CH- oder eine Einfachbindung bedeutet,
R⁰⁰ und R⁰⁰⁰ jeweils unabhängig voneinander H oder Alkyl mit 1 bis 12 C-Atomen bedeuten, und
Y² und Y³ jeweils unabhängig voneinander H, F, Cl oder CN bedeuten.

14. FK-Medium enthaltend eine oder mehrere polymerisierbare Verbindungen der Formel I nach einem oder mehreren der Ansprüche 10 bis 13.

15. FK-Medium nach Anspruch 14, enthaltend
- eine polymerisierbare Komponente A) enthaltend eine oder mehrere polymerisierbare Verbindungen, sowie
- eine flüssigkristalline Komponente B) enthaltend eine oder mehrere niedermolekulare Verbindungen,
**dadurch gekennzeichnet, dass** Komponente A) eine oder mehrere polymerisierbare Verbindungen wie in Anspruch 14 definiert enthält.

16. FK-Medium nach Anspruch 14 oder 15, worin Komponente B) eine oder mehrere Verbindungen ausgewählt aus Formel CY, PY und ZK nach Anspruch 7 oder 8 enthält.

17. Verbindungen der Formel II 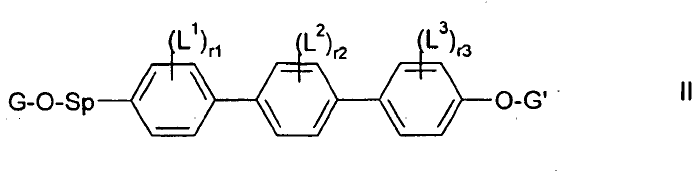 worin Sp, L¹,L², L³, r1, r2 und r3 die in Anspruch 3 angegebene Bedeutung besitzen, und G und G' jeweils unabhängig voneinander ein H-Atom oder eine Schutzgruppe bedeuten.

18. Verfahren zur Herstellung einer Verbindung nach einem oder mehreren der Ansprüche 13 bis 16, indem man eine Verbindung nach Anspruch 17 mit entsprechenden Säuren, Säurederivaten, oder halogenierten Verbindungen enthaltend eine Gruppe P^{a} oder P^{b}, in Gegenwart eines wasserentziehenden Reagens verestert oder verethert.

19. Verfahren zu Herstellung einer FK-Anzeige des PS- oder PSA-Typs, indem man ein FK-Medium nach einem oder mehreren der Ansprüche 10 bis 12 in eine FK-Zelle mit zwei Substraten und zwei Elektroden, wobei mindestens ein Substrat lichtdurchlässig ist und mindestens ein Substrat eine oder zwei Elektroden aufweist, füllt, und die polymerisierbaren Verbindungen, vorzugsweise unter Anlegen einer elektrischen Spannung an die Elektroden, polymerisiert.

## Claims

1. Use of polymerisable compounds containing a terphenyl group, which is at least monosubstituted by a halogen atom, and one or more polymerisable groups in LC media and LC displays of the PS or PSA type.

2. Use according to Claim 1, **characterised in that** the polymerisable compounds contain two or more than two polymerisable groups, where at least one of these polymerisable groups is connected to the terphenyl group via a spacer group and at least one of these polymerisable groups is connected directly to the terphenyl group.

3. Use according to Claim 1 or 2, **characterised in that** the polymerisable compounds are selected from formula I 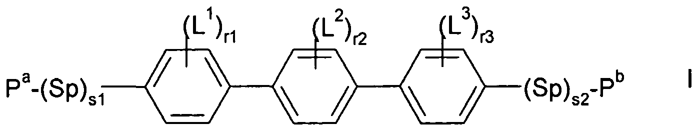 in which the individual radicals have the following meanings:
P^{a}, P^{b} each, independently of one another, denote a polymerisable group,
Sp on each occurrence, identically or differently, denotes a spacer group,
L¹,L², L³ each, independently of one another, denote F or Cl one or more of the radicals L¹,L² and L³ also denote fully or partially fluorinated alkyl or alkoxy having 1, 2 or 3 C atoms, P^{a} or P^{a}-Sp-, where at least one of the radicals L¹, L² and L³ denotes F or Cl, preferably F,
r1, r2, r3 each, independently of one another, denote 0, 1, 2, 3 or 4, where r1 +r2+r3 ≥ 1,
s1 and s2 each, independently of one another, denote 0 or 1.

4. Use according to one or more of Claims 1 to 3, **characterised in that** the compounds of the formula I are selected from the group consisting of the following sub-formulae: 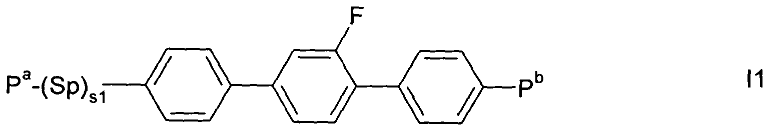 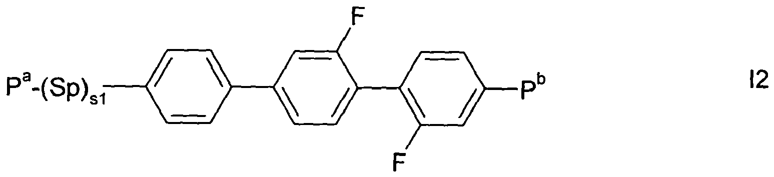 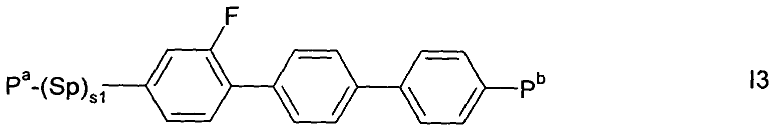 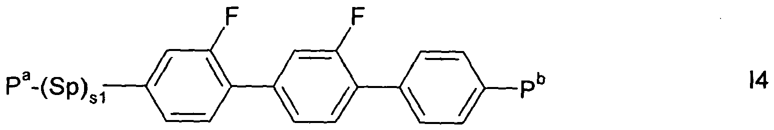 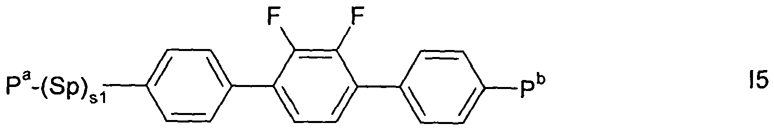 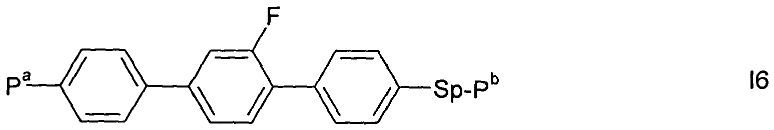 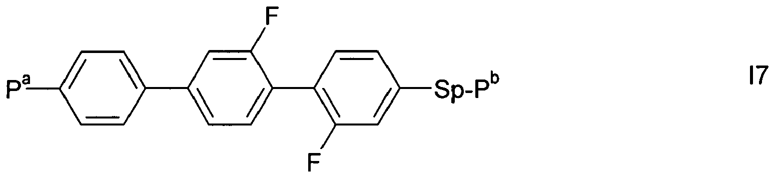 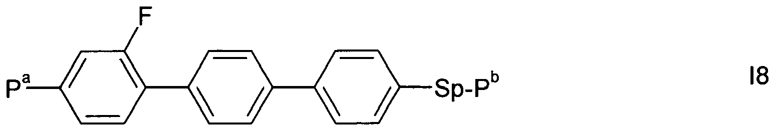 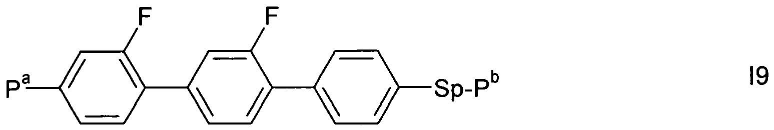 in which P^{a}, P^{b}, Sp and s1 have the meaning indicated in Claim 3.

5. Use of compounds according to one or more of Claims 1 to 4 in LC displays of the PS or PSA type containing an LC cell having two substrates and two electrodes, where at least one substrate is transparent to light and at least one substrate has one or two electrodes, and a layer, located between the substrates, of an LC medium comprising a polymerised component and a low-molecular-weight component, where the polymerised component is obtainable by polymerisation of one or more polymerisable compounds between the substrates of the LC cell in the LC medium, preferably with application of an electrical voltage to the electrodes, where at least one of the polymerisable compounds is a polymerisable compound according to one or more of Claims 1 to 4.

6. Use according to one or more of Claims 1 to 5, **characterised in that** the LC medium comprises one or more compounds of the formulae CY and/or PY: 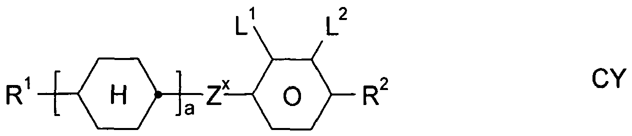 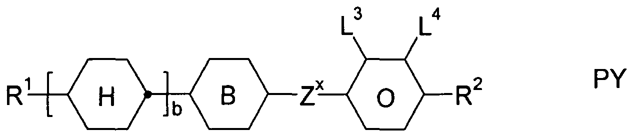 in which the individual radicals have the following meanings:
a denotes 1 or 2,
b denotes 0 or 1, 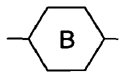 denotes 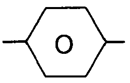 or 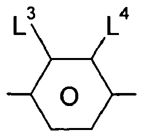
R¹ and R² each, independently of one another, denote alkyl having 1 to 12 C atoms, where, in addition, one or two non-adjacent CH₂ groups may be replaced by -O-, -CH=CH-, -CO-, -O-CO- or -CO-O- in such a way that O atoms are not linked directly to one another,
Z^{x} denotes -CH=CH-, -CH₂O-, -OCH₂-, -CF₂O-, -OCF₂-, -O-, -CH₂-, -CH₂CH₂- or a single bond, preferably a single bond,
L¹⁻⁴ each, independently of one another, denote F, Cl, OCF₃, CF₃, CH₃, CH₂F, CHF₂.

7. Use according to one or more of Claims 1 to 6, **characterised in that** the LC medium comprises one or more compounds of the following formula: 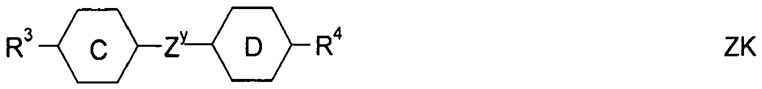 in which the individual radicals have the following meanings: 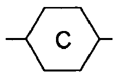 denotes 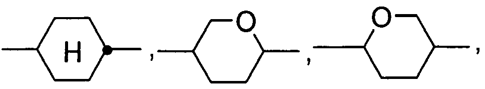 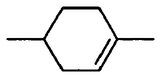 or 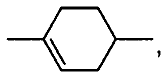 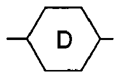 denotes 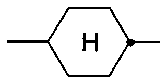 or 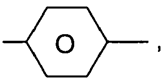
R³ and R⁴ each, independently of one another, denote alkyl having 1 to 12 C atoms, in which, in addition, one or two non-adjacent CH₂ groups may be replaced by -O-, -CH=CH-, -CO-, -O-CO- or -CO-O- in such a way that O atoms are not linked directly to one another,
Z^{y} denotes -CH₂CH₂-, -CH=CH-, -CF₂O-, -OCF₂-, -CH₂O-, -OCH₂-, -COO-, -OCO-, -C₂F₄-, -CF=CF- or a single bond.

8. LC display according to one or more of Claims 1 to 7.

9. LC display according to Claim 8, **characterised in that** it is a PSA-VA, PSA-OCB, PSA-IPS, PSA-FFS, PSA-positive-VA or PSA-TN display.

10. Compounds of the formula I 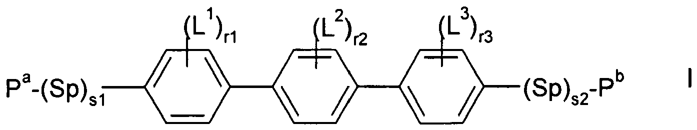 in which P^{a}, P^{b}, Sp, L¹,L², L³, r1, r2 and r3 have the meaning indicated in Claim 3, and s1 denotes 1 and s2 denotes 0, or s1 denotes 0 and s2 denotes 1.

11. Compounds according to Claim 10, selected from the group consisting of the following sub-formulae: 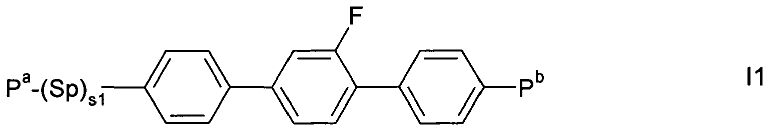 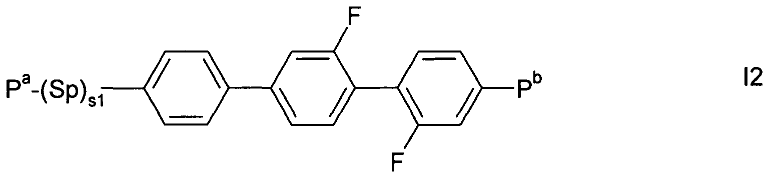 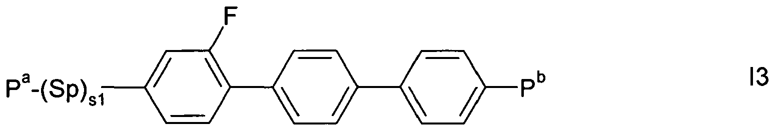 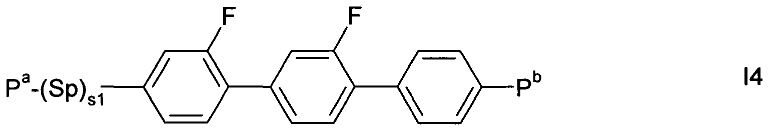 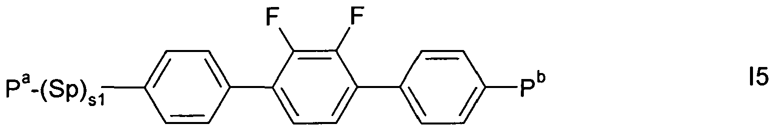 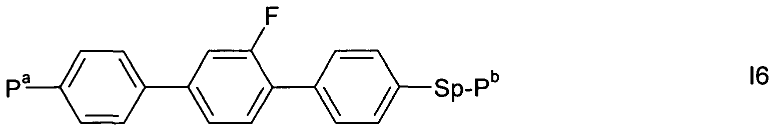 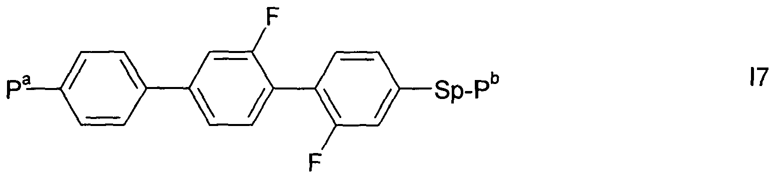 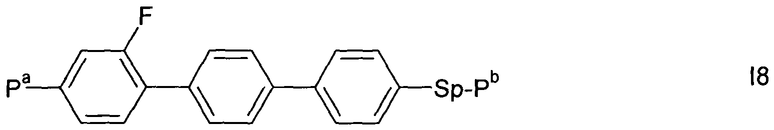 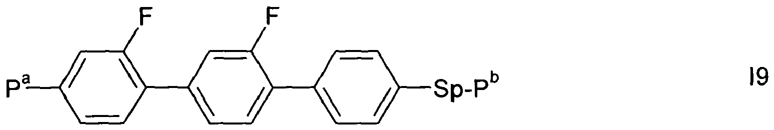 in which P^{a}, P^{b} and Sp have the meaning indicated in Claim 3, and s1 denotes 1.

12. Compounds according to Claim 10 or 11, **characterised in that** P^{a} and P^{b} each, independently of one another, denote a vinyloxy, acrylate, methacrylate, fluoroacrylate, chloroacrylate, oxetane or epoxy group.

13. Compounds according to one or more of Claims 10 to 12, **characterised in that** Sp is selected on each occurrence, identically or differently, from the formula Sp"-X", so that the radical "P^{a/b}-Sp- " conforms to the formula "P^{a/b}-Sp"-X"-", where
Sp" denotes alkylene having 1 to 20, preferably 1 to 12, C atoms, which is optionally mono- or polysubstituted by F, Cl, Br, I or CN, and in which, in addition, one or more non-adjacent CH₂ groups may each be replaced, independently of one another, by -O-, -S-, -NH-, -N(R⁰)-, -Si(R⁰⁰R⁰⁰⁰)-, -CO-, -CO-O-, -O-CO-, -O-CO-O-, -S-CO-, -CO-S-, -N(R⁰⁰)-CO-O-, -O-CO-N(R⁰⁰)-, -N(R⁰⁰)-CO-N(R⁰⁰)-, -CH=CH- or -C≡C- in such a way that O and/or S atoms are not linked directly to one another,
X" denotes -O-, -S-, -CO-, -CO-O-, -O-CO-, -O-CO-O-, -CON(R⁰⁰)-, -N(R⁰⁰)-CO-, -N(R⁰⁰)-CO-N(R⁰⁰)-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CY²=CY³-, -C≡C-, -CH=CH-CO-O-, -O-CO-CH=CH- or a single bond,
R⁰⁰ and R⁰⁰⁰ each, independently of one another, denote H or alkyl having 1 to 12 C atoms, and
Y² and Y³ each, independently of one another, denote H, F, Cl or CN.

14. LC medium comprising one or more polymerisable compounds of the formula I according to one or more of Claims 10 to 13.

15. LC medium according to Claim 14, comprising
- a polymerisable component A) comprising one or more polymerisable compounds, and
- a liquid-crystalline component B) comprising one or more low-molecular-weight compounds,
**characterised in that** component A) comprises one or more polymerisable compounds as defined in Claim 14.

16. LC medium according to Claim 14 or 15, in which component B) comprises one or more compounds selected from the formulae CY, PY and ZK according to Claim 6 or 7.

17. Compounds of the formula II 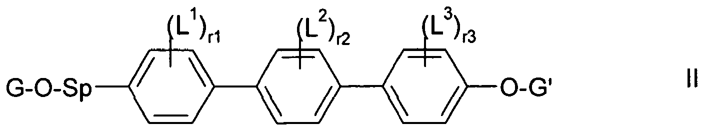 in which Sp, L¹,L², L³, r1, r2 and r3 have the meaning indicated in Claim 3, and G and G' each, independently of one another, denote an H atom or a protecting group.

18. Process for the preparation of a compound according to one or more of Claims 13 to 16, in which a compound according to Claim 17 is esterified or etherified using corresponding acids, acid derivatives, or halogenated compounds containing a group P^{a} or P^{b}, in the presence of a dehydrating reagent.

19. Process for the production of an LC display of the PS or PSA type, in which an LC medium according to one or more of Claims 10 to 12 is introduced into an LC cell having two substrates and two electrodes, where at least one substrate is transparent to light and at least one substrate has one or two electrodes, and the polymerisable compounds are polymerised, preferably with application of an electrical voltage to the electrodes.

## Revendications

1. Utilisation de composés polymérisables contenant un groupe terphényle, lequel est au moins monosubstitué par un atome d'halogène, et un ou plusieurs groupes polymérisables dans des milieux LC et des affichages LC du type PS ou PSA.

2. Utilisation selon la revendication 1, **caractérisée en ce que** les composés polymérisables contiennent deux ou plus de deux groupes polymérisables, où au moins l'un de ces groupes polymérisables est connecté au groupe terphényle via un groupe d'espaceur et au moins l'un de ces groupes polymérisables est connecté directement au groupe terphényle.

3. Utilisation selon la revendication 1 ou 2, **caractérisée en ce que** les composés polymérisables sont choisis à partir de la formule I 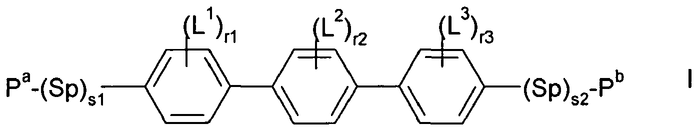 dans laquelle les radicaux individuels présentent les significations qui suivent :
P^{a}, P^{b} représentent, chacun indépendamment de l'autre, un groupe polymérisable,
Sp représente, pour chaque occurrence, de manière identique ou différente, un groupe d'espaceur,
L¹,L², L³ représentent, chacun indépendamment des autres, F ou Cl un ou plusieurs des radicaux L¹ L² et L³ représente(nt) également alkyle ou alcoxy totalement ou partiellement fluoré comportant 1, 2 ou 3 atomes de C, P^{a} ou P^{a}-Sp-, où au moins l'un des radicaux L¹ L² et L³ représente F ou Cl de préférence F,
r1, r2, r3 représentent, chacun indépendamment des autres, 0, 1, 2, 3 ou 4, où r1+r2+r3 ≥ 1,
s1 et s2 représentent, chacun indépendamment de l'autre, 0 ou 1.

4. Utilisation selon une ou plusieurs des revendications 1 à 3, **caractérisée en ce que** les composés de la formule I sont choisis parmi le groupe constitué par les sous-formules qui suivent : 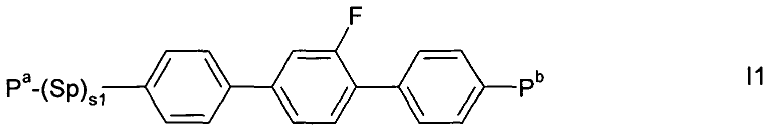 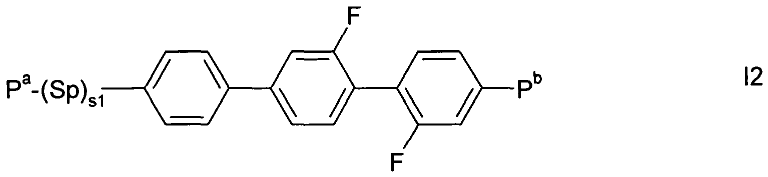 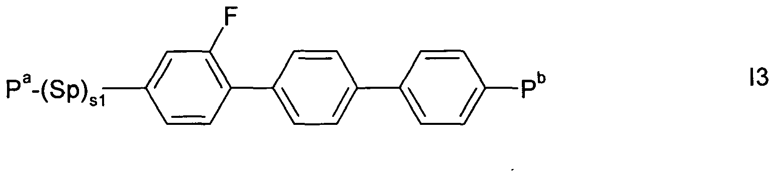 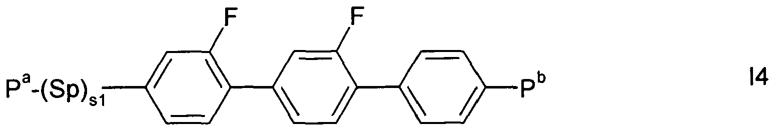 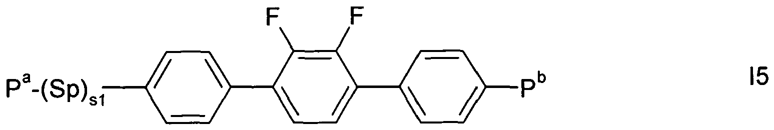 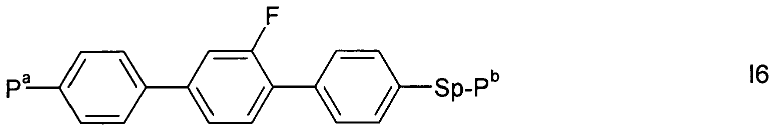 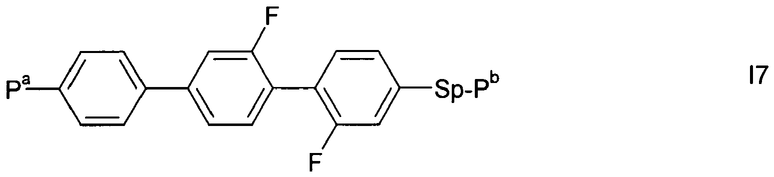 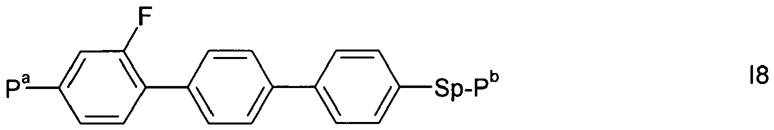 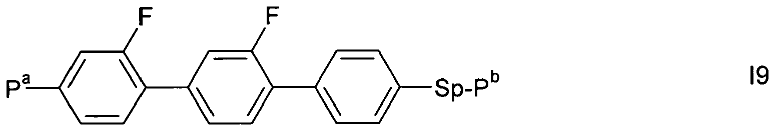 dans lesquelles P^{a}, P^{b}, Sp et s1 présentent la signification indiquée selon la revendication 3.

5. Utilisation de composés selon une ou plusieurs des revendications 1 à 4 dans des affichages LC du type PS ou PSA contenant une cellule LC comportant deux substrats et deux électrodes, où au moins un substrat est transparent vis-à-vis de la lumière et au moins un substrat comporte une ou deux électrodes, et une couche, située entre les substrats, d'un milieu LC comprenant un composant polymérisé et un composant de poids moléculaire faible, où le composant polymérisé peut être obtenu par polymérisation d'un ou de plusieurs composés polymérisables entre les substrats de la cellule LC dans le milieu LC, de préférence à l'aide de l'application d'une tension électrique entre les électrodes, où au moins l'un des composés polymérisables est un composé polymérisable selon une ou plusieurs des revendications 1 à 4.

6. Utilisation selon une ou plusieurs des revendications 1 à 5, **caractérisée en ce que** le milieu LC comprend un ou plusieurs composés des formules CY et/ou PY : 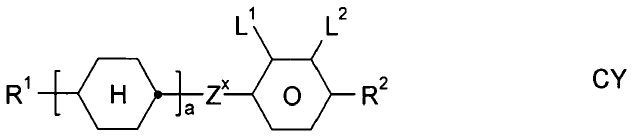 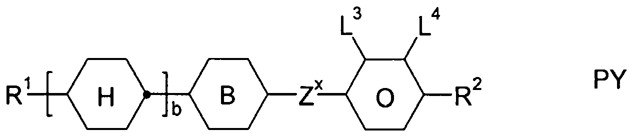 dans lesquelles les radicaux individuels présentent les significations qui suivent:
a représente 1 ou 2,
b représente 0 ou 1, 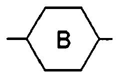 représente 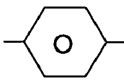 ou 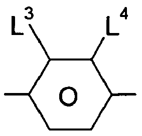
R¹ et R² représentent, chacun indépendamment de l'autre, alkyle comportant de 1 à 12 atomes de C, où, en outre, un ou deux groupes CH₂ non adjacents peut/peuvent être remplacé(s) par -O-, -CH=CH-, -CO-, -O-CO- ou -CO-O- de telle sorte que des atomes de O ne soient pas liés directement les uns aux autres,
Z^{x} représente -CH=CH-, -CH₂O-, -OCH₂-, -CF₂O-, -OCF₂-, -O-, -CH₂-, -CH₂CH₂- ou une liaison simple, de préférence une liaison simple,
L¹⁻⁴ représentent, chacun indépendamment des autres, F, Cl, OCF₃, CF₃, CH₃, CH₂F, CHF₂.

7. Utilisation selon une ou plusieurs des revendications 1 à 6, **caractérisée en ce que** le milieu LC comprend un ou plusieurs composés de la formule qui suit : 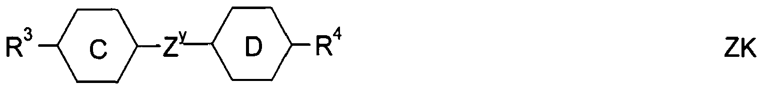 dans laquelle les radicaux individuels présentent les significations qui suivent : 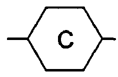 représente 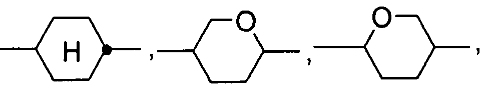 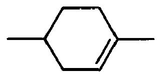 ou 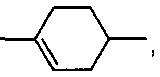 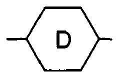 représente 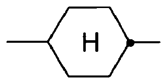 ou 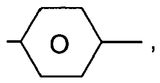
R³ et R⁴ représentent, chacun indépendamment de l'autre, alkyle comportant de 1 à 12 atomes de C, où, en outre, un ou deux groupes CH₂ non adjacents peut/peuvent être remplacé(s) par -O-, -CH=CH-, -CO-, -O-CO- ou -CO-O- de telle sorte que des atomes de O ne soient pas liés directement les uns aux autres,
Z^{y} représente -CH₂CH₂-, -CH=CH-, -CF₂O-, -OCF₂-, -CH₂O-, -OCH₂-, -COO-, -OCO-, -C₂F₄-, -CF=CF- ou une liaison simple.

8. Affichage LC selon une ou plusieurs des revendications 1 à 7.

9. Affichage LC selon la revendication 8, **caractérisé en ce qu'**il s'agit d'un affichage PSA-VA, PSA-OCB, PSA-IPS, PSA-FFS, PSA-positif-VA ou PSA-TN.

10. Composés de la formule I 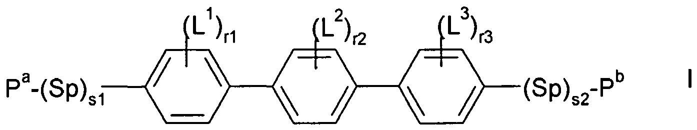 dans laquelle P^{a}, P^{b}, Sp, L¹ L², L³, r1, r2 et r3 présentent la signification indiquée selon la revendication 3, et s1 représente 1 et s2 représente 0, ou s1 représente 0 et s2 représente 1.

11. Composés selon la revendication 10, choisis parmi le groupe constitué par les sous-formules qui suivent : 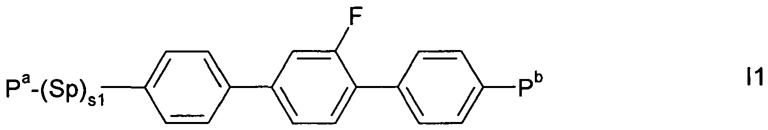 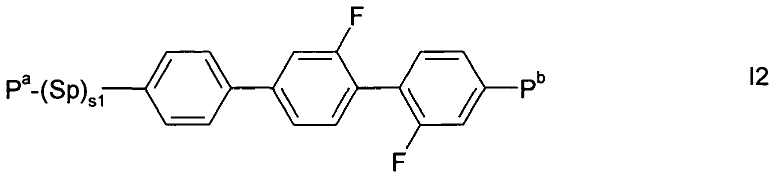 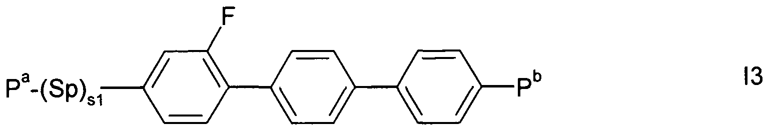 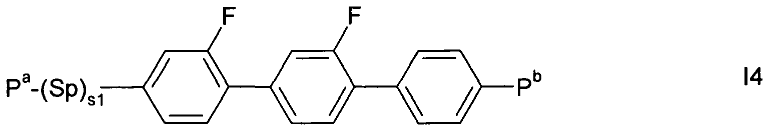 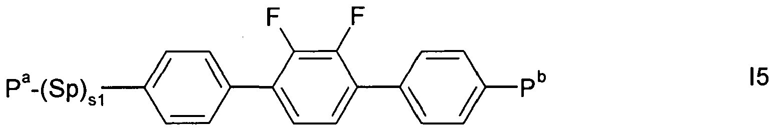 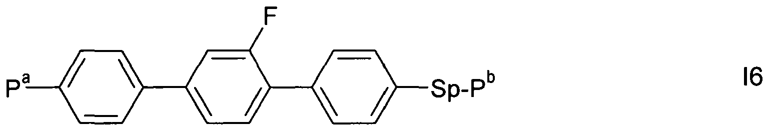 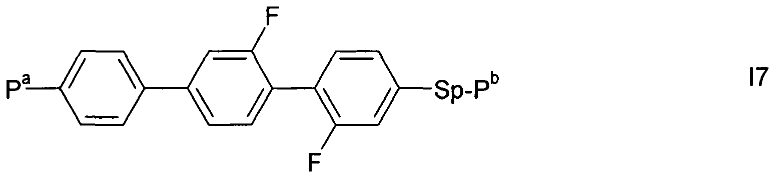 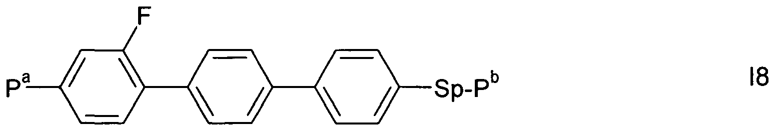 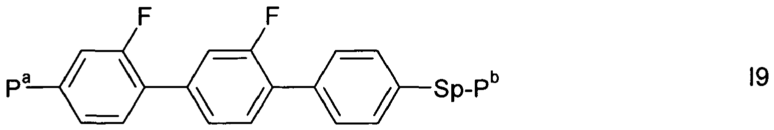 dans lesquelles P^{a}, P^{b} et Sp présentent la signification indiquée selon la revendication 3, et s1 représente 1.

12. Composés selon la revendication 10 ou 11, **caractérisés en ce que** P^{a} et P^{b} représentent, chacun indépendamment de l'autre, un groupe vinyloxy, acrylate, méthacrylate, fluoroacrylate, chloroacrylate, oxétane ou époxy.

13. Composés selon une ou plusieurs des revendications 10 à 12, **caractérisés en ce que** Sp est choisi, pour chaque occurrence, de manière identique ou différente, à partir de la formule Sp"-X", de telle sorte que le radical "P^{a/b}-Sp- "soit conforme à la formule "P^{a/b}-Sp"-X"- " où
Sp" représente alkylène comportant de 1 à 20, de préférence de 1 à 12, atomes de C, lequel est en option mono- ou polysubstitué par F, Cl Br, I ou CN, et où, en outre, un ou plusieurs groupes CH₂ non adjacents peuvent chacun être remplacés, indépendamment les uns des autres, par -O-, -S-, -NH-, -N(R⁰)-, -Si(R⁰⁰R⁰⁰⁰)-, -CO-, -CO-O-, -O-CO-, -O-CO-O-, -S-CO-, -CO-S-, -N(R⁰⁰)-CO-O-, -O-CO-N(R⁰⁰)-, -N(R⁰⁰)-CO-N(R⁰⁰)-, -CH=CH- ou -C≡C- de telle sorte que des atomes de O et/ou de S ne soient pas liés directement les uns aux autres,
X" représente -O-, -S-, -CO-, -CO-O-, -O-CO-, -O-CO-O-, -CON(R⁰⁰)-, -N(R⁰⁰)-CO-, -N(R⁰⁰)-CO-N(R⁰⁰)-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CY²=CY³-, -C≡C-, -CH=CH-CO-O-, -O-CO-CH=CH- ou une liaison simple,
R⁰⁰ et R⁰⁰⁰ représentent, chacun indépendamment de l'autre, H ou alkyle comportant de 1 à 12 atomes de C, et
Y² et Y³ représentent, chacun indépendamment de l'autre, H, F, Cl ou CN.

14. Milieu LC comprenant un ou plusieurs composés polymérisables de la formule I selon une ou plusieurs des revendications 10 à 13.

15. Milieu LC selon la revendication 14, comprenant
- un composant polymérisable A) comprenant un ou plusieurs composés polymérisables, et
- un composant cristallin liquide B) comprenant un ou plusieurs composés de poids moléculaire faible,
**caractérisé en ce que** le composant A) comprend un ou plusieurs composés polymérisables comme défini selon la revendication 14.

16. Milieu LC selon la revendication 14 ou 15, dans lequel un composant B) comprend un ou plusieurs composés choisis parmi les formules CY, PY et ZK selon la revendication 6 ou 7.

17. Composés de la formule II 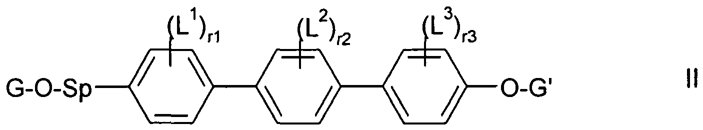 dans laquelle Sp, L¹ L², L³, r1, r2 et r3 présentent la signification indiquée selon la revendication 3, et G et G' représentent, chacun indépendamment de l'autre, un atome de H ou un groupe de protection.

18. Procédé pour la préparation d'un composé selon une ou plusieurs des revendications 13 à 16, où un composé selon la revendication 17 est estérifié ou éthérifié en utilisant des acides correspondants, des dérivés d'acide correspondants ou des composés halogénés correspondants contenant un groupe P^{a} ou P^{b}, en présence d'un réactif déshydratant.

19. Procédé pour la production d'un affichage LC du type PS ou PSA, dans lequel un milieu LC selon une ou plusieurs des revendications 10 à 12 est introduit à l'intérieur d'une cellule LC comportant deux substrats et deux électrodes, où au moins un substrat est transparent vis-à-vis de la lumière et au moins un substrat comporte une ou deux électrodes, et les composés polymérisables sont polymérisés, de préférence à l'aide de l'application d'une tension électrique entre les électrodes.
